(19) Europäisches Patentamt
European Patent Office
Office européen des brevets

(11) EP 1 326 576 B2

(12) # NOUVEAU FASCICULE DE BREVET EUROPEEN
Après la procédure d'opposition

(45) Date de publication et mention de la décision concernant l'opposition:
**25.03.2015 Bulletin 2015/13**

(45) Mention de la délivrance du brevet:
**20.12.2006 Bulletin 2006/51**

(21) Numéro de dépôt: **01972167.9**

(22) Date de dépôt: **19.09.2001**

(51) Int Cl.:
***A61K 8/46*** (2006.01) ***A61Q 5/08*** (2006.01)

(86) Numéro de dépôt international:
**PCT/FR2001/002910**

(87) Numéro de publication internationale:
**WO 2002/030369 (18.04.2002 Gazette 2002/16)**

(54) **COMPOSITION COSMETIQUE CONTENANT DES DERIVES D'ACIDE SULFINIQUE**
KOSMETISCHE ZUSAMMENSETZUNG, DIE SULFINSÄUREDERIVATE ENTHÄLT
COSMETIC COMPOSITION CONTAINING SULPHINIC ACID DERIVATIVES

(84) Etats contractants désignés:
**AT BE CH CY DE DK ES FI FR GB GR IE IT LI LU MC NL PT SE TR**

(30) Priorité: **11.10.2000 FR 0013022**

(43) Date de publication de la demande:
**16.07.2003 Bulletin 2003/29**

(73) Titulaire: **L'Oréal**
**75008 Paris (FR)**

(72) Inventeurs:
- **LEGRAND, Frédéric**
  **F-92400 Courbevoie (FR)**
- **MILLEQUANT, Jean-Marie**
  **F-94100 Saint Maur des Fosses (FR)**

(74) Mandataire: **Wattremez, Catherine**
**L'Oréal**
**D.I.P.I.**
**25-29 Quai Aulagnier**
**92665 Asnières (FR)**

(56) Documents cités:

| | |
|---|---|
| **EP-A- 0 576 287** | **WO-A1-99/18067** |
| **DE-A1- 19 743 759** | **US-A- 3 800 809** |
| **US-A- 3 892 845** | **US-A- 5 968 486** |

- **Technisher Versuchsbericht from OI P. 1-7**
- **S.V. MAKAROV ET AL.: 'Change in the Formaldehyde Sodium Sulfoxylate Reactivity during Storage' RUSSIAN JOURNAL OF PHYSICAL CHEMISTRY vol. 70, no. 10, 1996, pages 1773 - 1775**
- **M. GREENBLATT: 'Formaldehyde Toxicology: A Review of Recent Research and Regulatory Changes' LABORATORY MEDICINE vol. 19, no. 7, 07 Juillet 1988, pages 425 - 428**

Remarques:
Le dossier contient des informations techniques présentées postérieurement au dépôt de la demande et ne figurant pas dans le présent fascicule.

EP 1 326 576 B2

## Description

L'invention a pour objet l'utilisation pour la décoloration de fibres kératiniques humaines teintes par des colorants d'oxydation et/ou des colorants directs d'une composition cosmétique comprenant, dans un milieu cosmétiquement acceptable à un pH compris entre 1,5 et 9, à titre de réducteur de colorant, au moins un dérivé d'acide sulfinique de formule (I) ci-après définie. L'invention concerne aussi les dispositifs à plusieurs compartiments pour la teinture et la décoloration desdites fibres et le procédé de décoloration mettant en oeuvre cette composition.

Il est connu de teindre les fibres kératiniques et en particulier les cheveux humains avec des compositions tinctoriales contenant des colorants d'oxydation et/ou des colorants directs.

La teinture effectuée avec des colorants d'oxydation ou "coloration d'oxydation" est une coloration permanente; elle comprend à titre de colorants d'oxydation, les précurseurs de coloration d'oxydation et les coupleurs.

Les précurseurs de coloration d'oxydation, appelés couramment "bases d'oxydation", sont des composés initialement incolores ou faiblement colorés qui développent leur pouvoir tinctorial au sein du cheveu en présence d'agents oxydants ajoutés au moment de l'emploi, en conduisant à la formation de composés colorés et colorants. La formation de ces composés colorés et colorants résulte, soit d'une condensation oxydative des "bases d'oxydation" sur elles-mêmes, soit d'une condensation oxydative des "bases d'oxydation" sur des composés modificateurs de coloration appelés couramment "coupleurs" et généralement présents dans les compositions tinctoriales utilisées en teinture d'oxydation.

Les bases d'oxydation sont en particulier des ortho- ou para-phénylènediamines, des ortho- ou para-aminophénols, et des bases hétérocycliques.

Les coupleurs sont choisis notamment parmi les métadiamines aromatiques, les méta-aminophénols, les métadiphénols et certains composés hétérocycliques.

La variété des molécules mises en jeu au niveau des bases d'oxydation et des coupleurs, permet l'obtention d'une riche palette de couleurs.

Pour varier les nuances obtenues avec lesdits colorants d'oxydation, ou les enrichir de reflets, Il arrive qu'on leur ajoute des colorants directs.

La teinture effectuée avec des colorants directs donne une coloration semi-permanente
ou temporaire; les colorants directs apportent à la coloration naturelle des cheveux, une modification de couleur plus ou moins marquée résistant éventuellement à plusieurs shampooings.

Ces colorants directs peuvent être mis en oeuvre sans agent oxydant.

Les colorants directs classiquement utilisés sont choisis notamment parmi les colorants directs nitrés benzéniques, les colorants directs azoïques, les colorants directs quinoniques et en particulier anthraquinoniques, les colorants directs aziniques, les colorants directs triarylméthaniques, les colorants directs indoaminiques et les colorants directs naturels.

Plus récemment dans les demandes de brevets WO-95/15144, WO-95/01772 et EP-1025834, on a préconisé d'utiliser de nouveaux colorants directs cationiques se distinguant des colorants directs classiques par les nuances très chromatiques qu'ils confèrent aux fibres kératiniques.

Lesdits colorants directs cationiques peuvent être utilisés en milieu oxydant.

En présence d'oxydant, le but est d'obtenir une coloration éclaircissante. La coloration éclaircissante est mise en oeuvre en appliquant sur les cheveux le mélange extemporané du colorant direct cationique et d'un oxydant et permet notamment d'obtenir, par éclaircissement de la mélanine des cheveux, un effet avantageux tel qu'une couleur unie dans le cas des cheveux gris ou de faire ressortir la couleur dans le cas de cheveux naturellement pigmentés.

Cependant pour des raisons diverses, telles que le souhait de moduler partiellement ou totalement la nuance ainsi conférée à la chevelure par une teinture d'oxydation, une teinture directe, ou une teinture éclaircissante, ou le souhait d'éliminer cette coloration, on peut être amené à vouloir détruire partiellement ou totalement les pigments ainsi formés ou amenés dans ou sur le cheveu.

Cette décoloration a été jusqu'ici effectuée via des procédés mettant en oeuvre des systèmes oxydants ou réducteurs.

Dans les systèmes oxydants, les agents oxydants classiquement utilisés, sont le peroxyde d'hydrogène ou des composés susceptibles de produire le peroxyde d'hydrogène par hydrolyse, tels que le peroxyde d'urée ou les persels comme les perborates, les percarbonates et les persulfates, le peroxyde d'hydrogène et les persulfates étant particulièrement préférés.

Parmi les systèmes réducteurs, on connaît, de part le brevet allemand N°1 151 242, l'usage de l'acide hydroxyméthanesulfinique à un pH compris entre 7 et 9, pour décolorer des cheveux colorés. On connaît également l'usage du sulfite de sodium ($Na_2SO_3$) dans les brevets US-2149319 et US-3838966 et la demande JP-04356413A.

Un procédé pour décolorer les fibres kératiniques teintes est également décrit dans le brevet US-3892845 et consiste à appliquer sur les fibres une composition aqueuse comprenant la combinaison de deux types de réducteurs, un réducteur du colorant et un réducteur des liaisons covalentes disulfure de la kératine; le réducteur du colorant est un hydroxyméthanesulfinate ou un hydrosulfite de zinc, potassium, sodium ou de calcium, et le réducteur de la kératine est notamment l'acide thioglycolique, un bisulfate

ou un bisulfite de potassium ou de sodium, le bisulfure de potassium, la thiourée, ou certains composés phosphorés.

Mais il est également connu de décolorer les fibres kératiniques humaines telles que les cheveux et en particulier les cheveux teints artificiellement avec des colorants d'oxydation, à l'aide d'agents réducteurs à pH acide.

La technologie de décoloration par réduction à pH acide a l'avantage de moins sensibiliser la fibre capillaire et de ne pas éclaircir la base naturelle des cheveux.

Ainsi des produits commerciaux utilisent l'hydroxyméthanesulfinate de sodium comme réducteur des cheveux teints à pH acide.

Et dans la demande de brevet EP-0943316, on a récemment préconisé d'utiliser une association à pH acide comprenant de l'acide ascorbique et de l'acide alpha-oxocarboxylique pour décolorer les cheveux humains préalablement teints par une teinture d'oxydation, et un produit commercial de décoloration des cheveux comprend de l'acide ascorbique et de l'acide alpha-cétoglutarique.

Cependant, la demanderesse a constaté que toutes ces techniques de l'art antérieur n'engendrent pas une décoloration des fibres kératiniques teintes par des colorants d'oxydation et/ou des colorants directs classiques, suffisamment performante.

En effet, la décoloration est faiblement efficace, voire inefficace, vis-à-vis de certaines nuances, telles que les nuances fondamentales et les nuances à reflets dorés et cendrés.

En outre, ce type de décoloration ne permet pas de décaper suffisamment les fibres kératiniques teintes par les colorants directs cationiques très chromatiques tels que notamment ceux récemment divulgués dans les demandes de brevets WO-95/15144, WO-95101772 et EP-1025834.

Par ailleurs, l'hydroxyméthanesulfinate de sodium est générateur de formol, défavorable à l'innocuité des produits.

La demanderesse vient maintenant de découvrir de façon inattendue et tout à fait surprenante, que des composés de formule (I) décrite ci-après amélioraient de façon très significative la décoloration des fibres kératiniques humaines préalablement teintes avec des colorants d'oxydation et/ou des colorants directs classiques, et permettaient d'éliminer une palette beaucoup plus large de couleurs.

Plus particulièrement encore, il est maintenant devenu possible de décolorer partiellement ou totalement des fibres kératiniques teintes par des colorants directs cationiques tels que ceux décrits ci-dessus.

Ces découvertes sont donc à la base de la présente invention.

L'invention a donc pour premier objet l'utilisation pour la décoloration de fibres kératiniques humaine teintes par des colorants d'oxydation et/ou des colorants directs d'une composition cosmétique, caractérisée par le fait qu'elle comprend, dans un milieu cosmétiquement acceptable à un pH compris entre 1,5 et 9, à titre de réducteur de colorant, au moins un dérivé de l'acide sulfinique de formule (I) suivante :

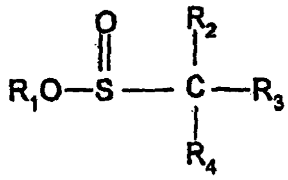

(I)

dans laquelle,

$R_1$ est choisi parmi, un atome d'hydrogène, un ion NH3$^+$, un ion de métal monovalent ou un équivalent ionique de métal bivalent des groupes la, IIa, IIb, IVa et VIIIb du système périodique des éléments,

$R_2$ est choisi parmi, un radical OH, un radical $NR_5R_6$ dans lequel $R_5$ et $R_6$, identiques ou différents, sont choisis parmi un atome d'hydrogène ou un radical alkyle en $C_1$-$C_6$,

$R_3$ est choisi parmi, un atome d'hydrogène, un radical alkyle ou alcényle ou cycloalkyle ou aryle non substitué ou substitué par 1 à 3 substituants choisis, identiques ou différents, parmi les radicaux OH, alkyle en $C_1$-$C_6$, O-alkyle en $C_1$-$C_6$, halogène ou $CF_3$,

$R_4$ est choisi parmi, un radical $COOR_1$, $SO_3R_1$, $COR_5$, $CONR_5R_6$ ou $COOR_5$ dans lesquels $R_1$, $R_5$, et $R_6$ ont les siginifications précédentes,

et $R_4$ désigne également un atome d'hydrogène, lorsque $R_3$ désigne un radical aryle, et en particulier, un radical aryle substitué comme décrit précédemment, et les sels desdits composés cosmétiquement acceptables.

Dans une forme particulièrement préférée, le pH est compris entre 1,8 et 6.

Ladite décoloration peut être partielle ou totale.

Lesdits composés de formule (I) présentent en outre l'avantage de ne pas générer de formol.

Les compositions destinées à la décoloration des cheveux à l'aide d'agents réducteurs se présentent principalement sous forme de compositions prêtes à l'emploi constituées de produits anhydres (poudres) ou de crèmes ou

de gels contenant le ou les agents réducteurs que l'on mélange au moment de l'emploi avec une composition aqueuse contenant un agent de pH. Les compositions de décoloration se présentent également sous forme de compositions prêtes à l'emploi aqueuses contenant le ou les agents réducteurs au pH approprié.

La composition utile selon l'invention est une composition prête à l'emploi.

Et par "composition prête à l'emploi", on entend, au sens de l'invention, la composition destinée à être appliquée telle quelle sur les fibres kératiniques, c'est à dire qu'elle peut être stockée telle quelle avant utilisation ou résulter du mélange extemporané de deux ou plusieurs compositions.

Les sels cosmétiquement acceptables des dérivés d'acides sulfiniques de formule (I) selon l'invention peuvent être choisis parmi les sulfinates alcalins (Na, K), alcalinoterreux (Ca, Mg) ou de zinc.

Les dérivés d'acides sulfiniques de formule (I) selon l'invention sont des composés connus, décrits et préparés dans la demande de brevet WO 99/18067.

Le ou les acides sulfiniques ou leurs sels peuvent être présents dans la composition de décoloration selon l'invention dans des proportions comprises entre 0,01 et 20% et de préférence entre 0,1 et 10% en poids du poids total de la composition.

Selon la présente invention, on préfère les dérivés d'acide sulfinique de formule (I) pour laquelle :

$R_1$ est choisi parmi un ion $NH_3^+$, un ion de métal alcalin, un équivalent ionique de métal alcalino-terreux ou de zinc,
$R_2$ est un radical OH ou un radical $NH_2$,
$R_3$ est choisi parmi, un atome d'hydrogène, un radical alkyle non substitué ou substitué par un ou deux radicaux OH ou un ou deux radicaux alkyle en $C_1$-$C_6$ ou O-alkyle en $C_1$-$C_6$.
$R_4$ est un radical $COOR_1$ ou $COOR_5$, dans lesquels $R_1$ et $R_5$ ont les mêmes significations que dans la formule (I).

On préfère plus particulièrement encore utiliser le composé de formule (I) pour laquelle $R_1$ est Na, $R_2$ est OH, $R_3$ est H et $R_4$ est COONa, c'est à dire le composé de formule (II) suivante :

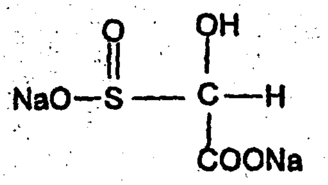

(II)

seul ou en mélange.

Des mélanges comprenant le composé de formule (II) comprennent par exemple en poids, 40 à 60% d'un composé de formule (II), 10 à 20% de $NaSO_3$-CHOH-COONa et de 30 à 40% de $Na_2SO_3$.

Un de ces mélanges vendu par la société BRUGGEMANN sous la dénomination commerciale Brüggolite FF6 comprend en poids, 50% du composé : $NaSO_2$-CHOH-COONa, 15% du composé $NaSO_3$-CHOH-COONa et 35% de $Na_2SO_3$.

L'invention a également pour objet des "kits" d'emballage ou dispositifs à plusieurs compartiments destinés (i)à la teinture puis (ii)à la décoloration des fibres kératiniques teintes par des colorants d'oxydation et/ou des colorants directs, en particulier des fibres kératiniques humaines telles que les cheveux, caractérisé par le fait qu'ils comprennent un premier compartiment comprenant une composition pour la teinture oxydante ou la teinture non-oxydante desdites fibres, et dans un second compartiment une composition pour la décoloration réductrice desdites fibres colorées comprenant au moins un composé de formule (I) décrit ci-dessus à un pH compris entre 1,5 et 9 et de préférence entre 1,8 et 6.

L'invention vise également un procédé de décoloration des fibres kératiniques teintes avec des colorants d'oxydation et/ou des colorants directs, et en particulier des fibres kératiniques humaines telles que les cheveux, mettant en oeuvre une composition de décoloration telle que décrite ci dessus.

Dans le premier compartiment du kit, la teinture oxydante peut être une teinture d'oxydation classique, une teinture directe ou une teinture éclaircissante.

Dans le cas d'une teinture d'oxydation classique, les fibres sont préalablement teintes par au moins un colorant d'oxydation et de préférence par au moins une base d'oxydation en présence d'un oxydant.

Ainsi, dans le premier compartiment du kit où l'on préconise d'utiliser une teinture oxydante telle que la teinture d'oxydation classique, on trouvera au moins une base d'oxydation de préférence choisie parmi la paraphénylène diamine et ses dérivés substitués sur une des fonctions amines et/ou sur le noyau benzénique, le para-aminophénol et ses dérivés substitués sur la fonctions amine et/ou sur le noyau benzénique, les bases doubles, les ortho-aminophénols, les orthophénylènediamines et les bases hétérocycliques.

Parmi les bases hétérocycliques utilisables à titre de base d'oxydation selon l'invention, on peut plus particulièrement citer les dérivés pyridiniques, les dérivés pyrimidiniques, les dérivés pyrazoliques. Tous ces composés peuvent être utilisés sous forme libre ou sous forme de leurs sels d'addition avec un acide.

On peut notamment citer :

- (**I**) les paraphénylènediamines de formule (I) suivante et leurs sels d'addition avec un acide :

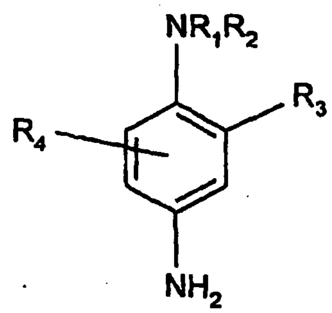

(I)

dans laquelle :

$R_1$ représente un atome d'hydrogène, un radical alkyle en $C_1$-$C_4$, monohydroxyalkyle en $C_1$-$C_4$, polyhydroxyalkyle en $C_2$-$C_4$ alcoxy($C_1$-$C_4$)alkyle($C_1$-$C_4$), alkyle en $C_1$-$C_4$ substitué par un groupement azoté, phényle ou 4'-aminophényle ;
$R_2$ représente un atome d'hydrogène, un radical alkyle en $C_1$-$C_4$, monohydroxyalkyle en $C_1$-$C_4$ ou polyhydroxyalkyle en $C_2$-$C_4$, alcoxy($C_1$-$C_4$)alkyle($C_1$-$C_4$) ou alkyle en $C_1$-$C_4$ substitué par un groupement azoté ;
$R_3$ représente un atome d'hydrogène, un atome d'halogène tel qu'un atome de chlore, un radical alkyle en $C_1$-$C_4$, sulfo, carboxy, monohydroxyalkyle en $C_1$-$C_4$ ou hydroxyalcoxy en $C_1$-$C_4$, acétylaminoalcoxy en $C_1$-$C_4$, mésylaminoalcoxy en $C_1$-$C_4$ ou carbamoylaminoalcoxy en $C_1$-$C_4$,
$R_4$ représente un atome d'hydrogène, d'halogène ou un radical alkyle en $C_1$-$C_4$ ;
$R_1$ et $R_2$ peuvent également former avec l'atome d'azote qui les porte un hétérocycle azoté à 5 ou 6 chaînons éventuellement substitué par un ou plusieurs groupements alkyle, hydroxy ou uréido.

Parmi les groupements azotés de la formule (I) ci-dessus, on peut citer notamment les radicaux amino, monoalkyl($C_1$-$C_4$)amino, dialkyl($C_1$-$C_4$)amino, trialkyl($C_1$-$C_4$)amino, monohydroxyalkyl($C_1$-$C_4$)amino, imidazolinium et ammonium.
Parmi les paraphénylènediamines de formule (I) ci-dessus, on peut plus particulièrement citer la paraphénylènediamine, la paratoluylènediamine, la 2-chloro-paraphénylènediamine, la 2,3-diméthyl-paraphénylènediamine, la 2,6-diméthyl-paraphénylènediamine, la 2,6-diéthyl-paraphénylènediamine, la 2,5-diméthyl-paraphénylènediamine, la N,N-diméthyl-paraphénylènediamine, la N,N-diéthyl-paraphénylènediamine, la N,N-dipropyl-paraphénylènediamine, la 4-amino-N,N-diéthyl-3-méthyl-aniline, la N,N-bis-(β-hydroxyéthyl)-paraphénylènediamine, la 4-N,N-bis-(β-hydroxyéthyl)amino-2-méthyl-aniline, la 4-N,N-bis-(β-hydroxyéthyl)-amino 2-chloro-aniline, la 2-β-hydroxyéthyl-paraphénylènediamine, la 2-fluoro-paraphénylènediamine, la 2-isopropyl-paraphénylènediamine, la N-(β-hydroxypropyl)-paraphénylènediamine, la 2-hydroxyméthyl-paraphénylènediamine, la N,N-diméthyl-3-méthylparaphénylènediamine, la N,N-(éthyl,β-hydroxyéthyl)-paraphénylènediamine, la N-(β,γ-dihydroxypropyl)-paraphénylènediamine, la N-(4'-àminophényt)-paraphénylènediamine, la N-phényl-paraphénylènediamine, la 2-β-hydroxyéthyloxy-paraphénylènediamine, la 2-β-acétylaminoéthyloxy-paraphénylènediamine, la N-(β-méthoxyéthyl)-paraphénylènediamine, 2-méthyl-1-N-β-hydroxyéthyl-paraphénylènediamine, et leurs sels d'addition avec un acide.
Parmi les paraphénylènediamines de formule (I) ci-dessus, on préfère tout particulièrement la paraphénylènediamine, la paratoluylènediamine, la 2-isopropyl-paraphénylènediamine, la 2-β-hydroxyéthyl-paraphénylènediamine, la 2-β-hydroxyéthyloxy-paraphénylènediamine, la 2,6-diméthyl-paraphénylène-diamine, la 2,6-diéthyl-paraphénylènediamine, la 2,3-diméthyl-paraphénylènediamine, la N,N-bis-(β-hydroxyéthyl)-paraphénylènediamine, la 2-chloro-paraphénylènediamine, et leurs sels d'addition avec un acide.

- (**II**) Selon l'invention, on entend par bases doubles, les composés comportant au moins deux noyaux aromatiques sur lesquels sont portés des groupements amino et/ou hydroxyle.
Parmi les bases doubles utilisables à titre de bases d'oxydation dans les compositions tinctoriales conformes à l'invention, on peut notamment citer les composés répondant à la formule (II) suivante, et leurs sels d'addition avec un acide :

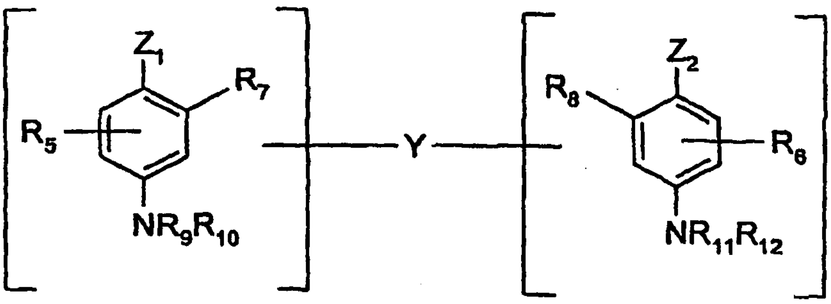

(II)

dans laquelle :

- $Z_1$ et $Z_2$, identiques ou différents, représentent un radical hydroxyle ou $-NH_2$ pouvant être substitué par un radical alkyle en $C_1$-$C_4$ ou par un bras de liaison Y ;
- le bras de liaison Y représente une chaîne alkylène comportant de 1 à 14 atomes de carbone, linéaire ou ramifiée pouvant être interrompue ou terminée par un ou plusieurs groupements azotés et/ou par un ou plusieurs hétéroatomes tels que des atomes d'oxygène, de soufre ou d'azote, et éventuellement substituée par un ou plusieurs radicaux hydroxyle ou alcoxy en $C_1$-$C_6$;
- $R_5$ et $R_6$ représentent un atome d'hydrogène ou d'halogène, un radical alkyle en $C_1$-$C_4$, monohydroxyalkyle en $C_1$-$C_4$, polyhydroxyalkyle en $C_2$-$C_4$, aminoalkyle en $C_1$-$C_4$

ou un bras de liaison Y ;

- $R_7$, $R_8$, $R_9$, $R_{10}$, $R_{11}$ et $R_{12}$, identiques ou différents, représentent un atome d'hydrogène, un bras de liaison Y ou un radical alkyle en $C_1$-$C_4$ ; étant entendu que les composés de formule (II) ne comportent qu'un seul bras de liaison Y par molécule.

Parmi les groupements azotés de la formule (II) ci-dessus, on peut citer notamment les radicaux amino, monoalkyl($C_1$-$C_4$)amino, dialkyl($C_1$-$C_4$)amino, trialkyl($C_1$-$C_4$)amino, monohydroxyalkyl($C_1$-$C_4$)amino, imidazolinium et ammonium.

Parmi les bases doubles de formules (II) ci-dessus, on peut plus particulièrement citer le N,N'-bis-(β-hydroxyéthyl) N,N'-bis-(4'-aminophényl) 1,3-diamino propanol, la N,N'-bis-(β-hydroxyéthyl) N,N'-bis-(4'-aminophényl) éthylènediamine, la N,N'-bis-(4-aminophényl) tétraméthylènediamine, la N,N'-bis-(β-hydroxyéthyl) N,N'-bis-(4-aminophényl) tétraméthylènediamine, la N,N'-bis-(4-méthyl-aminophényl) tétraméthylènediamine, la N,N'-bis-(éthyl) N,N'-bis-(4'-amino, 3'-méthylphényl) éthylènediamine, le 1,8-bis-(2,5-diaminophénoxy)-3,5-dioxaoctane, et leurs sels d'addition avec un acide.

Parmi ces bases doubles de formule (II), le N,N'-bis-(β-hydroxyéthyl) N,N'-bis-(4'-aminophényl) 1,3-diamino propanol, le 1,8-bis-(2,5-diaminophénoxy)-3,5-dioxaoctane ou l'un de leurs sels d'addition avec un acide sont particulièrement préférés.

- **(III)** les para-aminophénols répondant à la formule (III) suivante, et leurs sels d'addition avec un acide :

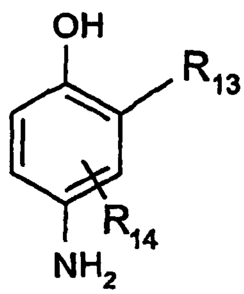

(III)

dans laquelle :

$R_{13}$ représente un atome d'hydrogène,un atome d'halogène tel que le fluor, un radical alkyle en $C_1$-$C_4$, monohydroxyalkyle en $C_1$-$C_4$, alcoxy($C_1$-$C_4$)alkyle($C_1$-$C_4$) ou aminoalkyle en $C_1$-$C_4$, ou hydroxyalkyl($C_1$-$C_4$)aminoalkyle en $C_1$-$C_4$.

$R_{14}$ représente un atome d'hydrogène ou un atome d'halogène tel que le fluor, un radical alkyle en $C_1$-$C_4$, monohydroxyalkyle en $C_1$-$C_4$, polyhydroxyalkyle en $C_2$-$C_4$, aminoalkyle en $C_1$-$C_4$, cyanoalkyle en $C_1$-$C_4$ ou alcoxy($C_1$-$C_4$)alkyle($C_1$-$C_4$).

Parmi les para-aminophénols de formule (III) ci-dessus, on peut plus particulièrement citer le para-aminophénol, le 4-amino-3-méthyl-phénol, le 4-amino-3-fluoro-phénol, le 4-amino-3-hydroxyméthyl-phénol, le 4-amino-2-méthylphénol, le 4-amino-2-hydroxyméthyl-phénol, le 4-amino-2-méthoxyrnéthyl-phénol, le 4-amino-2-aminométhylphénol, le 4-amino-2-(β-hydroxyéthyl-aminométhyl)-phénol, et leurs sels d'addition avec un acide.

- (**IV**) les ortho-aminophénols utilisables à titre de bases d'oxydation dans le cadre de la présente l'invention, sont notamment choisis parmi le 2-amino-phénol, le 2-amino-1-hydroxy-5-méthyl-benzène, le 2-amino-1-hydroxy-6-méthyl-benzène, le 5-acétamido-2-amino-phénol, et leurs sels d'addition avec un acide.

- (**V**) parmi les bases hétérocycliques utilisables à titre de bases d'oxydation dans les compositions tinctoriales conformes à l'invention, on peut plus particulièrement citer les dérivés pyridiniques, les dérivés pyrimidiniques, les dérivés pyrazoliques, et leurs sels d'addition avec un acide.

Parmi les dérivés pyridiniques, on peut plus particulièrement citer les composés décrits par exemple dans les brevets GB 1 026 978 et GB 1 153 196, comme la 2,5-diaminopyridine, la 2-(4-méthoxyphényl)amino-3-amino-pyridine, la 2,3-diamino-6-méthoxy-pyridine, la 2-(β-méthoxyéthyl)amino-3-amino-6-méthoxy pyridine, la 3,4-diaminopyridine, et leurs sels d'addition avec un acide.

Parmi les dérivés pyrimidiniques, on peut plus particulièrement citer les composés décrits par exemple dans les brevets allemand DE 2 359 399 ou japonais JP 88-169 571 et JP 91-10659 ou demandes de brevet WO 96/15765, comme la 2,4,5,6-tétra-aminopyrimidine, la 4-hydroxy-2,5,6-triaminopyrimidine, la 2-hydroxy-4,5,6-triaminopyrimidine, la 2,4-dihydroxy-5,6-diaminopyrimidine, la 2,5,6-triaminopyrimidine, et les dérivés pyrazolo-pyrimidiniques tels ceux mentionnés dans la demande de brevet FR-A-2 750 048 et parmi lesquels on peut citer la pyrazolo-[1,5-a]-pyrimidine-3,7-diamine ; la 2,5-diméthyl-pyrazolo-[1,5-a]-pyrimidine-3,7-diamine; la pyrazoto-[1,5-a]-pyrimidine-3,5-diamine ; la 2,7-diméthyl-pyrazolo-[1,5-a]-pyrimidine-3,5-diamine ; le 3-amino-pyrazolo-[1,5-a]-pyrimidin-7-ol; le 3-amino-pyrazolo-[1,5-a]-pyrimidin-5-ol ; le 2-(3-amino pyrazolo-[1,5-a]-pyrimidin-7-ylamino)-éthanol; le 2-(7-amino-pyrazolo-[1,5-a]-pyrimidin-3-ylamino)-éthanol; le 2-[(3-amino-pyrazolo[1,5-a]pyrimidin-7-yl)-(2-hydroxy-éthyl)-amino]-éthanol; le 2-[(7-amino-pyrazolo[1,5-a]pyrimidin-3-yl)-(2-hydroxy-éthyl)-amino]-éthanol; la 5,6-diméthyl-pyrazoto-[1,5-a]-pyrimidine-3,7-diamine; la 2,6-diméthyl-pyrazolo-[1,5-a]-pyrimidine-3,7-diamine; la 2, 5, N7, N7-tetraméthyl-pyrazolo-[1,5-a]-pyrimidine-3,7-diamine; la 3-amino-5-méthyl-7-imidazolylpropylamino pyrazolo-[1,5-a]-pyrimidine; et leurs sels d'addition et leurs formes tautomères, lorsqu'il existe un équilibre tautomérique et leurs sels d'addition avec un acide.

Parmi les dérivés pyrazoliques, on peut plus particulièrement citer les composés décrits dans les brevets DE 3 843 892, DE 4 133 957 et demandes de brevet WO 94/08969, WO 94/08970, FR-A-2 733 749 et DE 195 43 988 comme le 4,5-diamino-1-méthylpyrazole, le 3,4-diamino-pyrazole, le 4,5-diamino-1-(4'-chlorobenzyl)-pyrazole, le 4,5-diamino-1,3-diméthyl-pyrazole, le 4,5-diamino-3-méthyt-1-phényl pyrazole, le 4,5-diamino 1-méthyl-3-phényl-pyrazole, le 4-amino-1,3-diméthyl-5-hydrazino-pyrazole, le 1-benzyl-4,5-diamino-3-méthyl-pyrazole, le 4,5-diamino-3-tert-butyl-1-méthyl pyrazole, le 4,5-diamino-1-tert-butyl-3-méthyl-pyrazole, le 4,5-diamino-1-(β-hydroxyéthyl)-3-méthyl pyrazole, le 4,5-diamino-9-(β-hydroxyéthyl)-pyrazole, le 4,5-diamino-1-éthyl-3-méthyl-pyrazole, le 4,5-diamino-1-éthyl-3-(4'-méthoxyphényl)-pyrazole, le 4,5-diamino-1-éthyl-3-hydroxyméthyl-pyrazole, le 4,5-diamino-3-hydroxyméthyl-1-méthyl-pyrazole, le 4,5-diamino-3-hydroxyméthyl-1-isopropyl-pyrazole, le 4,5-diamino-3-méthyl-1-isopropylpyrazole, le 4-amino-5-(2'-aminoéthyl)amino-1,3-diméthyl-pyrazole, le 3,4,5-triaminopyrazole, le 1-méthyl-3,4,5-triamino-pyrazole, le 3,5-diamino-1-méthyl-4-méthylaminopyrazole, le 3,5-diamino-4-(β-hydroxyéthyl)amino-1-méthyl-pyrazole, et leurs sels d'addition avec un acide.

Généralement, les bases d'oxydation représentent de préférence de 0,0005 à 12% en poids environ du poids total de la composition et encore plus préférentiellement de 0,005 à 8% en poids environ de ce poids.

Plus préférentiellement encore, les fibres kératiniques sont teintes par au moins une base d'oxydation et au moins un coupleur en présence d'un oxydant.

Par conséquent, dans le premier compartiment du kit où l'on préconise d'utiliser une teinture d'oxydation classique, on trouvera au moins une base d'oxydation et au moins un coupleur.

Parmi ces coupleurs, on peut notamment citer les méta-aminophénols, les métaphénylènediamines, les métadiphénols, les coupleurs hétérocycliques tels que par exemple les dérivés indoliques, les dérivés indoliniques, les dérivés naphtaléniques, le sésamol et ses dérivés, les dérivés pyridiniques, les dérivés pyrazolotriazoles, les pyrazolones, et leurs sels d'addition avec un acide.

Ces coupleurs sont plus particulièrement choisis parmi le 2,4-diamino-1-(β-hydroxyéthyloxy)-benzène, le 2-méthyl-5-amino-phénol, le 5-N-(β-hydroxyéthyl)amino-2-méthyl-phénol, le 3-amino-phénol, le 1,3-dihydroxy-benzène, le 1,3-dihydroxy-2-méthyl-benzène, le 4-chloro-1,3-dihydroxy-benzène, le 2-amino 4-(β-hydroxyéthylamino)-1-méthoxy-benzène, le 1,3-diamino-benzène, le 1,3-bis-(2,4-diaminophénoxy)-propane, le sésamol, le 1-amino-2-méthoxy-4,5-méthylènedioxy benzène, l'α-naphtol, le 1-acétoxy-2-méthyl-naphtalène, le 2-méthyl-1-naphtol, le 6-hydroxy indole, le 4-hydroxy indole, le 4-hydroxy N-méthyl indole, la 6-hydroxy indoline, la 2,6-dihydroxy 4-méthyl pyridine, le 1-H 3-méthyl pyrazole 5-one, le 1-phényl 3-méthyl pyrazole 5-one, la 2-amino 3-hydroxypyridine, le 3,6-diméthyl-pyrazolo-[3,2-c]-1,2,4-triazole, le 2,6-diméthyl-pyrazolo-[1,5-b]-1,2,4-triazole et leurs sels d'addition avec un acide.

Généralement le ou les coupleurs représentent de préférence de 0,0001 à 15% en poids environ du poids total de la composition tinctoriale et encore plus préférentiellement de 0,001 à 10% environ.

D'une manière générale, les sels d'addition avec un acide des bases d'oxydation et des coupleurs sont choisis parmi les chlorhydrates, les bromhydrates, les sulfates et les tartrates, les lactates et les acétates.

Les colorations obtenues avec les teintures d'oxydation classiques peuvent être nuancées par l'adjonction de colorants directs; on pourra donc en outre également introduire au moins un colorant direct dans le premier compartiment du kit ou dans un autre compartiment séparé.

Dans le cas d'une teinture directe, les fibres sont préalablement teintes par au moins un colorant direct. Ainsi, dans le premier compartiment du kit où l'on préconise d'utiliser une teinture directe, on trouvera au moins un colorant direct.

Les colorants directs utilisables pour varier les nuances obtenues au moyen d'une teinture d'oxydation classique ou pour teindre les fibres par un procédé de teinture directe, peuvent être notamment choisis parmi les colorants directs nitrés benzéniques neutres, acides ou cationiques, les colorants directs azoïques neutres acides ou cationiques, les colorants directs méthiniques neutres acides ou cationiques, les colorants directs quinoniques et en particulier anthraquinoniques neutres, acides ou cationiques, les colorants directs aziniques, les colorants directs triarylméthaniques, les colorants directs indoaminiques et les colorants directs naturels.

Parmi les colorants directs benzéniques, on peut citer de manière non limitative les composés suivants:

- 1,4-diamino-2-nitrobenzène
- 1-amino-2-nitro-4-(β-hydroxyéthytamino)-benzène
- 1-amino-2-nitro-4-bis(β-hydroxyéthyl)-aminobenzène
- 1,4-bis(β-hydroxyéthylamino)-2-nitro-benzène
- 1-β-hydroxyéthylamino-2-nitro-4-bis-(β-hydroxyéthylamino)-benzène
- 1-β-hydroxyéthylamino-2-nitro-4-amino-benzène
- 1-β-hydroxyéthylamino-2-nitro-4-(éthyl)(β-hydroxyéthyl)-aminobenzène
- 1-amino-3-méthyl-4-β-hydroxyéthylamino-6-nitro-benzène
- 1-amino-2-nitro-4-β-hydroxyéthylamino-5-chloro-benzène
- 1,2-diamino-4-nitro-benzène
- 1-amino-2-β-hydroxyéthylamino-5-nitro-benzène
- 1,2-bis-(β-hydroxyéthylamino)-4-nitro-benzène
- 1-amino-2-[tris-(hydroxyméthyl)-méthylamino]-5-nitro-benzène
- 1-hydroxy-2-amino-5-nitro-benzène
- 1-hydroxy-2-amino-4-nitro-benzène
- 1-hydroxy-3-nitro-4-amina-benzène
- 1-hydroxy-2-amino-4,6-dinitro-benzène
- 1-β-hydroxyéthytoxy-2-β-hydroxyéthylamino-5-nitro-benzène
- 1-méthoxy-2-β-hydroxyéthylamino-5-nitro-benzène
- 1-β-hydroxyéthyloxy-3-méthylamino-4-nitrobenzène
- 1-β,γ-dihydroxypropyloxy-3-méthylamino-4-nitro-benzène
- 1-β-hydroxyéthylamino-4-β,γ-dihydroxypropyloxy-2-nitro-benzène
- 1-β,γ-dihydroxypropylamino-4-trifluorométhyl-2-nitro-benzène
- 1-β-hydroxyéthylamino-4-trifluorométhyl-2-nitro-benzène
- 1-β-hydroxyéthylamino-3-méthyl-2-nitro-benzène
- 1-β-aminoéthylamino-5-méthoxy-2-nitro-benzène
- 1-hydroxy-2-chloro-6-éthylamino-4-nitro-benzène
- 1-hydroxy-2-chloro-6-amino-4-nitro-benzène
- 1-hydroxy-6-[bis-(P-hydroxyéthyl)-amino]-3-nitro-benzène
- 1-β-hydroxyéthylamino-2-nitro-benzène

- 1-hydroxy-4-β-hydroxyéthylamino-3-nitro-benzène.

Parmi les colorants directs azoïques on peut citer les colorants azoïques cationiques décrits dans les demandes de brevets WO 95/15144, WO-95/01772 et EP-714954 dont le contenu fait partie intégrante de l'invention.
On peut également citer parmi les colorants directs azoïques, les colorants suivants, décrits dans le COLOUR INDEX INTERNATIONAL 3e édition :
-Disperse Red 17; Acid Yellow 9; Acid Black 1; Basic Red 22; Basic Red 76; Basic Yellow 57; Basic Brown 16; Acid Yellow 36; Acid Orange 7; Acid Red 33; Acid Red 35; Basic Brown 17; Acid Yellow 23; Acid Orange 24; Disperse Black 9.
On peut aussi citer : le 1-(4'-aminodiphénylazo)-2-méthyl-4-[bis-(β-hydroxyéthyl) amino]-benzène et l'acide 4-hydroxy-3-(2-méthoxyphénylazo)-1-naphtalène sulfonique.
Parmi les colorants directs méthiniques, on peut citer plus particulièrement les colorants méthiniques cationiques tels que le Basic Red 14, le Basic Yellow 13 et le Basic Yellow 29.
Parmi les colorants directs quinoniques on peut citer les colorants suivants: -Disperse Red 15; Solvent Violet 13; Acid Violet 43; Disperse Violet 1; Disperse Violet 4; Disperse Blue 1; Disperse Violet 8; Disperse Blue 3; Disperse Red 11; Acid Blue 62; Disperse Blue 7; Basic Blue 22; Disperse Violet 15; Basic Blue 99, ainsi que les composés suivants :

- 1-N-méthylmorpholiniumpropylamino-4-hydroxy-anthraquinone
- 1-aminopropylamino-4-méthylamino-anthraquinone
- 1-aminopropylaminoanthraquinone
- 5-β-hydroxyéthyl-1,4-diamino-anthraquinone
- 2-aminoéthylaminoanthraquinone
- 1,4-bis-(β,γ-dihydroxypropilamino)-anthraquinone.

Parmi les colorants directs aziniques on peut citer les composés suivants tels que le Basic Blue 17 et le Basic Red 2.
Parmi les colorants directs triarylméthaniques, on peut citer les composés suivants : -Basic Green 1; Acid blue 9; Basic Violet 3; Basic Violet 14; Basic Blue 7; Acid Violet 49; Basic Blue 26; Acid Blue 7.
Parmi les colorants directs indoaminiques, on peut citer les composés suivants :

- 2-β-hydroxyéthylamino-5-[bis-( -4'-hydroxyéthyl)-amino]-anilino-1,4-benzoquinone ;
- 2-β-hydroxyéthylamino-5-(2'-méthoxy-4'-amino)-anilino-1,4-benzoquinone;
- 3-N(2'-chloro-4'-hydroxy)-phényl-acétylamino-6-méthoxy-1,4-benzoquinone imine;
- 3-N(3'-chloro-4'-méthylamino)-phényl-uréido-6-méthyl-1,4-benzoquinone imine;
- 3-[4'-N-(éthyl,carbamylméthyl)-amino]-phényl-uréido-6-méthyl-1,4-benzo-quinone imine.

Parmi les colorants directs naturels, on peut citer la lawsone, la juglone, l'alizarine, la purpurine, l'acide carminique, l'acide kermésique, la purpurogalline, le protocatéchaldéhyde, l'indigo, l'isatine, la curcumine, la spinulosine, l'apigénidine. On peut également utiliser les extraits ou décoctions contenant ces colorants naturels et notamment les cataplasmes ou extraits à base de henné.
Dans le cas d'une teinture éclaircissante, les fibres sont préalablement teintes par au moins un colorant direct en présence d'un oxydant.
Ainsi, dans le premier compartiment du kit où l'on préconise d'utiliser une teinture éclaircissante, on trouvera au moins un colorant direct.
Parmi les colorants directs que l'on préfère utiliser en teinture éclaircissante, on peut citer les colorants méthiniques cationiques décrits ci-dessus, et plus particulièrement encore des colorants cationiques tels que ceux décrits dans la demande de brevet européen de la demanderesse N°-1025834 qui, en milieu oxydant, génèrent des nuances très chromatiques.
Lesdits colorants directs cationiques utilisés de préférence en teinture directe éclaircissante ou non éclaircissante sont notamment choisis parmi ceux de formule (I) suivante, et ceux de formules (II)a, (II)b, (III)a, (III)b, et (IV) à (VH), suivantes et leurs formes mésomères :

(i) colorants de formules (I), (II)a, (II)b, (III)a, (III)b:

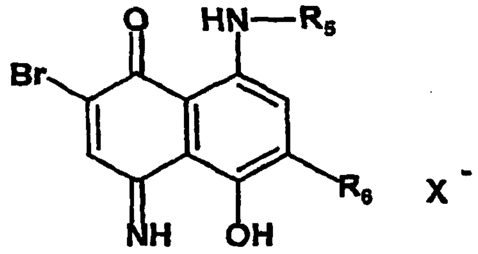

(I)

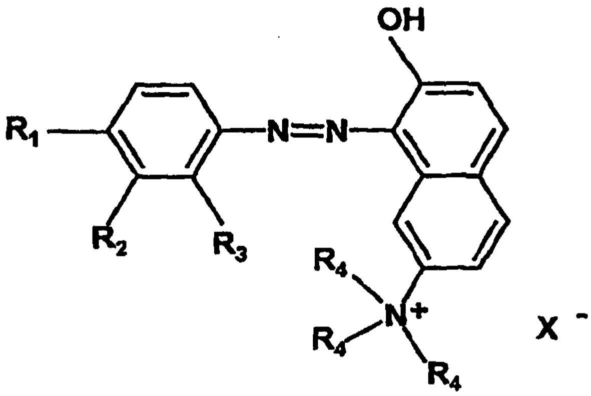

(II)a

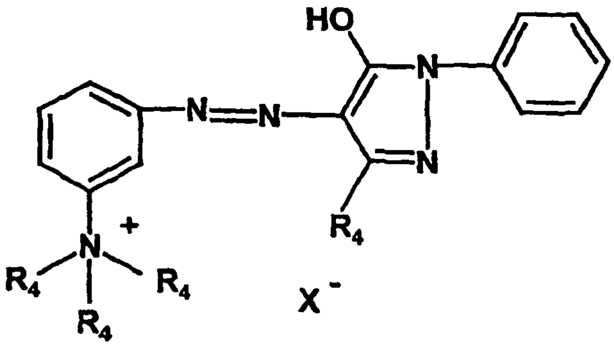

(II)b

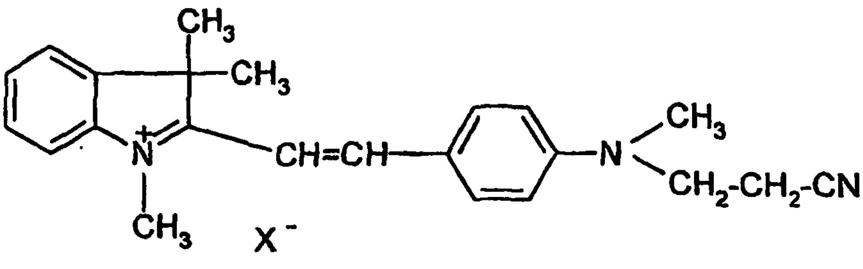

(III)a

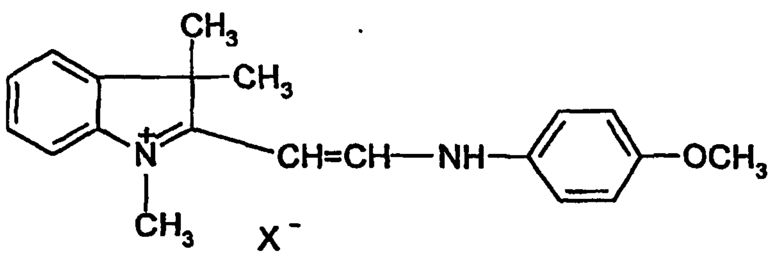

(III)b

formules (I), (II)a, (II)b, (III)a, (III)b dans lesquelles:

$R_1$ représente un atome d'hydrogène ou un radical amino ;
$R_2$ représente un atome d'hydrogène ou un groupement nitro ;
$R_3$ représente un atome d'hydrogène, un groupement nitro ou un radical alcoxy en $C_1$-$C_4$;
$R_4$ représente un radical alkyle en $C_1$-$C_4$ ;
$R_5$ représente un atome d'hydrogène ou un groupement paratrialkyl$C_1$-$C_4$ ammoniophényle ;
$R_6$ représente un atome de brome ou un groupement NHparatrialkyle$C_1$-$C_4$ ammoniophényle ;
X- représente un anion de préférence choisi parmi le chlorure, le méthyl sulfate et l'acétate;

(ii) colorants de formules (IV), (V), (VI), (VI'), (VII) :

**a) les composés de formule (IV) suivante:**

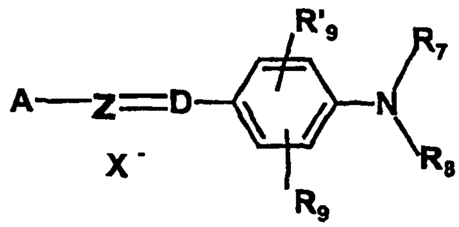

(IV)

dans laquelle :

Z et D représentent, identiques ou différents, un atome d'azote ou le radical -CH-,
$R_7$ et $R_8$, identiques ou différents, représentent un atome d'hydrogène ; un radical alkyle en $C_1$-$C_4$ pouvant être substitué par un radical -CN, -OH ou -$NH_2$ ou forment avec un atome de carbone du cycle benzénique un hétérocycle éventuellement oxygéné ou azoté, pouvant être substitué par un ou plusieurs radicaux alkyle en $C_1$-$C_4$ ; un radical 4'-aminophényle,
$R_9$ et $R'_9$, identiques ou différents, représentent un atome d'hydrogène ou d'halogène choisi parmi le chlore, le brome, l'iode et le fluor, un radical cyano, alkyl en $C_1$-$C_4$, alcoxy en $C_1$-$C_4$ ou acétyloxy,
X - représente un anion de préférence choisi parmi le chlorure, le méthyl sulfate et l'acétate,

A représente un groupement choisi par les structures A1 à A19 suivantes :

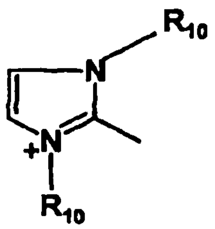

$A_1$

;

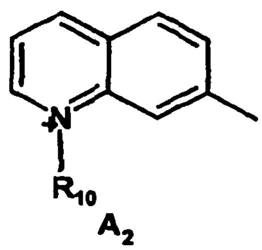

$A_2$

;

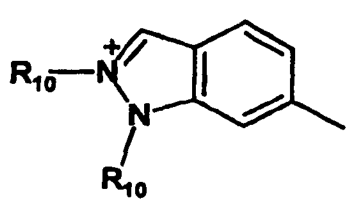

$A_3$

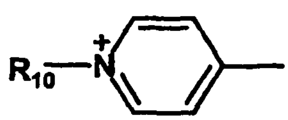

$A_4$

;

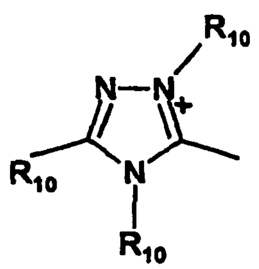

$A_5$

;

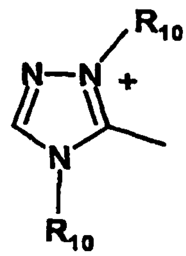

$A_6$

;

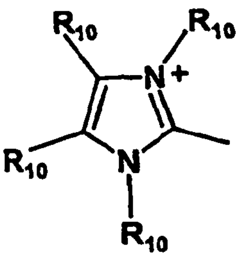

$A_7$

;

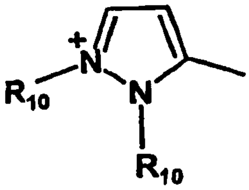

$A_8$

;

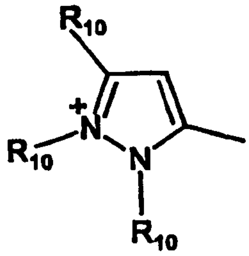

$A_9$

;

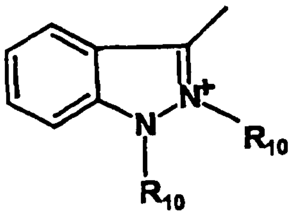

$A_{10}$

;

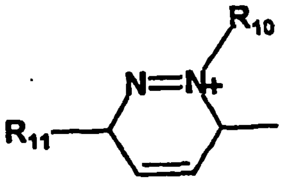

$A_{11}$

;

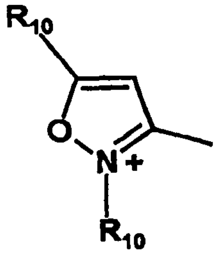

$A_{12}$

;

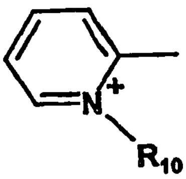

$A_{13}$

;

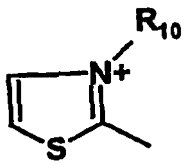

$A_{14}$

;

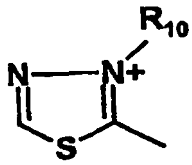

$A_{15}$

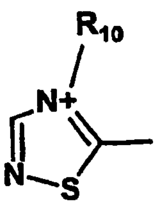

$A_{16}$

;

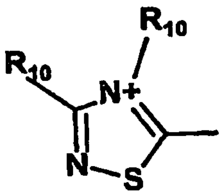

$A_{17}$

;

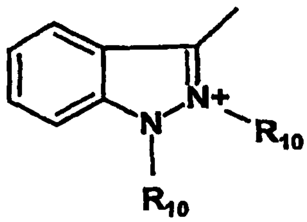

$A_{18}$

et

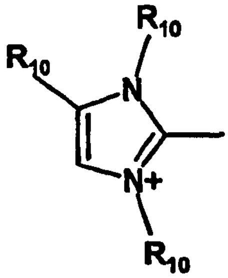

$A_{19}$

dans lesquelles $R_{10}$ représente un radical alkyle en $C_1$-$C_4$ pouvant être substitué par un radical hydroxyle et $R_{11}$ représente un radical alcoxy en $C_1$-$C_4$,;

**b) les composés de formule (V) suivante :**

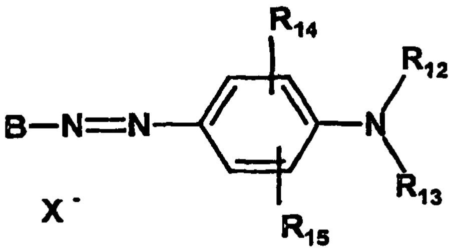

(V)

dans laquelle :

$R_{12}$ représente un atome d'hydrogène ou un radical alkyle en $C_1$-$C_4$,
$R_{13}$ représente un atome d'hydrogène, un radical alkyle pouvant être substitué par un radical -CN ou par un groupement amino, un radical 4'-aminophényle ou forme avec $R_{12}$ un hétérocycle éventuellement oxygéné et/ou azoté pouvant être substitué par un radical alkyle en $C_1$-$C_4$,
$R_{14}$ et $R_{15}$, identiques ou différents, représentent un atome d'hydrogène, un atome d'halogène tel que le brome, le chlore, l'iode ou le fluor, un radical alkyle en $C_1$-$C_4$ ou alcoxy en $C_1$-$C_4$, un radical -CN,
$X^-$ représente un anion de préférence choisi parmi le chlorure, le méthyl sulfate et l'acétate,

B représente un groupement choisi par les structures B1 à B6 suivantes :

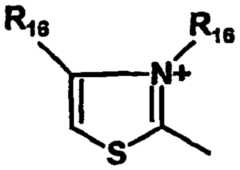

B1

;

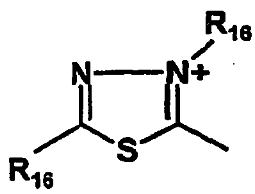

B2

;

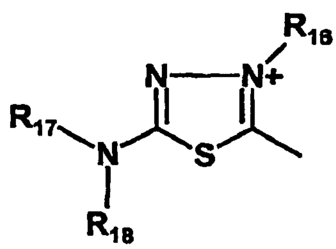

B3

;

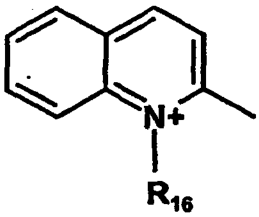

B4

;

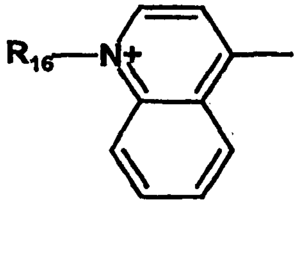

B5

et

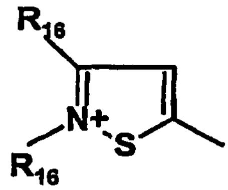

B6

;

dans lesquelles $R_{16}$ représente un radical alkyle en $C_1$-$C_4$, $R_{17}$ et $R_{18}$, identiques ou différents, représentent un atome d'hydrogène ou un radical alkyle en $C_1$-$C_4$ ;

**c) les composés de formules (VI) et (VI') suivantes :**

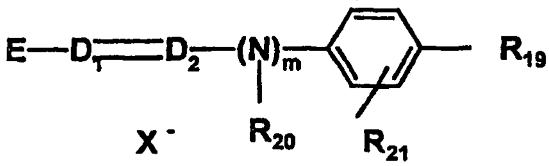

(VI)

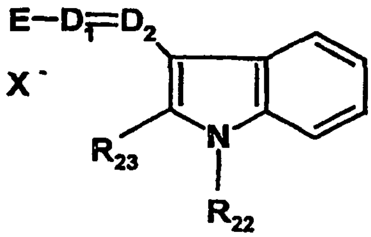

(VI')

dans lesquelles :

$R_{19}$ représente un atome d'hydrogène, un radical alcoxy en $C_1$-$C_4$, un atome d'halogène tel que le brome, le chlore, l'iode ou le fluor ou un radical amino,
$R_{20}$ représente un atome d'hydrogène, un radical alkyle en $C_1$-$C_4$ ou forme avec un atome de carbone du cycle benzénique un hétérocycle éventuellement oxygéné et/ou substitué par un ou plusieurs groupements alkyle en $C_1$-$C_4$,
$R_{21}$ représente un atome d'hydrogène ou d'halogène tel que le brome, le chlore, l'iode ou le fluor,
$R_{22}$ et $R_{23}$, identiques ou différents, représentent un atome d'hydrogène ou un radical alkyle en $C_1$-$C_4$,
$D_1$ et $D_2$, identiques ou différents, représentent un atome d'azote ou le groupement - CH,
m=0 ou 1,
$X^-$ représente un anion de préférence choisi parmi le chlorure, le méthyl sulfate et l'acétate,
E représente un groupement choisi par les structures E1 à E8 suivantes :

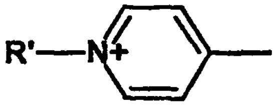

E1

;

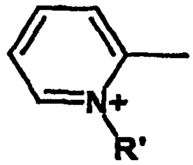

E2

;

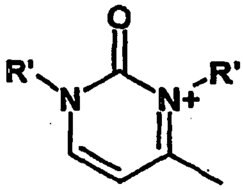 ; 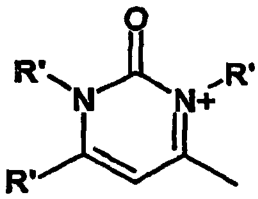 ; 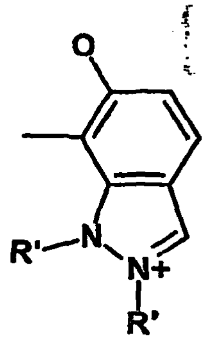 ;

E3

E4

E5

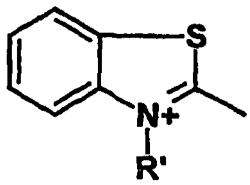 ; 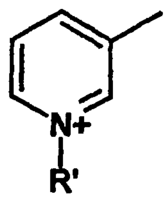 et 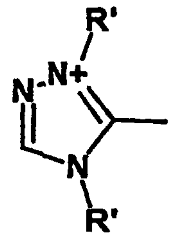

E6

E7

E8

dans lesquelles R' représente un radical alkyle en $C_1$-$C_4$ ;
lorsque m = 0 et que $D_{13}$ représente un atome d'azote, alors E peut également désigner un groupement de structure E9 suivante :

E9 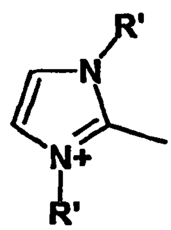

;

dans laquelle R' représente un radical alkyle en $C_1$-$C_4$.

**d) les composés de formule (VII) suivante :**

**G-N = N -J** (VII)

dans laquelle :

**le symbole G** représente un groupement choisi parmi les structures $G_1$ à $G_3$ suivantes :

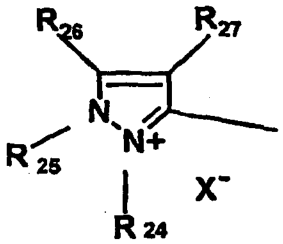

$G_1$

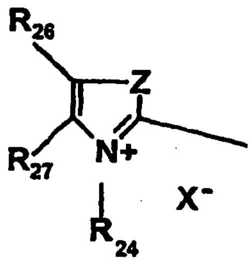

$G_2$

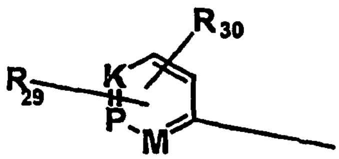

$G_3$

structures $G_1$ à $G_3$ dans lesquelles,

$R_{24}$ désigne un radical alkyle en $C_1$-$C_4$, un radical phényle pouvant être substitué par un radical alkyle en $C_1$-$C_4$ ou un atome d'halogène choisi parmi le chlore, le brome, l'iode et le fluor ;
$R_{25}$ désigne un radical alkyle en $C_1$-$C_4$ ou un radical phényle;
$R_{26}$ et $R_{27}$, identiques ou différents, représentent un radical alkyle en $C_1$-$C_4$, un radical phényle, ou forment ensemble dans $G_1$ un cycle benzénique substitué par un ou plusieurs radicaux alkyle en $C_1$-$C_4$, alcoxy en $C_1$-$C_4$, ou $NO_2$, ou forment ensemble dans $G_2$ un cycle benzénique éventuellement substitué par un ou plusieurs radicaux alkyle en $C_1$-$C_4$, alcoxy en $C_1$-$C_4$, ou $NO_2$;
$R_{26}$ peut désigner en outre un atome d'hydrogène;
Z désigne un atome d'oxygène, de soufre ou un groupement -$NR_{25}$;
M représente un groupement -CH, -CR (R désignant alkyle en $C_1$-$C_4$), ou -$NR_{28}(X^-)_r$;
K représente un groupement -CH, -CR (R désignant alkyle en $C_1$-$C_4$), ou -$NR_{28}(X^-)_r$;
P représente un groupement -CH, -CR (R désignant alkyle en $C_1$-$C_4$), ou -$NR_{28}(X^-)_r$; r désigne zéro ou 1;
$R_{28}$ représente un atome $O^-$, un radical alcoxy en $C_1$-$C_4$, ou un radical alkyle en $C_1$-$C_4$;
$R_{29}$ et $R_{30}$, identiques ou différents, représentent un atome d'hydrogène ou d'halogène choisi parmi le chlore, le brome, l'iode et le fluor, un radical alkyle en $C_1$-$C_4$, alcoxy en $C_1$-$C_4$, un radical -$NO_2$;
$X^-$ représente un anion de préférence choisi parmi le chlorure, l'iodure, le méthyl sulfate, l'éthyl sulfate, l'acétate et le perchlorate;

**le symbole J** représente :

- **(a)** un groupement de structure $J_1$ suivante :

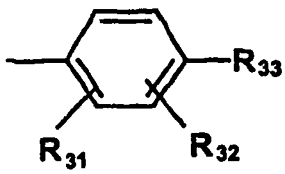

$J_1$

structure $J_1$ dans laquelle,
$R_{31}$ représente un atome d'hydrogène, un atome d'halogène choisi parmi le chlore, le brome, l'iode et

le fluor, un radical alkyle en $C_1$-$C_4$, alcoxy en $C_1$-$C_4$, un radical -OH, - $NO_2$, -$NHR_{34}$, -$NR_{35}R_{36}$, -NH-COalkyle en $C_1$-$C_4$, ou forme avec $R_{32}$ un cycle à 5 ou 6 chaînons contenant ou non un ou plusieurs hétéroatomes choisis parmi l'azote, l'oxygène ou le soufre;

$R_{32}$ représente un atome d'hydrogène, un atome d'halogène choisi parmi le chlore, le brome, l'iode et le fluor, un radical alkyle en $C_1$-$C_4$, alcoxy en $C_1$-$C_4$

ou forme avec $R_{33}$ ou $R_{34}$ un cycle à 5 ou 6 chaînons contenant ou non un ou plusieurs hétéroatomes choisis parmi l'azote, l'oxygène ou le soufre;

$R_{33}$ représente un atome d'hydrogène, un radical -OH, un radical -$NHR_{34}$, un radical - $NR_{35}R_{36}$;

$R_{34}$ représente un atome d'hydrogène, un radical alkyle en $C_1$-$C_4$, un radical monohydroxyalkyle en $C_1$-$C_4$, polyhydroxyalkyle en $C_2$-$C_4$, un radical phényle;

$R_{35}$ et $R_{36}$, identiques ou différents, représentent un radical alkyle en $C_1$-$C_4$, un radical monohydroxyalkyle en $C_1$-$C_4$, polyhydroxyalkyle en $C_2$-$C_4$;

- (**b**) un groupement hétérocyclique azoté à 5 ou 6 chaînons susceptible de renfermer d'autres hétéroatomes et/ou des groupements carbonylés et pouvant être substitué par un ou plusieurs radicaux alkyle en $C_1$-$C_4$, amino ou phényle,

  et notamment un groupement de structure $J_2$ suivante :

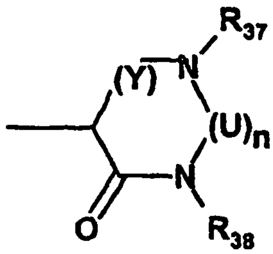

$J_2$

structure $J_2$ dans laquelle,

$R_{37}$ et $R_{38}$, identiques ou différents, représentent un atome d'hydrogène, un radical alkyle en $C_{13}$-$C_{10}$, un radical phényle;

Y désigne le radical -CO- ou le radical

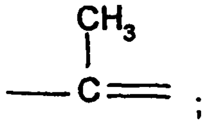

n = 0 ou 1, avec, lorsque n désigne 1, U désigne le radical -CO-.

Parmi les colorants directs cationiques de formule (IV), on peut plus particulièrement citer les composés répondant aux structures (IV1) à (IV54) suivantes :

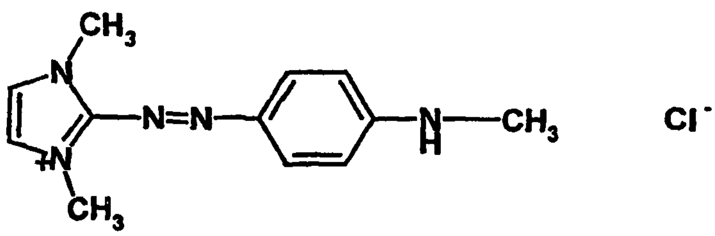

(IV1)

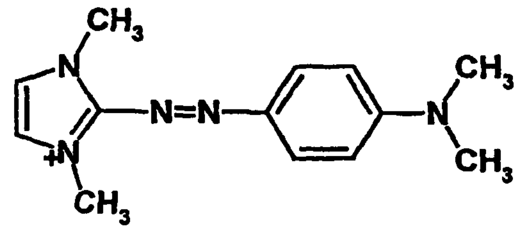

$Cl^-$ (IV2)

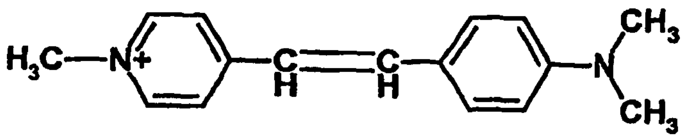

$Cl^-$ (IV3)

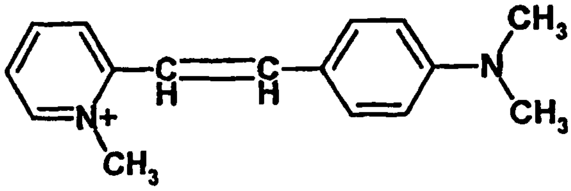

$Cl^-$ (IV4)

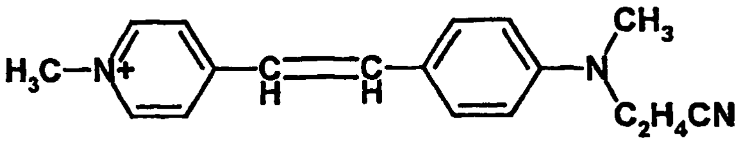

$Cl^-$ (IV5)

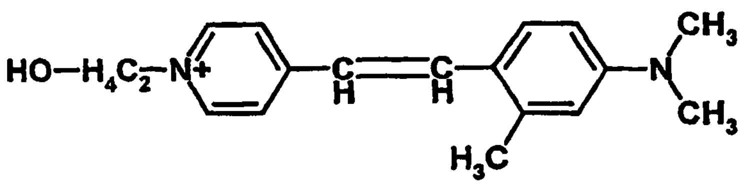

$Cl^-$ (IV6)

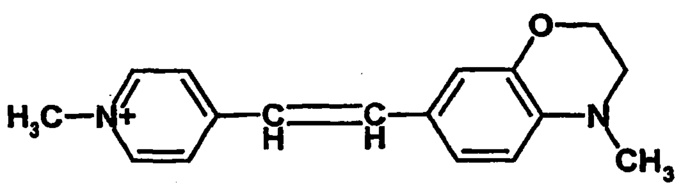

$Cl^-$ (IV7)

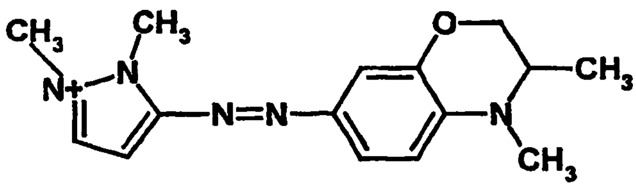

$Cl^-$ (IV8)

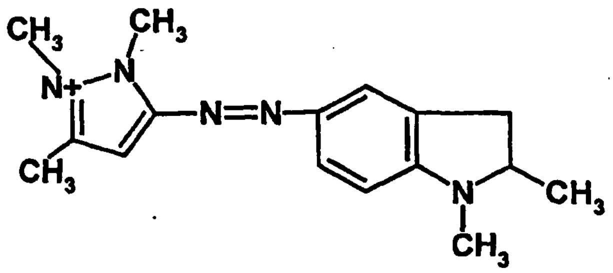

Cl⁻ (IV9)

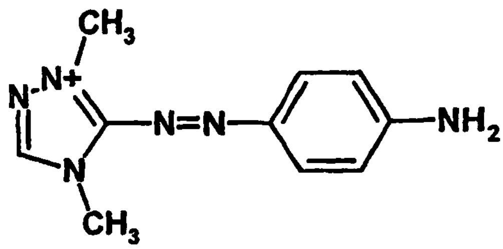

Cl⁻ (IV10)

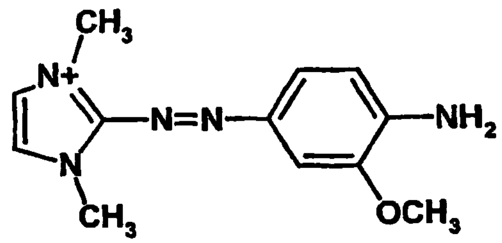

Cl⁻ (IV11)

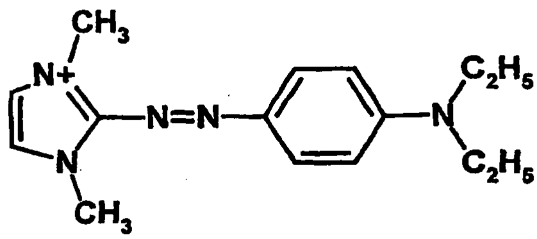

Cl⁻ (IV12)

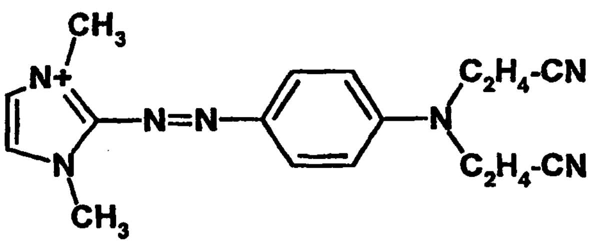

Cl⁻ (IV13)

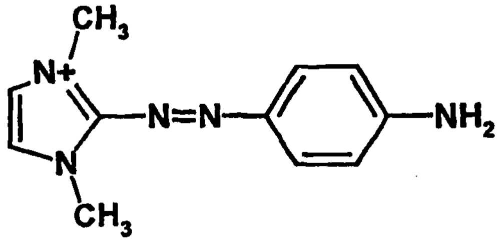

$Cl^-$ (IV14)

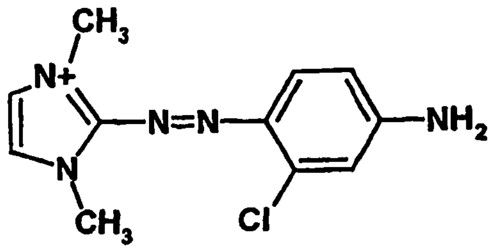

$Cl^-$ (IV15)

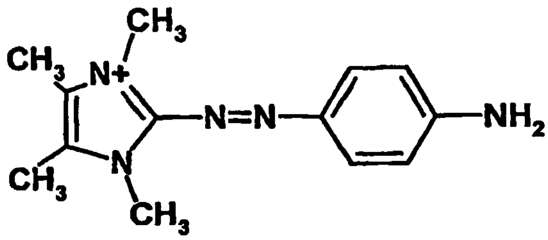

$Cl^-$ (IV16)

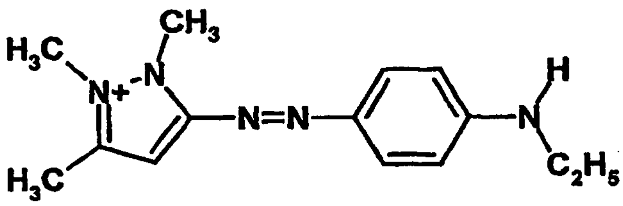

$Cl^-$ (IV17)

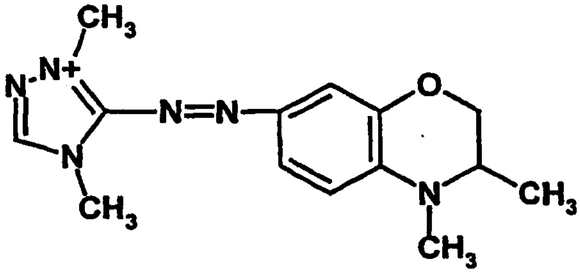

$Cl^-$ (IV18)

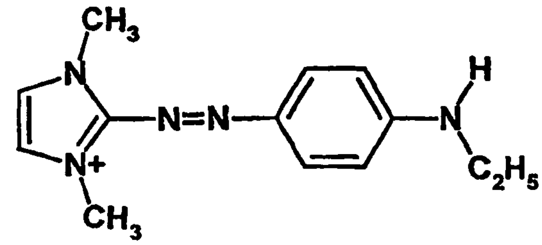

$Cl^-$ (IV19)

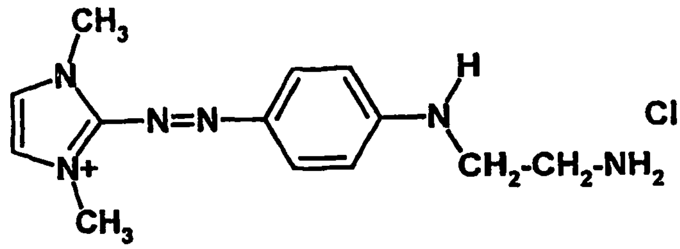

$Cl^-$ (IV20)

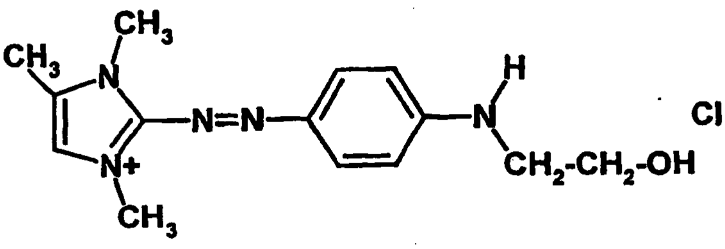

$Cl^-$ (IV21)

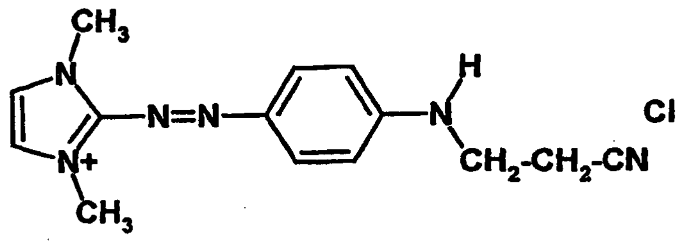

$Cl^-$ (IV22)

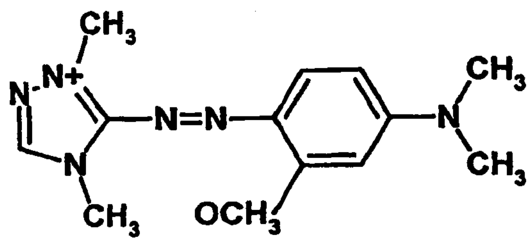

$Cl^-$ (IV23)

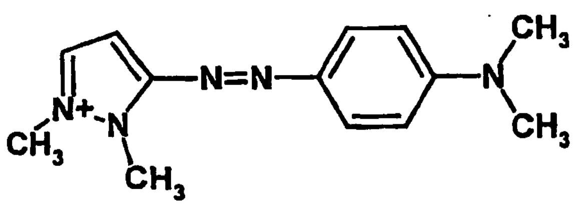

$Cl^-$ (IV24)

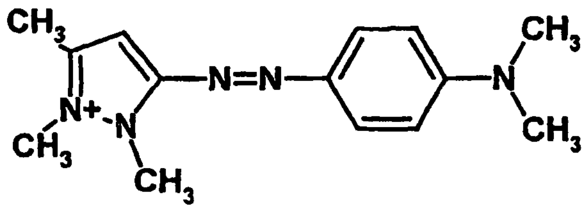

$Cl^-$ (IV25)

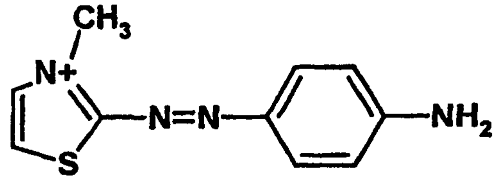

$Cl^-$ (IV26)

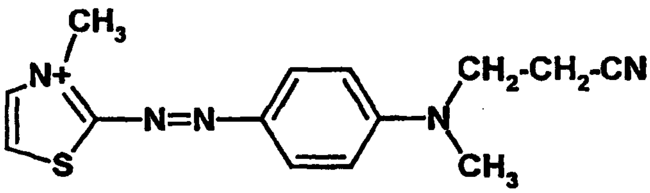

$Cl^-$ (IV27)

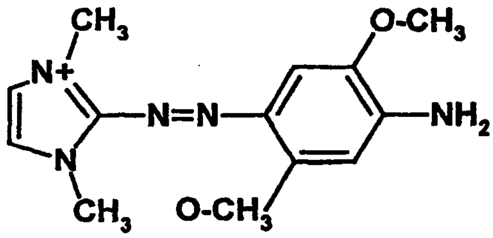

$Cl^-$ (IV28)

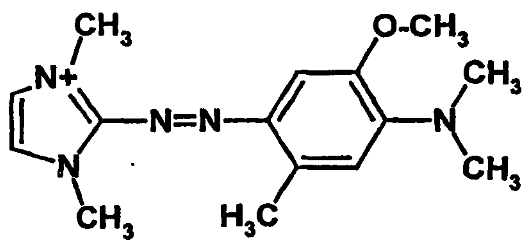

$Cl^-$ (IV29)

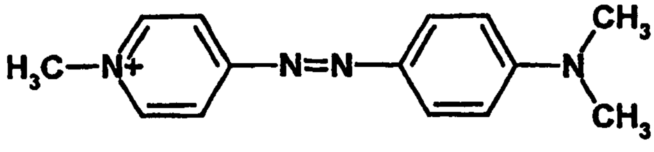

$Cl^-$ (IV30)

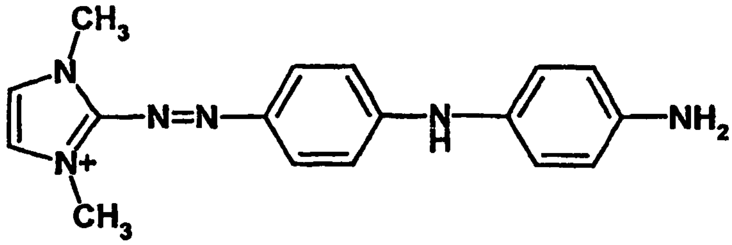

Cl ⁻ (IV31)

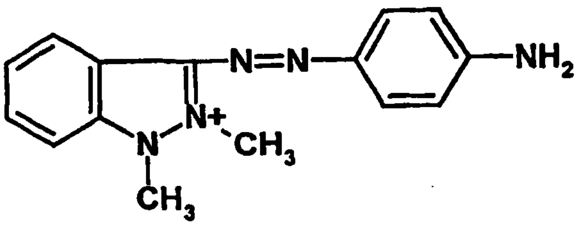

Cl ⁻ (IV32)

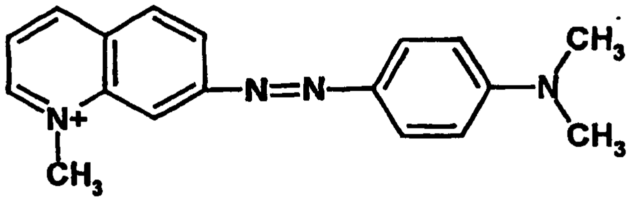

Cl ⁻ (IV33)

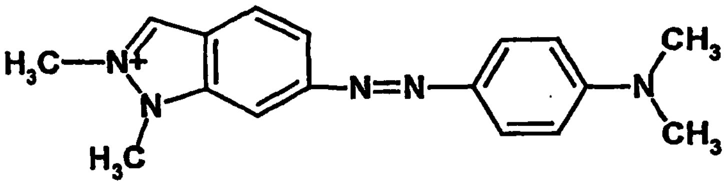

Cl ⁻ (IV34)

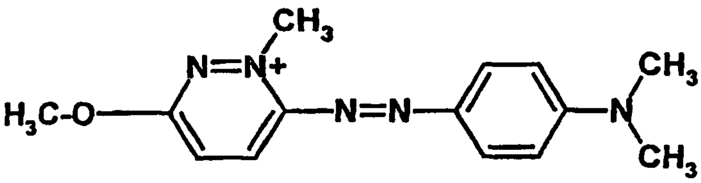

Cl ⁻ (IV35)

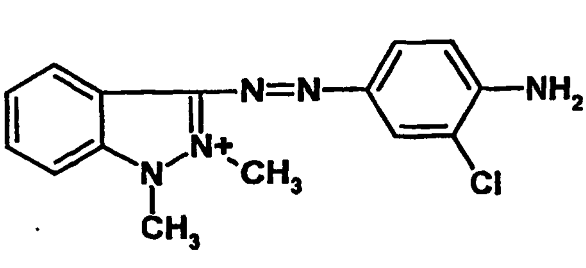

Cl ⁻ (IV36)

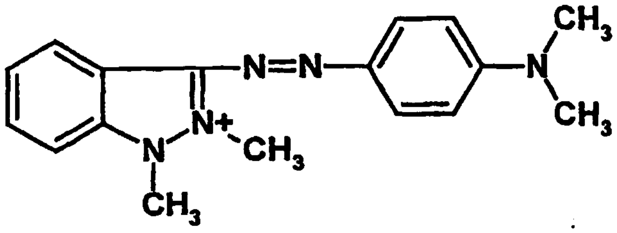

$Cl^-$ (IV37)

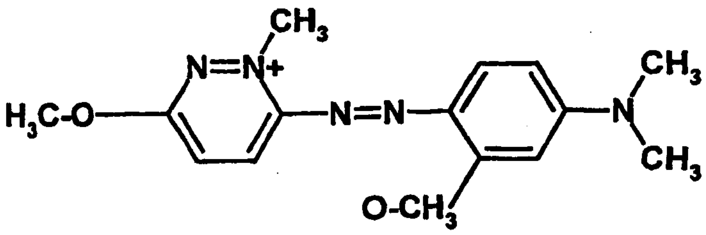

$Cl^-$ (IV38)

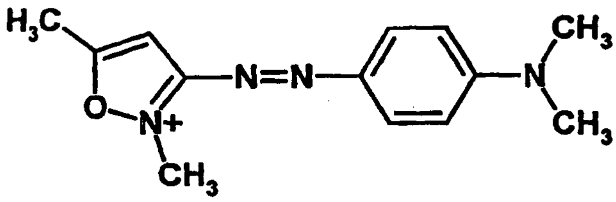

$Cl^-$ (IV39)

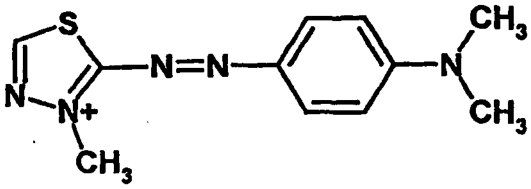

$Cl^-$ (IV40)

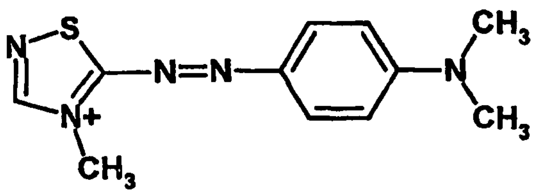

$Cl^-$ (IV41)

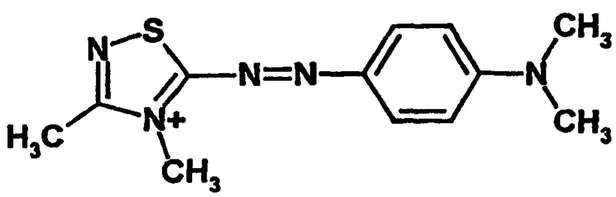

$Cl^-$ (IV42)

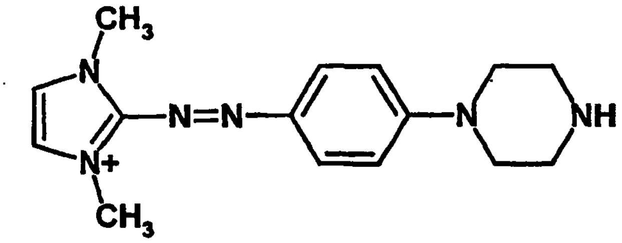

$Cl^-$ (IV43)

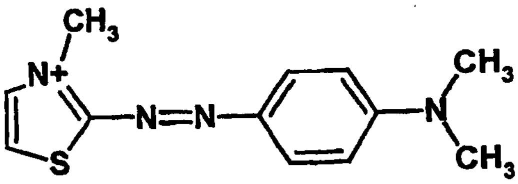

$Cl^-$ (IV44)

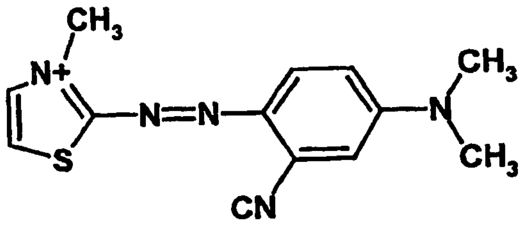

$Cl^-$ (IV45)

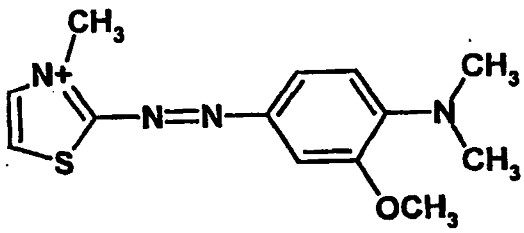

$Cl^-$ (IV46)

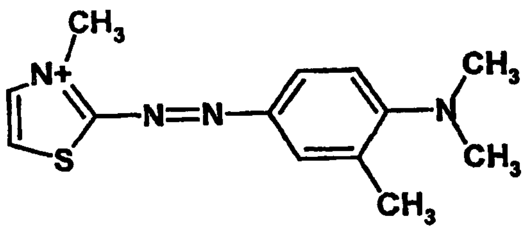

$Cl^-$ (IV47)

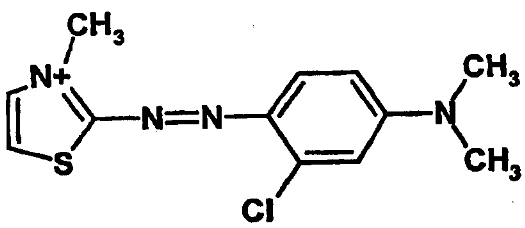

$CH_3SO_4^-$ (IV48)

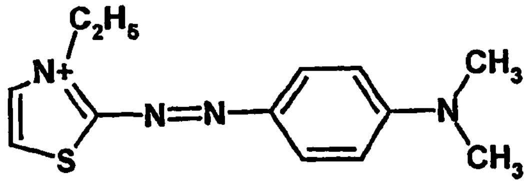

$CH_3SO_4^-$ (IV49)

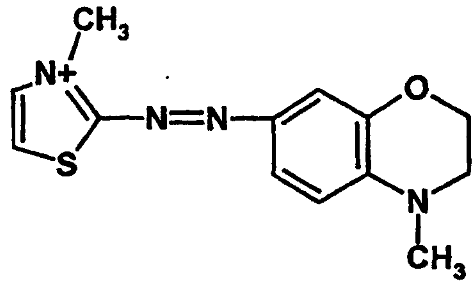

$Cl^-$ (IV50)

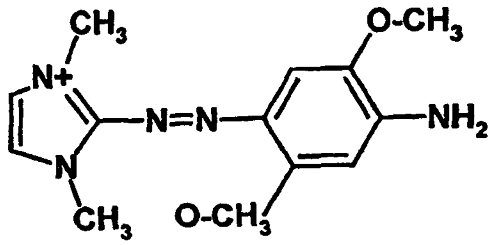

$Cl^-$ (IV51)

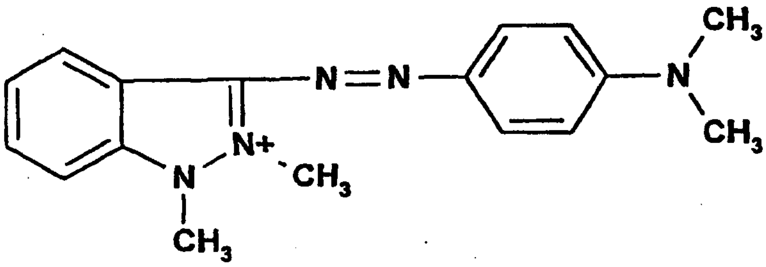

$Cl^-$

(IV52)

;

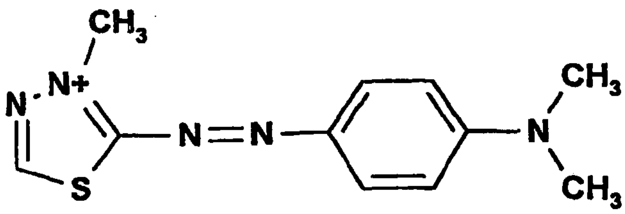

$CH_3SO_4^-$ (IV53)

;

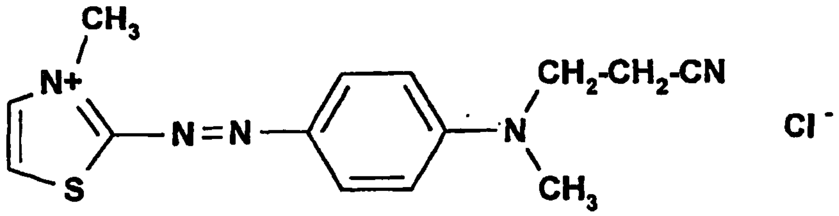 (IV54)

Parmi les composés de structures (IV1) à (IV54) décrits ci-dessus, on préfère tout particulièrement les composés répondant aux structures (IV1), (IV2), (IV14) et (IV31).

Parmi les colorants directs cationiques de formule (V), on peut plus particulièrement citer les composés répondant aux structures (V1) à (V9) suivantes :

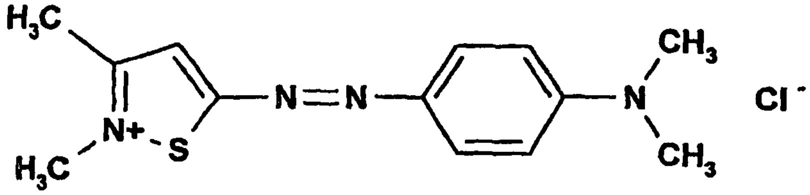 (V1)

;

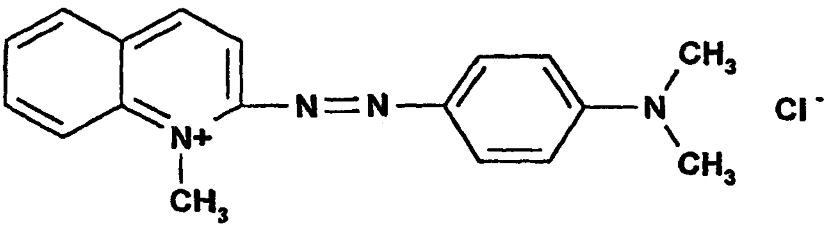 (V2)

;

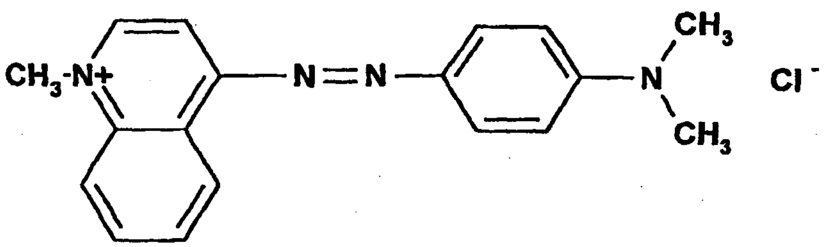 (V3)

;

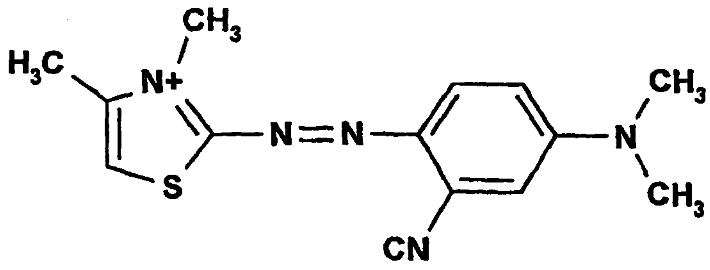 $Cl^-$ (V4)

;

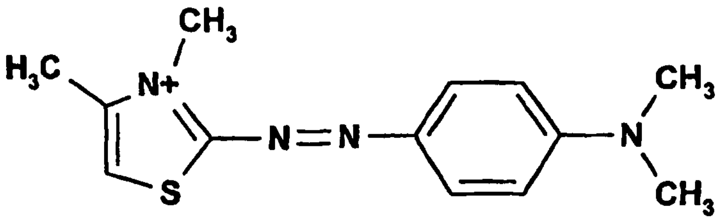 $CH_3SO_4^-$ (V5)

;

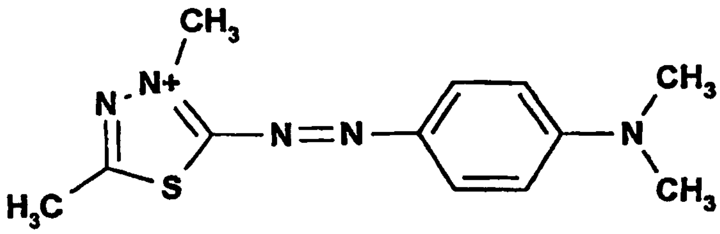 $CH_3SO_4^-$ (V6)

;

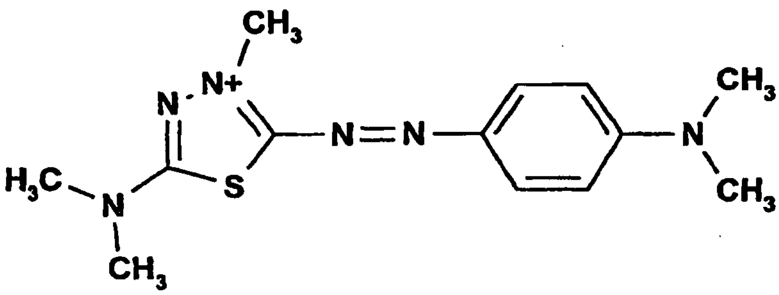 $CH_3SO_4^-$ (V7)

;

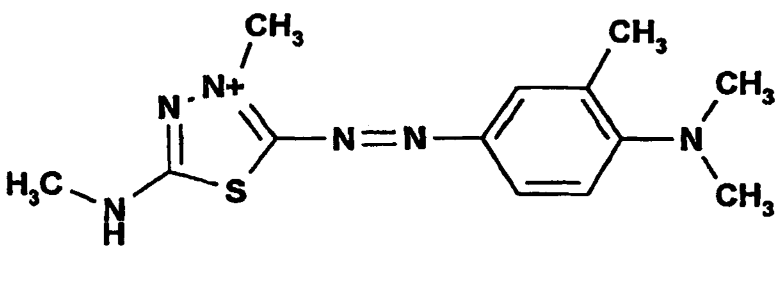 $CH_3SO_4^-$ (V8)

; et

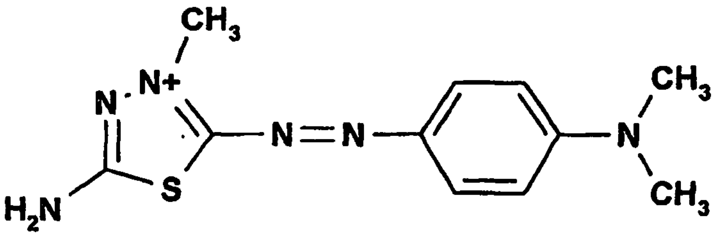

$CH_3SO_4^-$ (V9)

.

Parmi les colorants directs cationiques de formule (VI), utilisables dans les compositions tinctoriales conformes à l'invention, on peut plus particulièrement citer les composés répondant aux structures (VI1) à (VI18) suivantes :

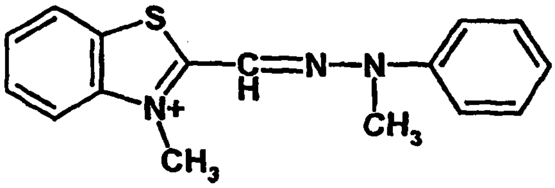

$Cl^-$ (VI1)

;

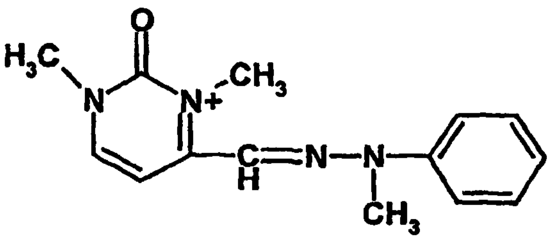

$Cl^-$ (VI2)

;

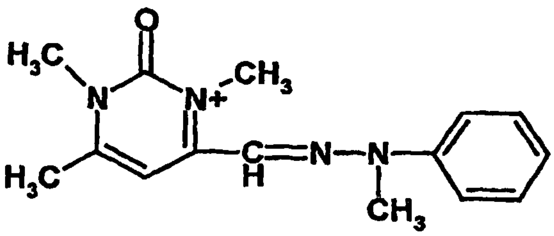

$Cl^-$ (VI3)

;

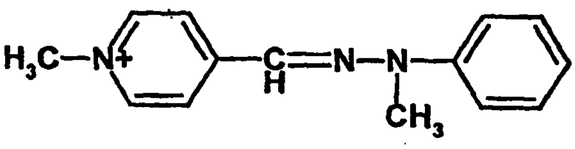

$CH_3SO_4^-$ (VI4)

;

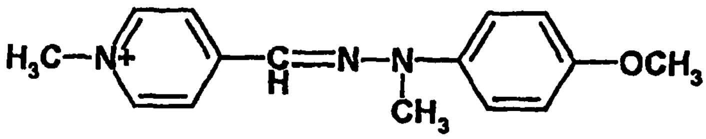

$Cl^-$ (VI5)

;

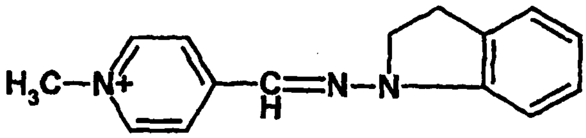

$CH_3SO_4^-$ (VI6)

;

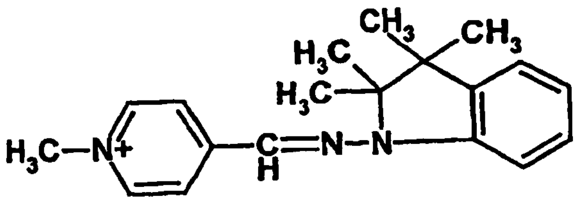

$CH_3SO_4^-$ (VI7)

;

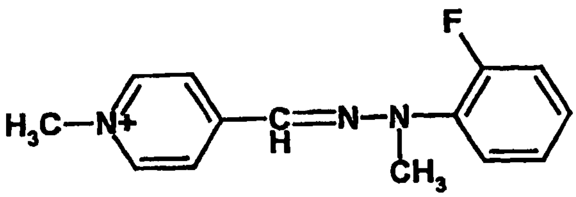

$Cl^-$ (VI8)

;

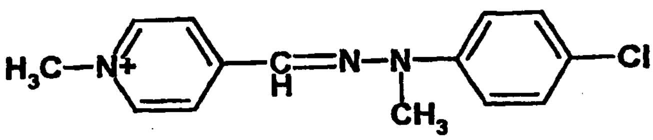

$Cl^-$ (VI9)

;

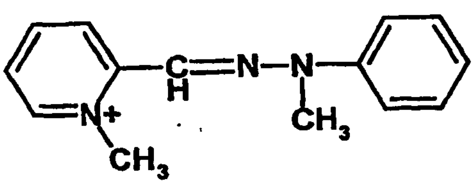

$CH_3SO_4^-$ (VI10)

;

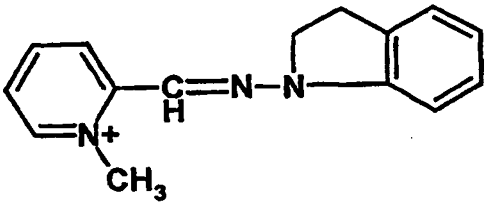

$CH_3SO_4^-$ (VI11)

;

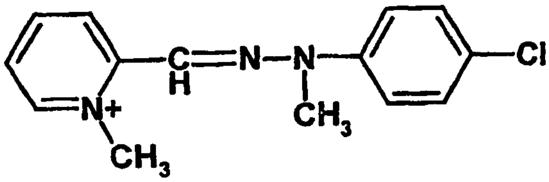

$CH_3SO_4^-$ (VI12)

;

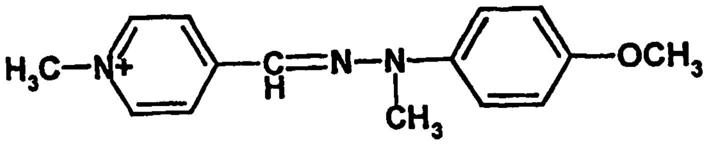

$CH_3SO_4^-$ (VI13)

;

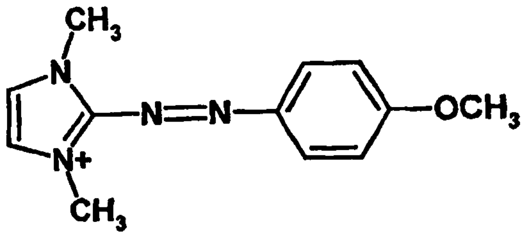

$Cl^-$ (VI14)

;

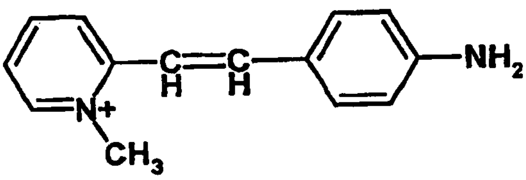

$CH_3COO^-$ (VI15)

;

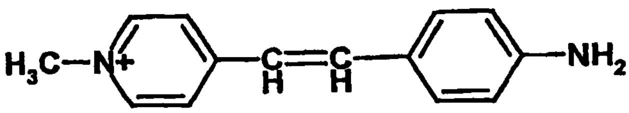

$CH_3COO^-$ (VI16)

;

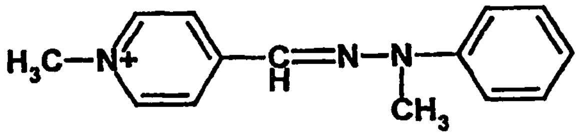

Cl⁻ (VI17)

;

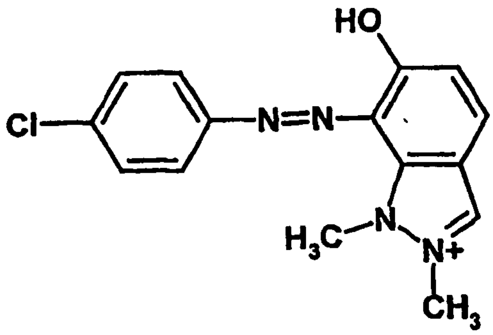

Cl⁻ (VI18)

.

Parmi les composés particuliers de structures (VI1) à (VI18) décrits ci-dessus, on préfère tout particulièrement les composés répondant aux structures (VI4), (VI5) et (VI13).

Parmi les colorants directs cationiques de formule (VI'), on peut plus particulièrement citer les composés répondant aux structures (VI'1) à (VI'3) suivantes :

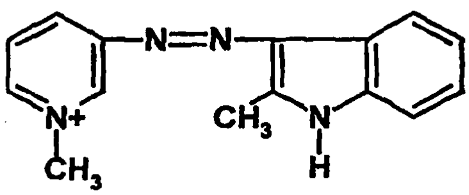

Cl⁻ (VI'1)

;

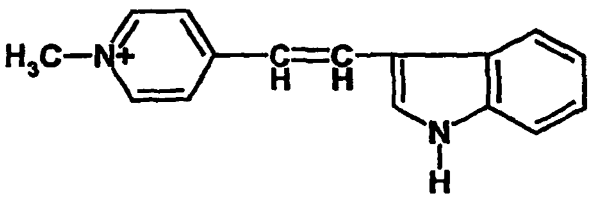

Cl⁻ (VI'2)

;

et

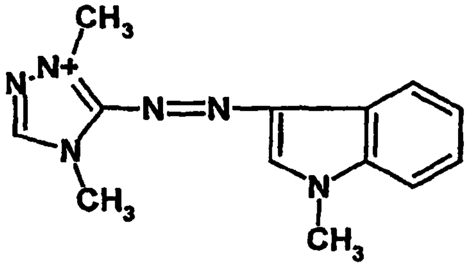

Cl⁻ (VI'3)

Parmi les colorants directs cationiques de formule (VII), on peut citer plus particulièrement les composés de structures ($VII_1$) à ($VII_{77}$) suivantes :

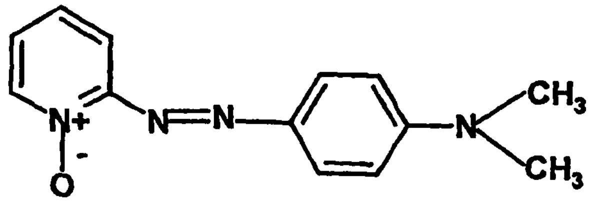

(VII1)

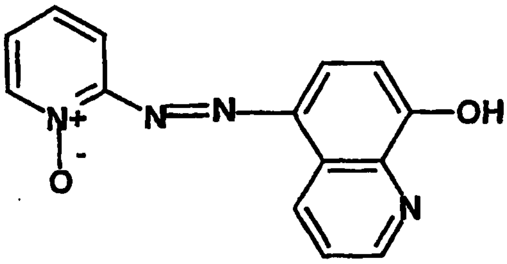

(VII2)

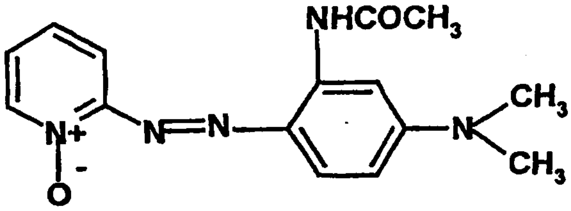

(VII3)

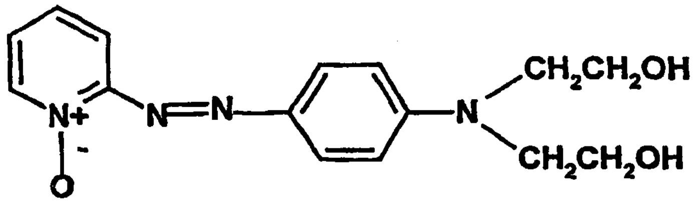

(VII4)

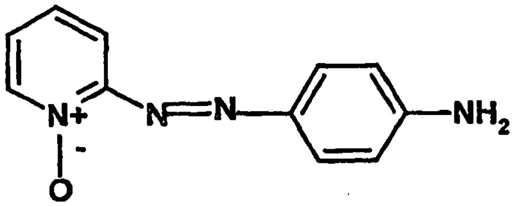

(VII5)

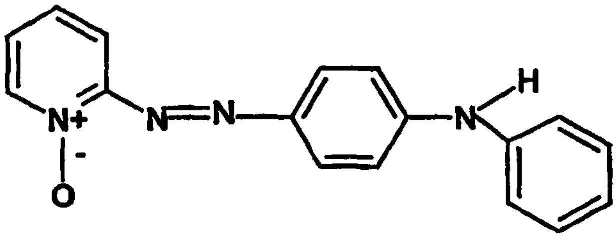

(VII6)

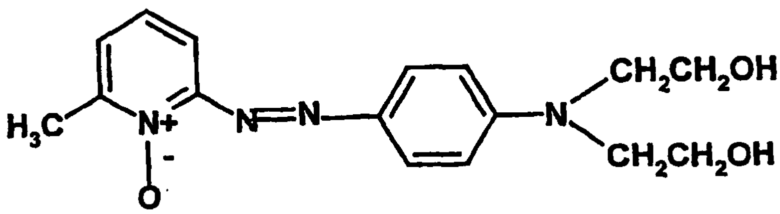

(VII7)

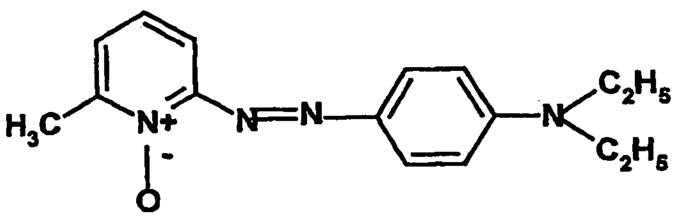

(VII8)

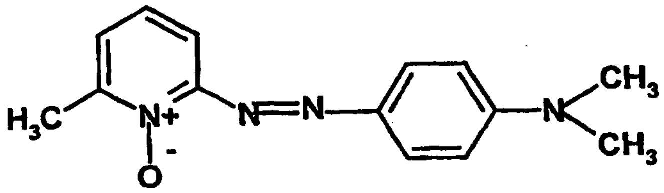

(VII9)

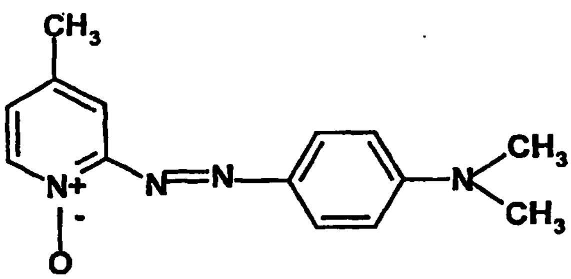

(VII10)

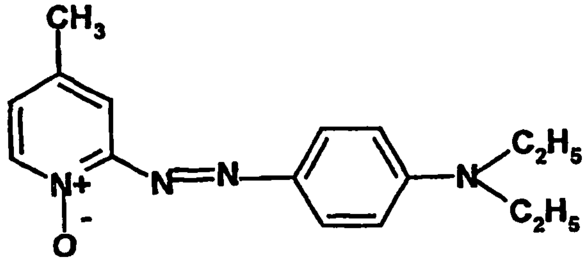

(VII11)

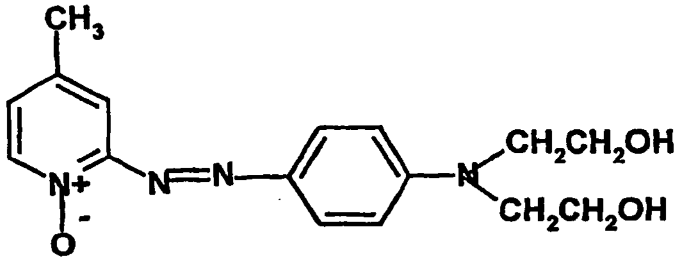

(VII12)

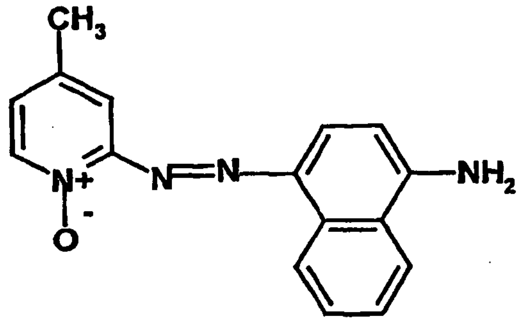

(VII13)

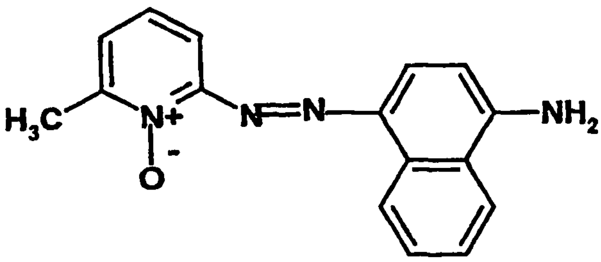

(VII14)

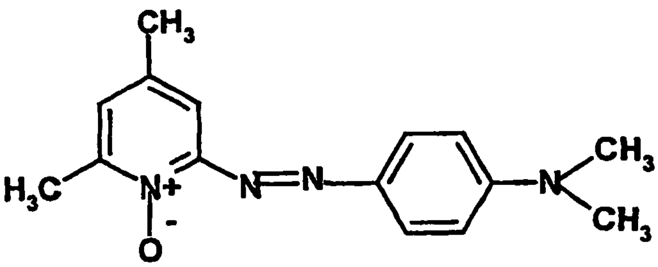

(VII15)

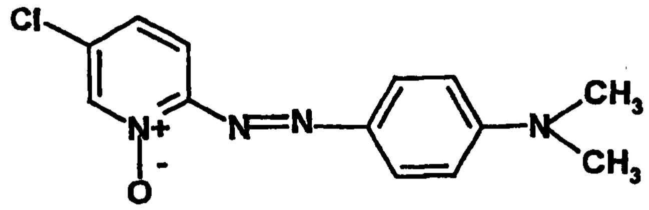

(VII16)

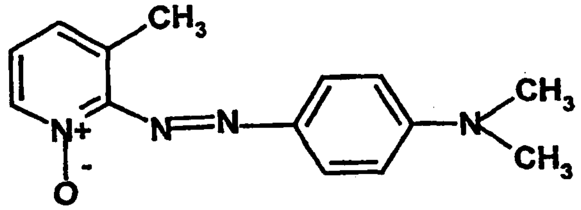

(VII17)

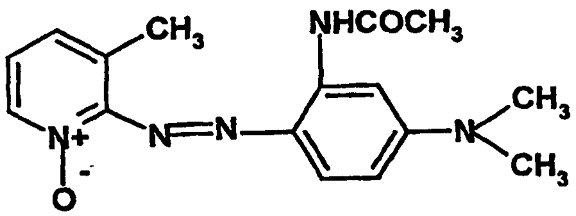

(VII18)

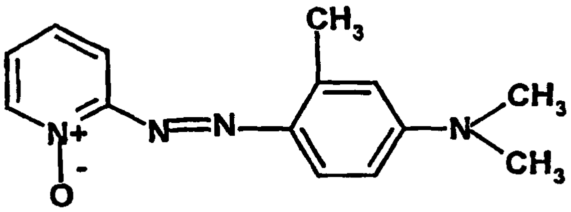

(VII19)

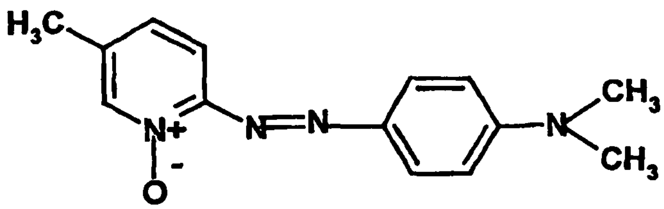

(VII20)

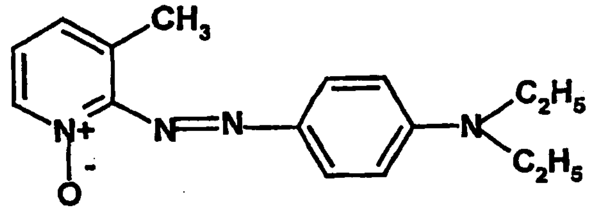

(VII21)

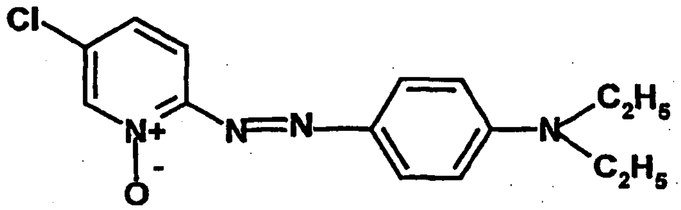

(VII22)

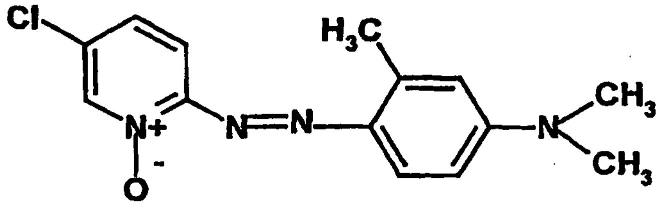

(VII23)

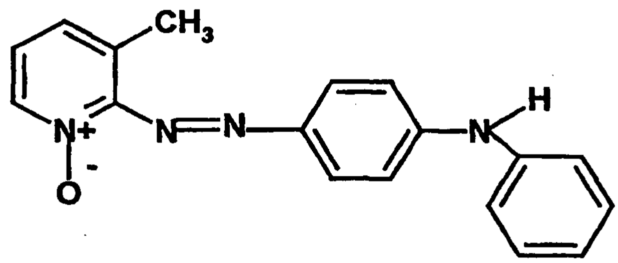

(VII24)

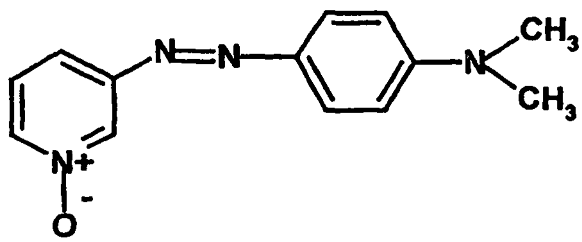

(VII25)

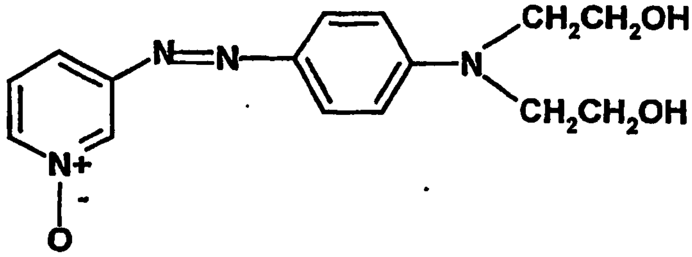

(VII26)

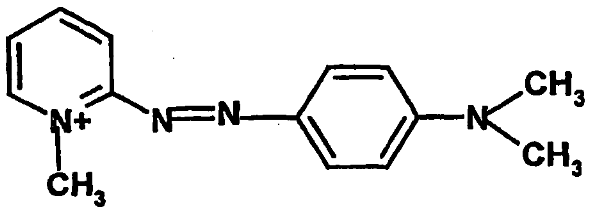

$CH_3SO_4^-$

(VII27)

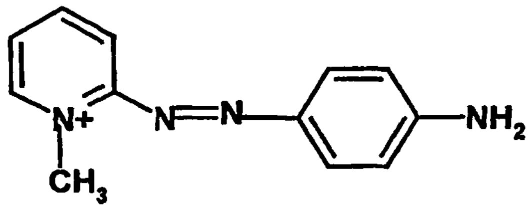

$CH_3SO_4^-$

(VII28)

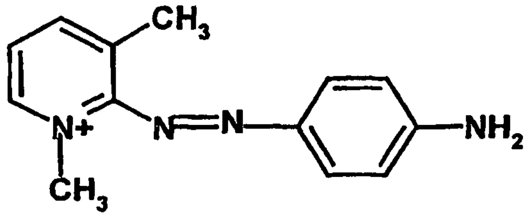

$CH_3SO_4^-$

(VII29)

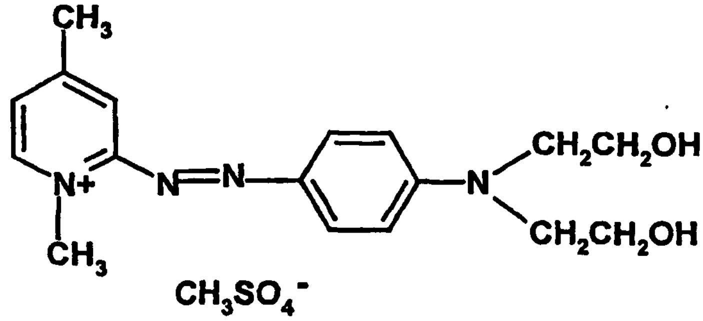

$CH_3SO_4^-$

(VII30)

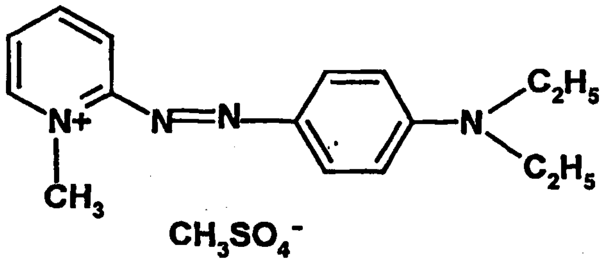

(VII31)

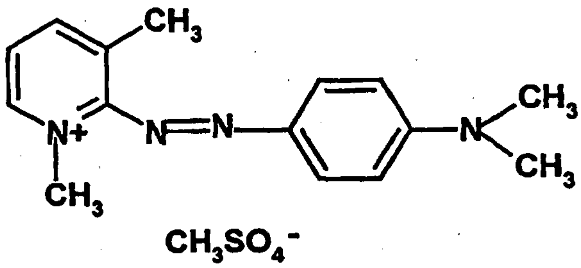

(VII32)

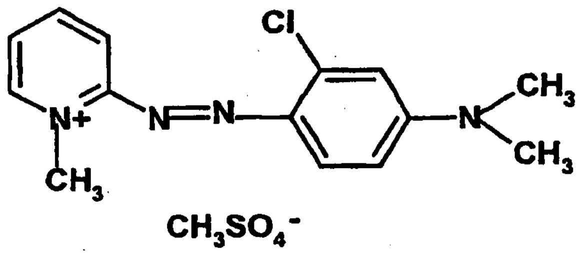

(VII33)

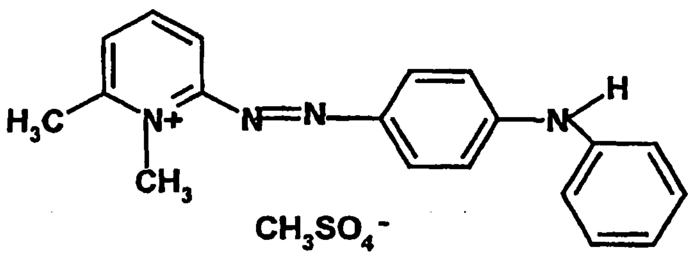

(VII34)

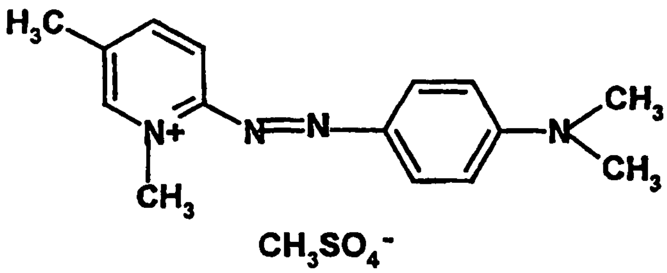

(VII35)

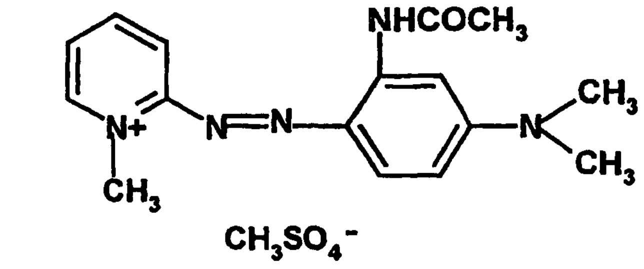

(VII36)

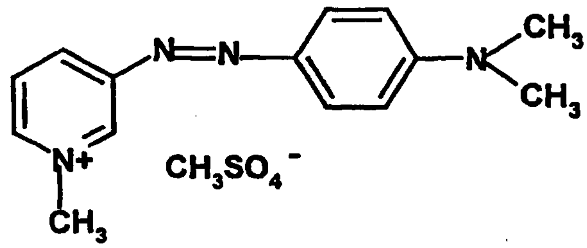

(VII37)

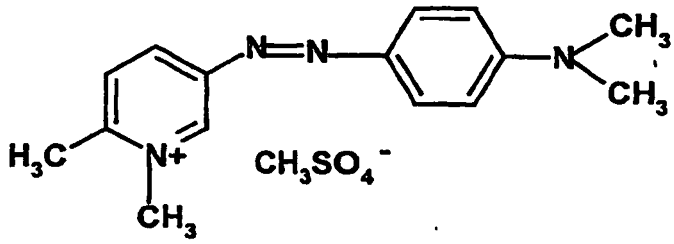

(VII38)

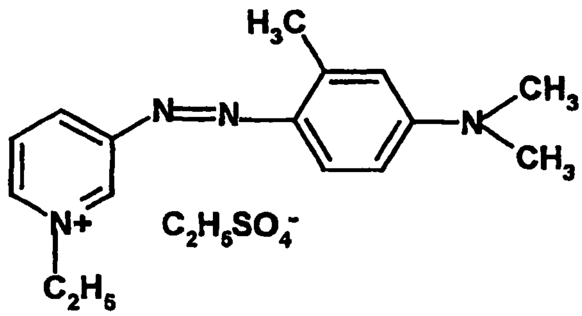

(VII39)

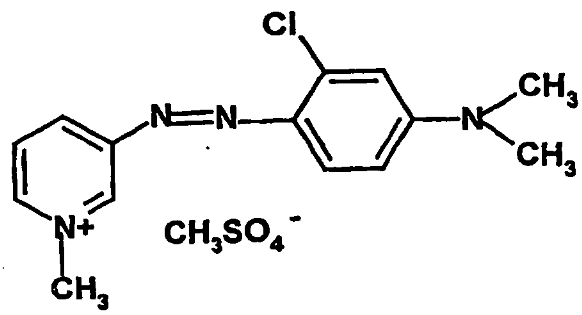

(VII40)

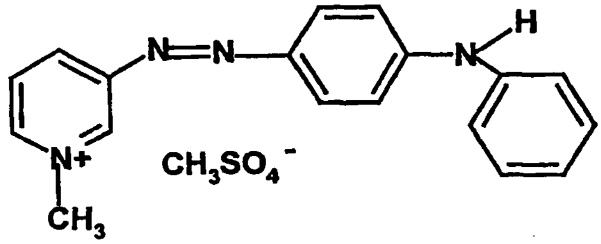

(VII41)

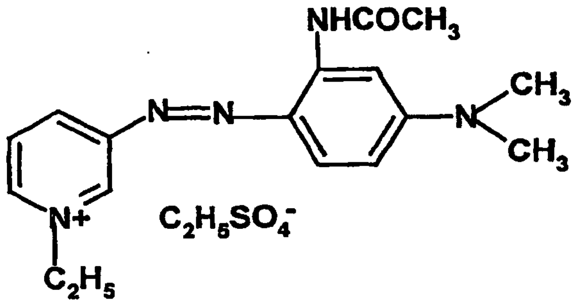

(VII42)

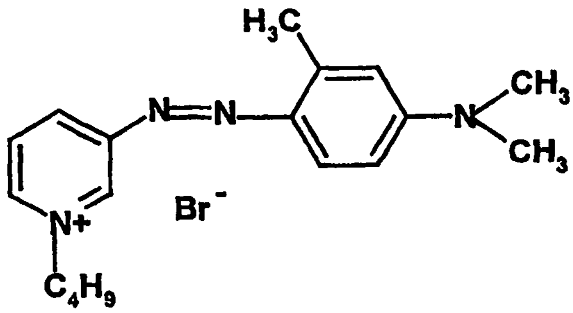

(VII43)

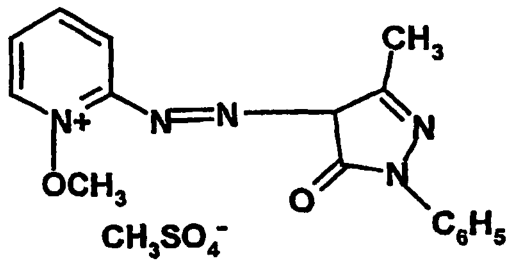

(VII44)

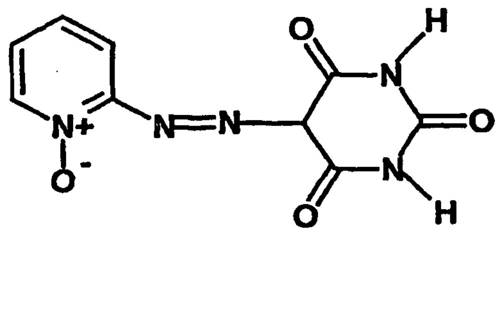

(VII45)

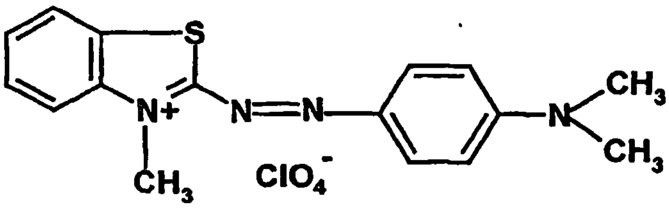

(VII46)

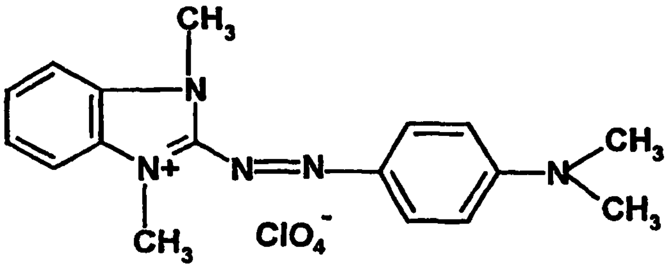

(VII47)

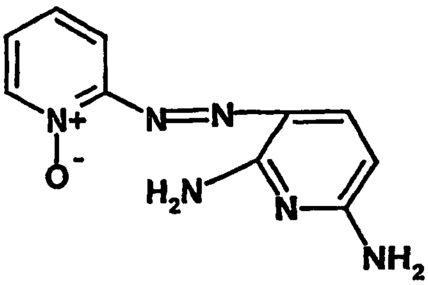

(VII48)

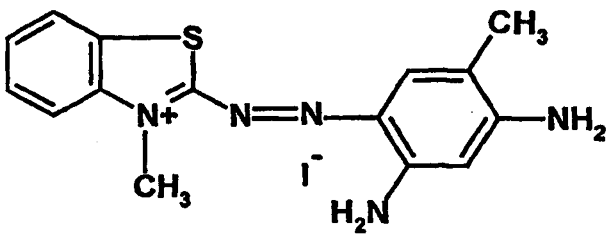

(VII49)

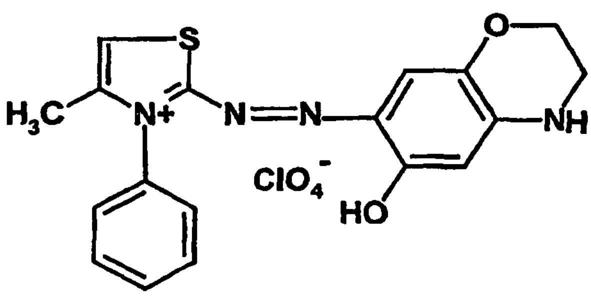

(VII50)

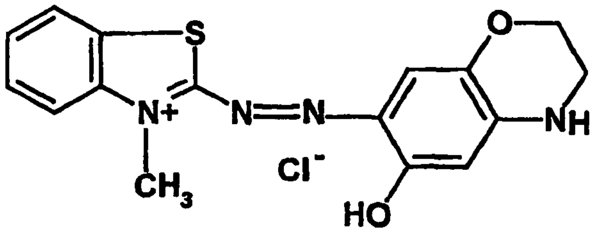

(VII51)

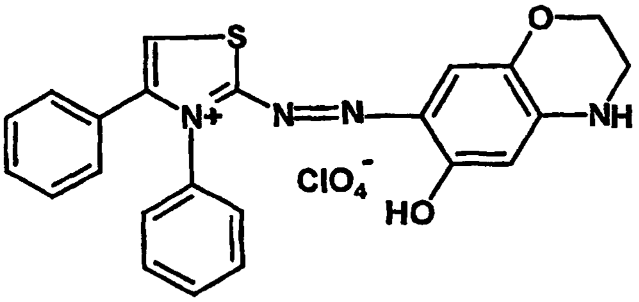

(VII52)

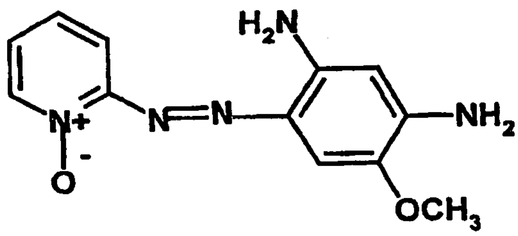

(VII53)

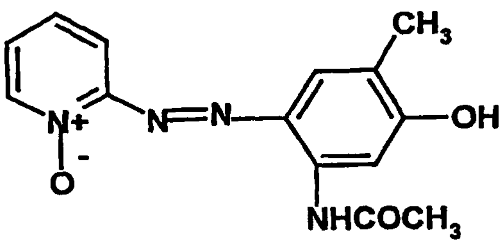

(VII54)

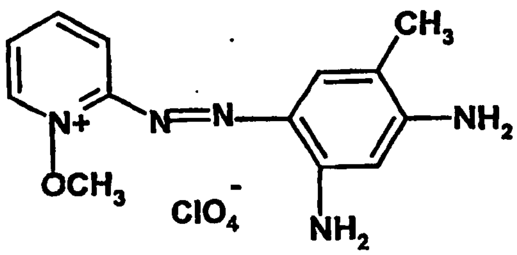

(VII55)

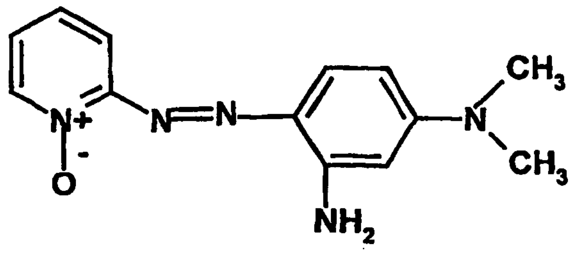

(VII56)

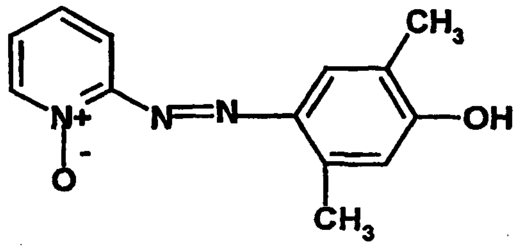

(VII57)

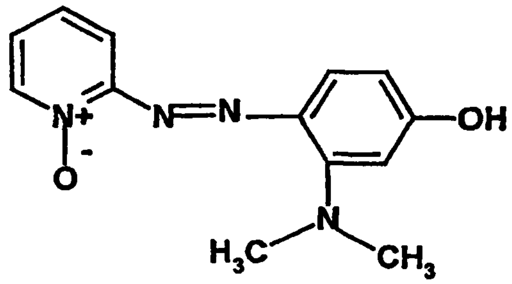

(VII58)

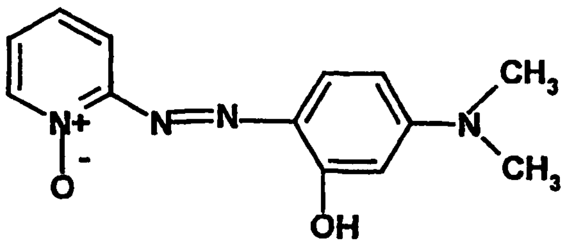

(VII59)

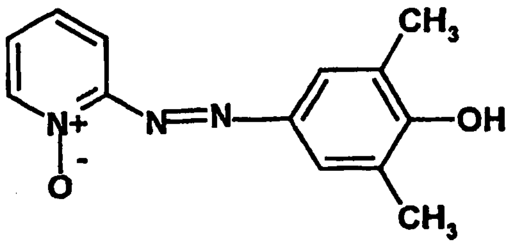

(VII60)

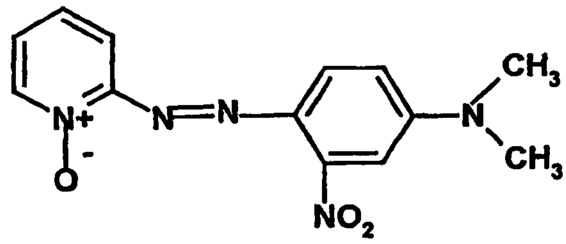

(VII61)

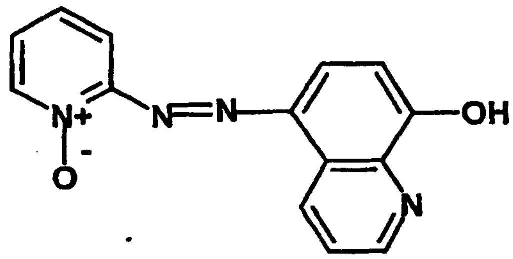

(VII62)

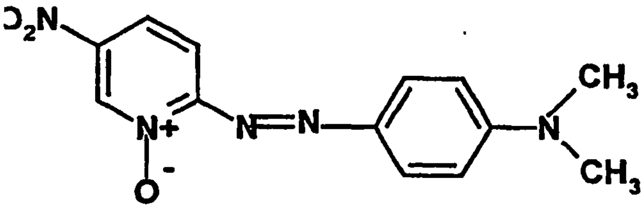

(VII63)

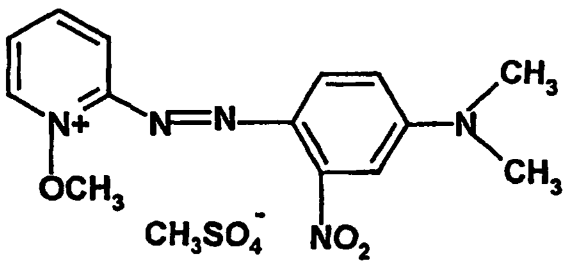

(VII64)

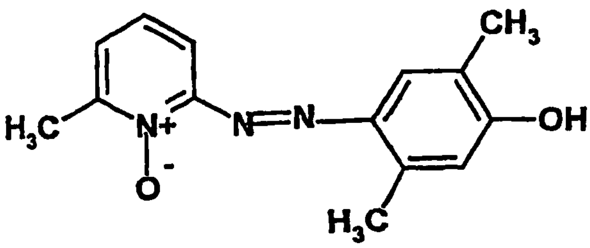

(VII65)

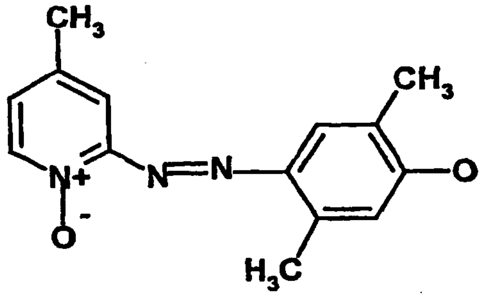

(VII66)

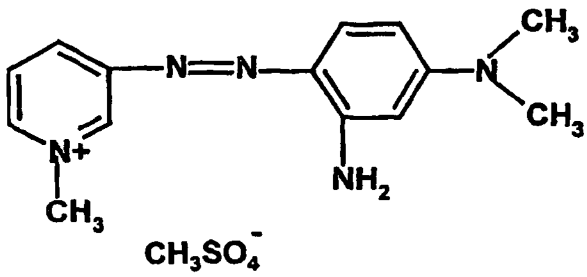

(VII67)

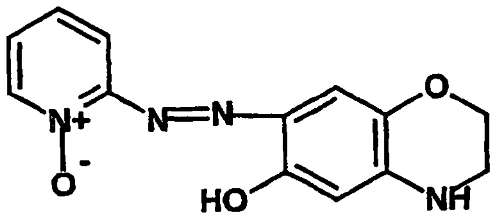

(VII68)

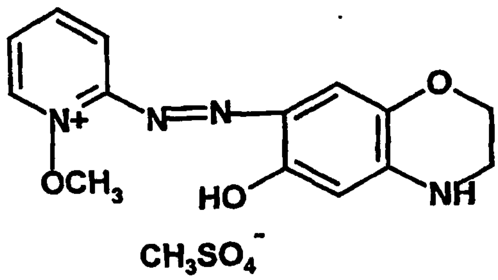

(VII69)

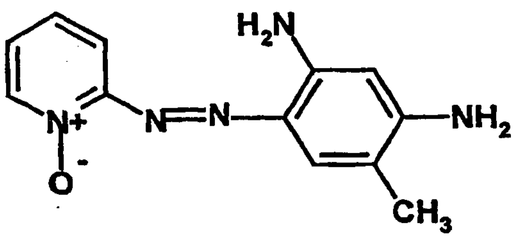

(VII70)

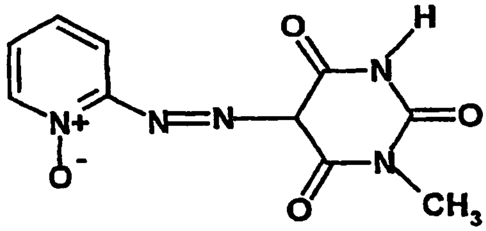

(VII71)

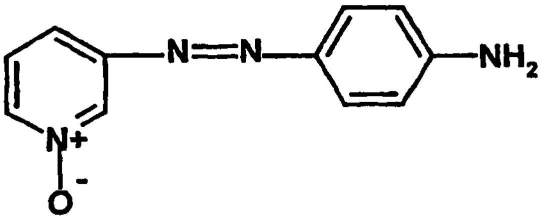

(VII72)

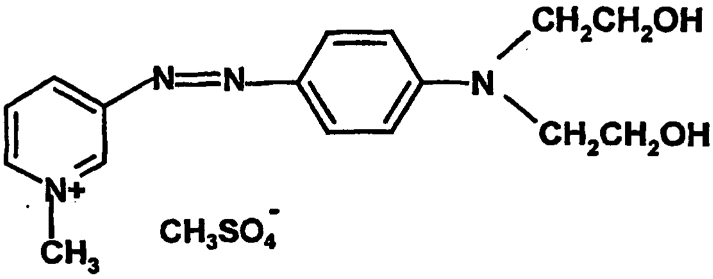

(VII73)

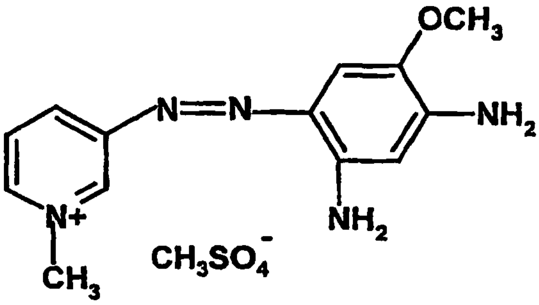

(VII74)

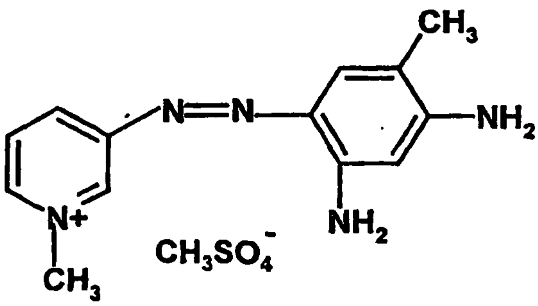

(VII75)

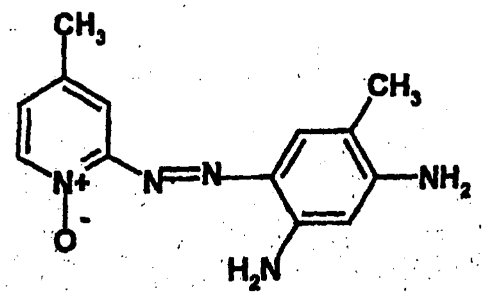

(VII76)

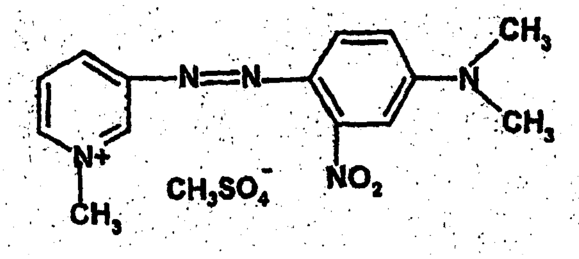

(VII77)

Tous ces colorants de formules (I) à (VII) particulièrement tenaces sont décapables au moyen de la composition de décoloration selon la présente invention et tout particulièrement les colorants de formules (IV2) (=Basic Red 51), (IV14) (=Basic Orange 31), (VI4) (=Basic Yellow 87).

Le ou les colorants directs représentent de préférence de 0,001 à 20% en poids environ du poids total de la composition de teinture oxydante ou non oxydante et encore plus préférentiellement de 0,005 à 10% en poids environ.

Dans le kit à plusieurs compartiments, l'oxydant nécessaire à réaliser la teinture d'oxydation ou la teinture éclaircissante, est séparé du ou des colorants d'oxydation ou du ou des colorants directs cationiques. Il est choisi de préférence parmi le peroxyde d'hydrogène, le peroxyde d'urée, les bromates ou ferricyanures de métaux alcalins, les persels tels que les perborates et les persulfates, l'utilisation du peroxyde d'hydrogène étant particulièrement préférée. Cet agent oxydant est avantageusement constitué par une solution d'eau oxygénée dont le titre peut varier, plus particulièrement, d'environ 1 à 40 volumes, et encore plus préférentiellement d'environ 5 à 40.

On peut également utiliser à titre d'agent oxydant une ou plusieurs enzymes d'oxydoréduction telles que les oxydo-réductases à 4 électrons (telles que les laccases), les peroxydases et les oxydo-réductases à 2 électrons (telles que l'uricase), le cas échéant en présence de leur donneur ou cofacteur respectif.

Le milieu cosmétiquement acceptable et approprié pour la décoloration (ou support de la composition), conforme à l'invention, est généralement constitué par de l'eau ou par un mélange d'eau et d'au moins un solvant organique pour solubiliser les composés qui ne seraient pas suffisamment solubles dans l'eau. A titre de solvant organique, on peut par exemple citer les alcanols en $C_1$-$C_4$, tels que l'éthanol et l'isopropanol ; le glycérol ; les glycols et éthers de glycols comme le 2-butoxyéthanol, le propylèneglycol, le dipropylène glycol, l'hexylène glycol, le monométhyléther de propylèneglycol, le monoéthyléther et le monométhyléther du diéthylèneglycol, ainsi que les alcools aromatiques comme l'alcool benzylique ou le phénoxyéthanol, les produits analogues et leurs mélanges.

Lesdits solvants peuvent alors être présents dans des proportions de préférence comprises entre 0,5 à 20% et, plus particulièrement, de 2 à 10% en poids par rapport au poids total de la composition de décoloration.

Le pH de la composition de décoloration utile selon l'invention, est de préférence compris entre 1,8 et 6.

IL est ajusté par des agents acidifiants ou alcalinisants, dans des quantités allant de 0,01 à 30% en poids du poids total de la composition.

Parmi les agents acidifiants, on peut citer, à titre d'exemple, les acides minéraux ou organiques comme l'acide étidronique, l'acide chlorhydrique, l'acide orthophosphorique, l'acide sulfurique, les acides carboxyliques comme l'acide acétique, l'acide tartrique, l'acide citrique, l'acide lactique, et les acides sulfoniques.

Parmi les agents alcalinisants on peut citer, à titre d'exemple, l'ammoniaque, les carbonates alcalins, les alcanolamines telles que les mono-, di- et triéthanolamines, le 2-méthyl-2-amino-1-propanol ainsi que leurs dérivés, les hydroxydes de sodium ou de potassium et les composés de formule (III) suivante :

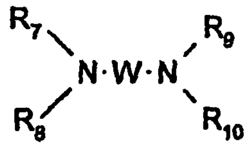

(III)

dans laquelle W est un reste propylène éventuellement substitué par un groupement hydroxyle ou un radical alkyle en $C_1$-$C_4$ ; $R_7$, $R_8$, $R_9$ et $R_{10}$, identiques ou différents, représentent un atome d'hydrogène, un radical alkyle en $C_1$-$C_4$ ou hydroxyalkyle en $C_1$-$C_4$.

La composition de décoloration utile selon l'invention peut en outre renfermer d'autres réducteurs que ceux de formule (I) selon l'invention et choisis notamment parmi les acides alpha-oxocarboxyliques tels que l'acide oxalique, l'acide glyoxalique, l'acide pyruvique ou l'acide alpha-cétoglutarique.

La composition de décoloration utile selon l'invention peut renfermer au moins un agent épaississant dans une proportion comprise entre 0,01 et 10% et en particulier entre 0,1 et 5% environ en poids du poids total de la composition. Ils sont choisis de préférence parmi les dérivés de cellulose, les dérivés de guar, les gommes d'origine microbienne ou végétale et les épaississants synthétiques.

**Agents épaississants :**

Parmi les dérivés de cellulose, on peut citer les hydroxyalkyl($C_1$-$C_6$)celluloses et carboxyalkyl($C_1$-$C_6$)celluloses, Les hydroxyalkyl($C_1$-$C_6$)celluloses sont plus particulièrement des hydroxyéthylcelluloses telles que celles vendues sous les dénominations CELLOSIZE QP3L, CELLOSIZE QP4400H, CELLOSIZE QP30000H, CELLOSIZE HEC30000A, CELLOSIZE POLYMER PCG10, par la société AMERCHOL, ou NATROSOL 250HHR, NATROSOL 250MR, NATROSOL 250M, NATROSOL 250HHXR, NATROSOL 250HHX, NATROSOL 250HR, NATROSOL HX, par la société HERCULES, ou encore TYLOSE H1000 par la société HOECHST. Les hydroxyalkyl($C_1$-$C_6$)celluloses sont également plus particulièrement des hydroxypropylcelluloses comme les produits vendus sous les dénominations KLUCEL EF, KLUCEL H, KLUCEL LHF, KLUCEL MF, KLUCEL G, par la société AQUALON. Parmi les carboxyalkyl($C_1$-$C_6$)cellulOses, on utilise de préférence la carboxyméthylcellulose dont on peut citer les produits vendus sous les dénominations BLANOSE 7M8/SF, BLANOSE RAFFINEE 7M, BLANOSE 7LF, BLANOSE 7MF, BLANOSE 9M31F, BLANOSE 12M31XP, BLANOSE 12M31P, BLANOSE 9M31XF, BLANOSE 7H, BLANOSE 7M31, BLANOSE 7H3SXF, par la société AQUALON, ou encore AQUASORB A500 et AMBERGUM 1221, par le société HERCULES, ou encore CELLOGEN HP810A et CELLOGEN HP6HS9, par la société MONTELLO, ou encore PRIMELLOSE par la société AVEBE.

Parmi les dérivés de guar, on peut citer les gommes de guar non ioniques modifiées ou non modifiées. Les gommes de guar non modifiées sont par exemple les produits vendus sous la dénomination VIDOGUM GH 175 par la société UNIPECTINE, et sous les dénominations MEYPRO-GUAR 50 et JAGUAR C par la société MEYHALL. Les gommes de guar non-ioniques modifiées sont notamment modifiées par des groupements hydroxyalkyle en $C_1$-$C_6$. Parmi les groupements hydroxyalkyle, on peut mentionner à titre d'exemple, les groupements hydroxyméthyle, hydroxyéthyle, hydroxypropyle et hydroxybutyle.
De telles gommes de guar non-ioniques éventuellement modifiées par des groupements hydroxyalkyle sont par exemple vendues sous les dénominations commerciales JAGUAR HP8, JAGUAR HP60 et JAGUAR HP120, JAGUAR DC 293 et JAGUAR HP 105 par la société RHONE POULENC (MEYHALL) ou sous la dénomination GALACTASOL 4H4FD2 par la société AQUALON.

Parmi les gommes d'origine microbienne, on peut citer les gommes de biopolysaccharides tels que les Scléroglucanes ou les Xanthanes.
Les scléroglucanes sont représentés par exemple par les produits vendus sous la dénomination ACTIGUM CS par la société SANOFI BIO INDUSTRIES et en particulier ACTIGUM CS 11 et sous la dénomination AMIGEL par la société ALBAN MULLER INTERNATIONAL. D'autres scléroglucanes tels que celui traité au glyoxal dans la demande de brevet français N°2633940 peuvent également être utilisés.
Les Xanthanes sont représentés par exemple par les produits vendus sous les dénominations KELTROL, KELTROL T, KELTROL TF, KELTROL BT, KELTROL RD, KELTROL CG par la société NUTRASWEET KELCO, ou sous les dénominations RHODICARE S, RHODICARE H par la société RHODIA CHIMIE.
Parmi les gommes d'origine végétale, on peut citer celles issues d'exudats végétaux telles que les gommes Aabique, gomme Ghatti, gomme Karaya, Tragacanthe, Carrageenane, Agar et Caroube.
On peut également utiliser les pectines, les alginates et les amidons.
Tous ces composés sont bien connus de l'homme de l'art et décrits notamment dans l'ouvrage de Robert L. DAVIDSON intitulé "Handbook of Water soluble gums and resins" édité chez Mc Graw Hill Book Company (1980).

Epaississants synthétiques :

Parmi eux, les polymères amphiphiles, encore appelés "associatifs", comportant au moins une chaîne grasse, et de type anionique, non-ionique, cationique ou amphotère.

Polymères amphiphiles ou "associatifs" :

Parmi les polymères comportant au moins une chaîne grasse et de type anionique, on peut citer :

- (**I**) ceux comportant au moins un motif hydrophile, et au moins un motif éther d'allyle à chaîne grasse, plus particulièrement ceux dont le motif hydrophile est constitué par un monomère anionique insaturé éthylénique, plus particulièrement encore par un acide carboxylique vinylique et tout particulièrement par un acide acrylique ou un acide méthacrylique ou les mélanges de ceux ci, et dont le motif éther d'allyle à chaîne grasse correspond au monomère de formule (XV) suivante :

$$CH_2 = C\ R'\ CH_2\ O\ B_n\ R \qquad (XV)$$

dans laquelle R' désigne H ou $CH_3$, B désigne le radical éthylèneoxy, n est nul ou désigne un entier allant de 1 à 100, R désigne un radical hydrocarboné choisi parmi les radicaux alkyl, arylalkyle, aryle, alkylaryle, cycloalkyle, comprenant de 8 à 30 atomes de carbone, de préférence 10 à 24, et plus particulièrement encore de 12 à 18 atomes de carbone. Un motif de formule (XV) plus particulièrement préféré est un motif dans lequel R' désigne H, n est égal à 10, et R désigne un radical stéaryl ($C_{18}$).
Des polymères amphiphiles anioniques de ce type sont décrits et préparés, selon un procédé de polymérisation en émulsion, dans le brevet EP-0 216 479. Parmi ces polymères épaississants anioniques à chaîne grasse, on préfère particulièrement selon l'invention, les polymères formés à partir de 20 à 60% en poids d'acide acrylique et/ou d'acide méthacrylique, de 5 à 60% en poids de (méth)acrylates d'alkyles inférieurs, de 2 à 50% en poids d'éther d'allyl à chaîne grasse de formule (XV), et de 0 à 1% en poids d'un agent réticulant qui est un monomère insaturé polyéthylénique copolymérisable bien connu, comme le phtalate de diallyle, le (méth)acrylate d'allyl, le divinylbenzène, le diméthacrylate de (poly)éthylèneglycol, et le méthylène-bis-acrylamide.
Parmi ces derniers, on préfère tout particulièrement les terpolymères réticulés d'acide méthacrylique, d'acrylate d'éthyle, de polyéthylèneglycol (10 OE) éther d'alcool stéarylique (Steareth 10), notamment ceux vendus par la société ALLIED COLLOIDS sous les dénominations SALCARE SC 80 et SALCARE SC90 qui sont des émulsions aqueuses à 30% d'un terpolymère réticulé d'acide méthacrylique, d'acrylate d'éthyle et de steareth-10-allyl éther (40/50/10).

- (**II**) ceux comportant au moins un motif hydrophile de type acide carboxylique insaturé oléfinique, et au moins un motif hydrophobe de type ester d'alkyl ($C_{10}$-$C_{30}$) d'acide carboxylique insaturé.
De préférence, ces polymères sont choisis parmi ceux dont le motif hydrophile de type acide carboxylique insaturé oléfinique correspond au monomère de formule (XVI) suivante :

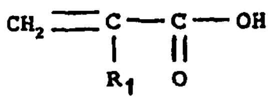

(XVI)

dans laquelle, $R_1$ désigne H ou $CH_3$ ou $C_2H_5$, c'est-à-dire des motifs acide acrylique, acide méthacrylique ou acide éthacrylique, et dont le motif hydrophobe de type ester d'alkyl ($C_{10}$-$C_{30}$) d'acide carboxylique insaturé correspond au monomère de formule (XVII) suivante :

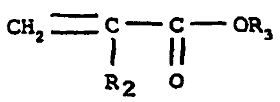

(XVII)

dans laquelle, $R_2$ désigne H ou $CH_3$ ou $C_2H_5$ (c'est-à-dire des motifs acrylates, méthacrylates ou éthacrylates) et de préférence H (motifs acrylates) ou $CH_3$ (motifs méthacrylates), $R_3$ désignant un radical alkyle en $C_{10}$-$C_{30}$, et de préférence en $C_{12}$-$C_{22}$.

Des esters d'alkyles ($C_{10}$-$C_{30}$) d'acides carboxyliques insaturés conformes à l'invention comprennent par exemple, l'acrylate de lauryle, l'acrylate de stéaryle, l'acrylate de décyle, l'acrylate d'isodécyle, l'acrylate de dodécyle, et les méthacrylates correspondants, le méthacrylate de lauryle, le méthacrylate de stéaryle, le méthacrylate de décyle, le méthacrylate d'isodécyle, et le méthacrylate de dodécyle.
Des polymères anioniques de ce type sont par exemple décrits et préparés, selon les brevets US-3 915 921 et 4 509 949.
Parmi ce type de polymères épaississants anioniques à chaîne grasse, on utilisera plus particulièrement des polymères formés à partir d'un mélange de monomères comprenant :

(i) essentiellement de l'acide acrylique,
(ii) un ester de formule (XVI) décrite ci-dessus et dans laquelle $R_2$ désigne H ou $CH_3$, $R_3$ désignant un radical alkyle ayant de 12 à 22 atomes de carbone,
(iii) et un agent réticulant, qui est un monomère insaturé polyéthylénique copolymérisable bien connu, comme le phtalate de diallyle, le (méth)acrylate d'allyl, le divinylbenzène, le diméthacrylate de (poly)éthylèneglycol, et le méthylène-bis-acrylamide.
Parmi ce type de polymères épaississants anioniques à chaîne grasse, on utilisera plus particulièrement ceux constitués de 95 à 60% en poids d'acide acrylique (motif hydrophile), 4 à 40% en poids d'acrylate d'alkyles en $C_{10}$-$C_{30}$ (motif hydrophobe), et 0 à 6% en poids de monomère polymérisable réticulant, ou bien ceux constitués de 98 à 96% en poids d'acide acrylique (motif hydrophile), 1 à 4% en poids d'acrylate d'alkyles en $C_{10}$-$C_{30}$ (motif hydrophobe), et 0,1 à 0,6% en poids de monomère polymérisable réticulant tel que ceux décrits précedemment.
Parmi lesdits polymères ci-dessus, on préfère tout particulièrement selon la présente invention, les produits vendus par la société GOODRICH sous les dénominations commerciales PEMULEN TR1, PEMULEN TR2, CARBOPOL 1382, et encore plus préférentiellement le PEMULEN TR1, et le produit vendu par la société S.E.P.P.I.C. sous la dénomination COATEX SX.

- (**III**) les terpolymères d'anhydride maléique/$\alpha$-olëfine en $C_{30}$-$C_{38}$/ maléate d'alkyle tel que le produit (copolymère anhydride matéique/$\alpha$-oléfine en $C_{30}$-$C_{38}$/maléate d'isopropyle) vendu sous le nom PERFORMA V 1608 par la société NEWPHASE TECHNOLOGIES.

- (**IV**) les terpolymères acryliques comprenant :

  (a) environ 20% à 70% en poids d'un acide carboxylique à insaturation $\alpha,\beta$-monoéthylénique,
  (b) environ 20 à 80% en poids d'un monomère à insaturation $\alpha,\beta$-monoéthylénique non-tensio-actif différent de (a),
  (c) environ 0,5 à 60% en poids d'un mono-uréthane non-ionique qui est le produit de réaction d'un tensio-actif monohydrique avec un monoisocyanate à insaturation monoéthylénique,
  tels que ceux décrits dans la demande de brevet EP-A-0173109 et plus particulièrement celui décrit dans l'exemple 3, à savoir, un terpolymère acide méthacrylique /acrylate de méthyle/diméthyl métaisopropényl benzyl isocyanate d'alcool béhényle éthoxylé (40OE) en dispersion aqueuse à 25%.

- (**V**) les copolymères comportant parmi leurs monomères un acide carboxylique à insaturation $\alpha,\beta$-monoéthylénique et un ester d'acide carboxylique à insaturation $\alpha,\beta$-monoéthylénique et d'un alcool gras oxyalkyléné.
  Préférentiellement ces composés comprennent également comme monomère un ester d'acide carboxylique à insaturation $\alpha,\beta$-monoéthylénique et d'alcool en C1-C4.

A titre d'exemple de ce type de composé on peut citer l'ACULYN 22 vendu par la société ROHM et HAAS, qui est un terpolymère acide méthacrylique/acrylate d'éthyle/méthacrylate de stéaryle oxyalkyléné.
<u>Parmi les polymères à chaîne grasse de type non ionique</u>, on choisit de préférence:

- (**1**) les celluloses modifiées par des groupements comportant au moins une chaîne grasse ; on peut citer à titre d'exemple :

  - les hydroxyéthylcelluloses modifiées par des groupements comportant au moins une chaîne grasse tels que des groupes alkyle, arylalkyle, alkylaryle, ou leurs mélanges, et dans lesquels les groupes alkyle sont de préférence en $C_8$-$C_{22}$, comme le produit NATROSOL PLUS GRADE 330 CS (alkyles en $C_{16}$) vendu par la société AQUALON, ou le produit BERMOCOLL EHM 100 vendu par la société BEROL NOBEL,

- celles modifiées par des groupes polyalkylène glycol éther d'alkyl phénol, tel que le produit AMERCELL POLYMER HM-1500 (polyéthylène glycol (15) éther de nonyl phénol) vendu par la société AMERCHOL.

- (**2**) les hydroxypropylguars modifiés par des groupements comportant au moins une chaîne grasse tel que le produit ESAFLOR HM 22 (chaîne alkyle en $C_{22}$) vendu par la société LAMBERTI, les produits RE210-18 (chaîne alkyle en $C_{14}$) et RE205-1 (chaîne alkyle en $C_{20}$) vendus par la société RHONE POULENC.

- (**3**) les copolymères de vinyl pyrrolidone et de monomères hydrophobes à chaîne grasse ; on peut citer à titre d'exemple :

  - les produits ANTARON V216 ou GANEX V216 (copolymère vinylpyrrolidone /hexadécène) vendu par la société I.S.P.
  - les produits ANTARON V220 ou GANEX V220 (copolymère vinylpyrrolidone /eicosène) vendu par la société I.S.P.

- **(4)** les copolymères de méthacrylates ou d'acrylates d'alkyles en $C_1$-$C_6$ et de monomères amphiphiles comportant au moins une chaîne grasse tels que par exemple le copolymère acrylate de méthyle/acrylate de stéaryle oxyéthyléné vendu par la société GOLDSCHMIDT sous la dénomination ANTIL 208.

- (**5**) les copolymères de méthacrylates ou d'acrylates hydrophiles et de monomères hydrophobes comportant au moins une chaîne grasse tels que par exemple le copolymère méthacrylate de polyéthylèneglycol/méthacrylate de lauryle.

- (**6**) les polyuréthanes polyéthers comportant dans leur chaîne, à la fois des séquences hydrophiles de nature le plus souvent polyoxyéthylénée et des séquences hydrophobes qui peuvent être des enchaînements aliphatiques seuls et/ou des enchaînements cycloaliphatiques et/ou aromatiques.

- (**7**) les polymères à squelette aminoplaste éther possédant au moins une chaîne grasse, tels que les composés PURE THIX proposés par la société SUD-CHEMIE.

De préférence, les polyéthers polyuréthanes comportent au moins deux chaînes lipophiles hydrocarbonées, ayant de 6 à 30 atomes de carbone, séparées par une séquence hydrophile, les chaînes hydrocarbonées pouvant être des chaînes pendantes ou des chaînes en bout de séquence hydrophile. En particulier, il est possible qu'une ou plusieurs chaînes pendantes soient prévues. En outre, le polymère peut comporter, une chaîne hydrocarbonée à un bout ou aux deux bouts d'une séquence hydrophile.
Les polyéthers polyuréthanes peuvent être multiséquencés en particulier sous forme de tribloc. Les séquences hydrophobes peuvent être à chaque extrémité de la chaîne (par exemple: copolymère tribloc à séquence centrale hydrophile) ou réparties à la fois aux extrémités et dans la chaîne (copolymère multiséquencé par exemple). Ces mêmes polymères peuvent être également en greffons ou en étoile.
Les polyéthers polyuréthanes non-ioniques à chaîne grasse peuvent être des copolymères triblocs dont la séquence hydrophile est une chaîne polyoxyéthylénée comportant de 50 à 1000 groupements oxyéthylénés. Les polyéthers polyuréthanes non-ioniques comportent une liaison uréthanne entre les séquences hydrophiles, d'où l'origine du nom.
Par extension figurent aussi parmi les polyéthers polyuréthanes non-ioniques à chaîne grasse, ceux dont les séquences hydrophiles sont liées aux séquences lipophiles par d'autres liaisons chimiques.
A titre d'exemples de polyéthers polyuréthanes non-ioniques à chaîne grasse utilisables dans l'invention, on peut aussi utiliser aussi le Rhéolate 205 à fonction urée vendu par la société RHEOX ou encore les Rhéolates 208 , 204 ou 212, ainsi que l'Acrysol RM 184, l'Aculyn 44 et l'Aculyn 46 de la société ROHM & HAAS [l'ACULYN 46 est un polycondensat de polyéthylèneglycol à 150 ou 180 moles d'oxyde d'éthylène, d'alcool stéarylique et de méthylène bis(4-cyclohexylisocyanate) (SMDI), à 15% en poids dans une matrice de maltodextrine (4%) et d'eau (81%); l'ACULYN 44 est un polycondensat de polyéthylèneglycol à 150 ou 180 moles d'oxyde d'éthylène, d'alcool décylique et de méthylène bis(4-cyclohexylisocyanate) (SMDI), à 35% en poids dans un mélange de propylèneglycol (39%) et d'eau (26%)].
On peut également citer le produit ELFACOS T210 à chaîne alkyle en $C_{12-14}$ et le produit ELFACOS T212 à chaîne alkyle en $C_{18}$ de chez AKZO.
Le produit DW 1206B de chez ROHM & HAAS à chaîne alkyle en $C_{20}$ et à liaison uréthanne, proposé à 20 % en matière sèche dans l'eau, peut aussi être utilisé.
On peut aussi utiliser des solutions ou dispersions de ces polymères notamment dans l'eau ou en milieu hydroalcoolique. A titre d'exemple, de tels polymères on peut citer, le Rhéolate 255, le Rhéolate 278 et le Rhéolate 244 vendus par la société RHEOX. On peut aussi utiliser le produit DW 1206F et le DW 1206J proposés par la société ROHM & HAAS.

Les polyéthers polyuréthanes utilisables selon l'invention sont en particulier ceux décrits dans l'article de G. Fonnum, J. Bakke et Fk. Hansen - Colloid Polym. Sci 271, 380.389 (1993).

Parmi les polymères à chaîne grasse et de type cationique, on peut les dérivés de cellulose quaternisée qui sont, en particulier,

- les celluloses quaternisées modifiées par des groupements comportant au moins une chaîne grasse, tels que les groupes alkyle, arylalkyle, alkylaryle comportant au moins 8 atomes de carbone, ou des mélanges de ceux-ci,
- les hydroxyéthylcelluloses quaternisées modifiées par des groupements comportant au moins une chaîne grasse, tels que les groupes alkyle, arylalkyle, alkylaryle comportant au moins 8 atomes de carbone, ou des mélanges de ceux-ci.

Les radicaux alkyle portés par les celluloses ou hydroxyéthylcelluloses quaternisées ci-dessus comportent de préférence de 8 à 30 atomes de carbone. Les radicaux aryle désignent de préférence les groupements phényle, benzyle, naphtyle ou anthryle. On peut indiquer comme exemples d'alkylhydroxyéthylcelluloses quaternisées à chaînes grasses en $C_8$-$C_{30}$, les produits QUATRISOFT LM 200, QUATRISOFT LM-X 529-18-A, QUATRISOFT LM-X 529-18B (alkyle en $C_{12}$) et QUATRISOFT LM-X 529-8 (alkyle en $C_{18}$) commercialisés par la société AMERCHOL et les produits CRODACEL QM, CRODACEL QL (alkyle en $C_{12}$) et CRODACEL QS (alkyle en C18) commercialisés par la société CRODA.

Les polyacrylates à groupements latéraux aminés, quaternisés ou non, possèdent par exemple des groupements hydrophobes du type stéareth 20 (alcool stéarylique polyoxyéthyléné(20)).

Comme exemples de polyacrylates à chaînes latérales aminées, on peut citer les polymères 8781-121B ou 9492-103 proposés par la société NATIONAL STARCH.

Parmi les polymères comportant au moins une chaîne grasse et de type amphotère, on choisit de préférence ceux comportant au moins un motif cationique non cyclique. Plus particulièrement encore, on préfère ceux préparés à partir ou comprenant 1 à 20 moles% de monomère comportant une chaîne grasse, et de préférence 1,5 à 15 moles% et plus particulièrement encore 1,5 à 6 moles%, par rapport au nombre total de moles de monomères.

Les polymères amphotères à chaîne grasse préférés selon l'invention comprennent, ou sont préparés en copolymérisant :

1) au moins un monomère de formule (Ia) ou (Ib):

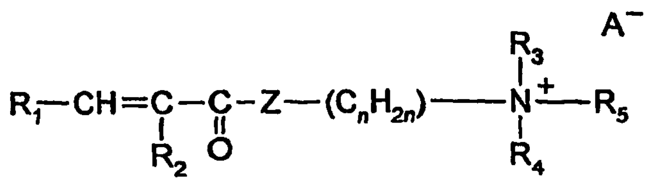

(Ia)

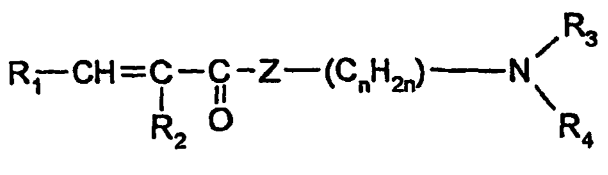

(Ib)

dans lesquelles, $R_1$ et $R_2$, identiques ou différents, représentent un atome d'hydrogène ou un radical méthyle, $R_3$, $R_4$ et $R_5$, identiques ou différents, représente un radical alkyle linéaire ou ramifié ayant de 1 à 30 atomes de carbone,
Z représente un groupe NH ou un atome d'oxygène,
n est un nombre entier de 2 à 5,
$A^-$ est un anion issu d'un acide organique ou minéral, tel qu'un anion méthosulfate ou un halogénure tel que chlorure ou bromure;

2) au moins un monomère de formule (II)

$$R_6\text{-}CH\text{= }CR_7\text{-}COOH \qquad (II)$$

dans laquelle, $R_6$ et $R_7$, identiques ou différents, représentent un atome d'hydrogène

ou un radical méthyle;
et

3) au moins un monomère de formule (III) :

$$R_6\text{-CH} = CR_7\text{-COXR}_8 \qquad (III)$$

dans laquelle $R_6$ et $R_7$, identiques ou différents, représentent un atome d'hydrogène ou un radical méthyle, X désigne un atome d'oxygène ou d'azote et $R_8$ désigne un radical alkyle linéaire ou ramifié ayant de 1 à 30 atomes de carbone ;
l'un au moins des monomères de formule (Ia), (Ib) ou (III) comportant au moins une chaîne grasse.

Les monomères de formule (Ia) et (Ib) de la présente invention sont choisis, de préférence, dans le groupe constitué par :

- le diméthylaminoéthylméthacrylate, le diméthylaminoéthylacrylate,
- le diéthylaminoéthylméthacrylate, le diéthylaminoéthylacrylate,
- le diméthylaminopropylméthacrylate, le diméthylaminopropylacrylate,
- le diméthylaminopropylméthacrylamide, le diméthylaminopropylacrylamide,

ces monomères étant éventuellement quatemisés, par exemple par un halogénure d'alkyle en $C_1$-$C_4$ ou un sulfate de dialkyle en $C_1$-$C_4$.

Plus particulièrement, le monomère de formule (la) est choisi parmi le chlorure d'acrylamidopropyl triméthyl ammonium et le chlorure de méthacrylamidopropyl triméthyl ammonium.

Les monomères de formule (II) de la présente invention sont choisis, de préférence, dans le groupe constitué par l'acide acrylique, l'acide méthacrylique, l'acide crotonique et l'acide méthyl-2 crotonique. Plus particulièrement, le monomère de formule (II) est l'acide acrylique.

Les monomères de formule (III) de la présente invention sont choisis, de préférence, dans le groupe constitué par des acrylates ou méthacrylates d'alkyle en $C_{12}$-$C_{22}$ et plus particulièrement en $C_{16}$-$C_{18}$.

Les monomères constituant les polymères amphotères à chaîne grasse de l'invention sont de préférence déjà neutralisés et/ou quaternisés.

Le rapport du nombre de charges cationiques/charges anioniques est de préférence égal à environ 1.

Les polymères amphotères à chaîne grasse selon l'invention comprennent de préférence de 1 à 10 moles% du monomère comportant une chaîne grasse (monomère de formule (Ia), (Ib) ou (III)), et de préférence de 1,5 à 6 moles %.

Les poids moléculaires moyens en poids des polymères amphotères à chaîne grasse selon l'invention peuvent varier de 500 à 50.000.000 et sont de préférence compris entre 10.000 et 5 000 000.

Les polymères amphotères à chaîne grasse selon l'invention peuvent également contenir d'autre monomères tels que des monomères non ioniques et en particulier tels que les acrylates ou méthacrylates d'alkyle en $C_1$-$C_4$.

Des polymères amphotères à chaîne grasse selon l'invention sont par exemple décrits et préparés dans la demande de brevet WO9844012.

Parmi les polymères amphotères à chaîne grasse selon l'invention, on préfère les copolymères acide acrylique/chlorure dé (méth)acrylamidopropyl triméthyl ammonium/méthacrylate de stéaryle.

La composition de décoloration utile selon l'invention, peut également renfermer divers adjuvants utilisés classiquement dans les compositions pour la décoloration des cheveux.

**Adjuvants :**

Parmi ces adjuvants, les agents tensioactifs peuvent être présents et être indifféremment choisis, seuls ou en mélanges, au sein des tensioactifs anioniques, amphotères, non ioniques, zwittérioniques et cationiques.

Les tensioactifs convenant à la mise en oeuvre de la présente invention sont notamment les suivants :

(i) Tensioactif(s) anionique(s):

A titre d'exemple de tensio-actifs anioniques utilisables, seuls ou mélanges, dans le cadre de la présente invention, on peut citer notamment (liste non limitative) les sels (en particulier sels alcalins, notamment de sodium, sels d'ammonium, sels d'amines, sels d'aminoalcools ou sels de magnésium) des composés suivants : les alkylsulfates, les alkyléthersulfates, alkylamidoéthersulfates, alkylarylpolyéthersulfates, monoglycérides sulfates ; les alkylsulfonates, alkylphosphates, alkylamidesulfonates, alkylarylsulfonates, $\alpha$-oléfine-sulfonates, parafhne-sulfonates ; les alkyl($C_6$-$C_{24}$)

sulfosuccinates, les alkyl($C_6$-$C_{24}$) éthersulfosuccinates, les alkyl($C_6$-$C_{24}$) amidesulfosuccinates ; les alkyl($C_6$-$C_{24}$) sulfoacétates ; les acyl($C_6$-$C_{24}$) sarcosinates et les acyl($C_6$-$C_{24}$) glutamates. On peut également utiliser les esters d'alkyl($C_6$-$C_{24}$)polyglycosides carboxyliques tels que les alkylglucoside citrates, les alkylpolyglycoside tartrate et les alkylpolyglycoside sulfosuccinates., les alkylsulfosuccinamates ; les acyliséthionates et les N-acyltaurates, le radical alkyle ou acyle de tous ces différents composés comportant de préférence de 12 à 20 atomes de carbone, et le radical aryl désignant de préférence un groupement phényle ou benzyle. Parmi les tensioactifs anioniques encore utilisables, on peut également citer les sels d'acides gras tels que les sels des acides oléique, ricinoléique, palmitique, stéarique, les acides d'huile de coprah ou d'huile de coprah hydrogénée ; les acyl-lactylates dont le radical acyle comporte 8 à 20 atomes de carbone. On peut également utiliser les acides d'alkyl D galactoside uroniques et leurs sels, les acides alkyl ($C_6$-$C_{24}$) éther carboxyliques polyoxyalkylénés, les acides alkyl($C_6$-$C_{24}$)aryl éther carboxyliques polyoxyalkylénés, les acides alkyl($C_6$-$C_{24}$) amido éther carboxyliques polyoxyalkylénés et leurs sels, en particulier ceux comportant de 2 à 50 groupements oxyde d'alkylène en particulier d'éthylène, et leurs mélanges.

(ii) Tensioactif(s) non ionique(s) :

Les agents tensioactifs non-ioniques sont, eux aussi, des composés bien connus en soi (voir notamment à cet égard "Handbook of Surfactants" par M.R. PORTER, éditions Blackie & Son (Glasgow and London), 1991, pp 116-178) et leur nature ne revêt pas, dans le cadre de la présente invention, de caractère critique. Ainsi, ils peuvent être notamment choisis parmi (liste non limitative) les alcools, les alpha-diols, les alkylphénols, polyéthoxylés, polypropoxylés, ayant une chaîne grasse comportant par exemple 8 à 18 atomes de carbone, le nombre de groupements oxyde d'éthylène ou oxyde de propylène pouvant aller notamment de 2 à 50. On peut également citer les copolymères d'oxyde d'éthylène et de propylène, les condensats d'oxyde d'éthylène et de propylène sur des alcools gras ; les amides gras polyéthoxylés ayant de préférence de 2 à 30 moles d'oxyde d'éthylène, les amides gras polyglycérolés comportant en moyenne 1 à 5 groupements glycérol et en particulier 1,5 à 4 ; les amines grasses polyéthoxylées ayant de préférence 2 à 30 moles d'oxyde d'éthylène ; les esters d'acides gras du sorbitan oxyéthylénés ayant de 2 à 30 moles d'oxyde d'éthylène ; les esters d'acides gras du sucrose, les esters d'acides gras du polyéthylèneglycol, les alkylpolyglycosides, les dérivés de N-alkyl glucamine, les oxydes d'amines tels que les oxydes d'alkyl ($C_{10}$-$C_{14}$) amines ou les oxydes de N-acylaminopropylmorpholine.

(iii) Tensioactif(s) amphotère(s) ou zwittérionique(s) :

Les agents tensioactifs amphotères ou zwitterioniques, dont la nature ne revet pas dans le cadre de la présente invention de caractère critique, peuvent être notamment (liste non limitative) des dérivés d'amines secondaires ou tertiaires aliphatiques, dans lesquels le radical aliphatique est une chaîne linéaire ou ramifiée comportant 8 à 18 atomes de carbone et contenant au moins un groupe anionique hydrosolubilisant (par exemple carboxylate, sulfonate, sulfate, phosphate ou phosphonate) ; on peut citer encore les alkyl ($C_8$-$C_{20}$) bétaïnes, les sulfobétaïnes, les alkyl ($C_8$-$C_{20}$) amidoalkyl ($C_1$-$C_6$) betaïnes ou les alkyl ($C_8$-$C_{20}$) amidoalkyl ($C_1$-$C_6$) sulfobétaïnes.

Parmi les dérivés d'amines, on peut citer les produits vendus sous la dénomination MIRANOL, tels que décrits dans les brevets US-2 528 378 et US-2 781 354 et classés dans le dictionnaire CTFA, 3ème édition, 1982, sous les dénominations Amphocarboxyglycinates et Amphocarboxypropionates de structures respectives :

$$R_{34}\text{-CONHCH}_2\text{CH}_2\text{-N}(R_{35})(R_{36})(\text{CH}_2\text{COO}^-)$$

dans laquelle : $R_{34}$ désigne un radical alkyle d'un acide $R_{34}$-COOH présent dans l'huile de coprah hydrolysée, un radical heptyle, nonyle ou undécyle, $R_{35}$ désigne un groupement bêta-hydroxyéthyle et $R_{36}$ un groupement carboxyméthyle; et

$R_{34}$'-$CONHCH_2CH_2$-N(B)(C)

dans laquelle :

B représente $-CH_2CH_2OX'$, C représente $-(CH_2)_z-Y'$, avec z = 1 ou 2,
X' désigne le groupement $-CH_2CH_2-COOH$ ou un atome d'hydrogène
Y' désigne -COOH ou le radical $-CH_2-CHOH-SO_3H$
$R_{34}$' désigne un radical alkyle d'un acide $R_{37}$-COOH présent dans l'huile de coprah ou dans l'huile de lin hydrolysée, un radical alkyle, notamment en $C_7$, $C_9$, $C_{11}$ ou $C_{13}$, un radical alkyle en $C_{17}$ et sa forme iso, un radical $C_{17}$ insaturé.

Ces composés sont classés dans le dictionnaire CTFA, 5ème édition, 1993, sous les dénominations Disodium Cocoamphodiacetate, Disodium Lauroamphodiacetate, Disodium Caprylamphodiacetate, Disodium Capryloamphodiacetate, Disodium Coco-amphodipropionate, Disodium Lauroamphodipropionate, Disodium Caprylamphodipropionate,

Disodium Capryloamphodipropionate, Lauroamphodipropionic acid, Cocoamphodipropionic acid.
A titre d'exemple on peut citer le cocoamphodiacetate commercialisé sous la dénomination commerciale MIRANOL® C2M concentré par la société RHODIA CHIMIE.

(iv) <u>Tensioactifs cationiques</u> :

Parmi les tensioactifs cationiques on peut citer en particulier (liste non limitative) : les sels d'amines grasses primaires, secondaires ou tertiaires, éventuellement polyoxyalkylénées ; les sels d'ammonium quaternaire tels que les chlorures ou les bromures de tétraalkylammonium, d'alkylamidoalkyltrialkylammonium, de trialkylbenzylammonium, de trialkylhydroxyalkyl-ammonium ou d'alkylpyridinium; les dérivés d'imidazoline ; ou les oxydes d'amines à caractère cationique.

Les quantités d'agents tensioactifs présents dans la composition prête à l'emploi selon l'invention peuvent varier de 0,01 à 40% et de préférence de 0,1 à 30% du poids total de la composition.

D'autres adjuvants peuvent également être présents et parmi eux, des polymères conditionneurs non-ioniques, anioniques, amphotères, zwittérioniques et cationiques,
ou leurs mélanges, et de préférence des polymères substantifs cationiques ou amphotères.

Le caractère substantif (c'est à dire l'aptitude au dépôt sur les cheveux) des polymères utilisés conformément à l'invention est classiquement déterminé au moyen du test décrit par Richard J. Crawford, Journal of the Society of Cosmetic Chemists, 1980, 31 - (5) - pages 273 à 278 (révélation par colorant acide Red 80).

<u>Polymères substantifs cationiques :</u>

Au sens de la présente invention, l'expression "polymère cationique" désigne tout polymère contenant des groupements cationiques et/ou des groupements ionisables en groupements cationiques.

Les polymères cationiques utilisables conformément à la présente invention peuvent être choisis parmi tous ceux déja connus en soi comme améliorant les propriétés cosmétiques des cheveux, à savoir notamment ceux décrits dans la demande de brevet EP-A-337 354 et dans les brevets français FR-2 270 846, 2 383 660, 2 598 611, 2 470 596 et 2 519 863.

Les polymères cationiques préférés sont choisis parmi ceux qui contiennent des motifs comportant des groupements amine primaire, secondaire, tertiaire et/ou quaternaire pouvant, soit faire partie de la chaîne principale polymère, soit être portés par un substituant latéral directement relié à celle-ci.

Les polymères cationiques utilisés ont généralement une masse moléculaire moyenne en nombre comprise entre 500 et $5.10^6$ environ, et de préférence comprise entre $10^3$ et $3.10^6$ environ.

Parmi les polymères cationiques, on peut citer plus particulièrement les polymères du type polyamine, polyaminoamide et polyammonium quaternaire. Ce sont des produits connus. Ils sont notamment décrits dans les brevets français n° 2 505 348 ou 2 542 997. Parmi lesdits polymères, on peut citer :

(**1**) Les homopolymères ou copolymères dérivés d'esters ou d'amides acryliques ou méthacryliques et comportant au moins un des motifs de formules (I), (II); (III) ou (IV) suivantes:

(I)

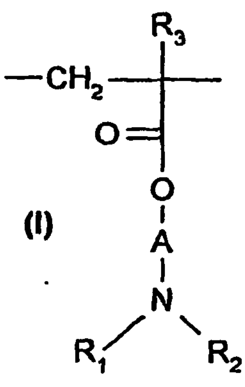

(II)

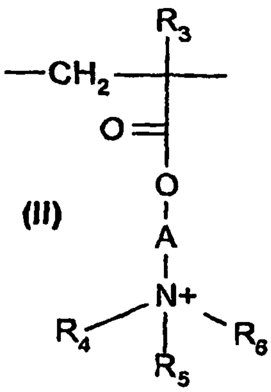

(III)

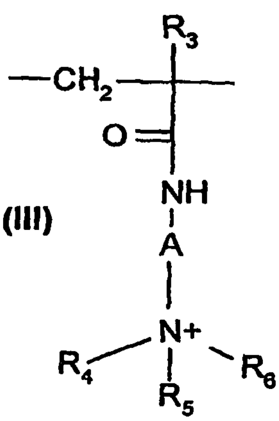

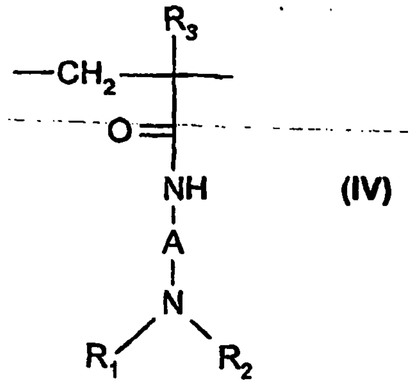

(IV)

dans lesquelles:

$R_3$, identiques ou différents, désignent un atome d'hydrogène ou un radical $CH_3$;
A, identiques ou différents, représentent un groupe alkyle, linéaire ou ramifié, de 1 à 6 atomes de carbone, de préférence 2 ou 3 atomes de carbone ou un groupe hydroxyalkyle de 1 à 4 atomes de carbone ;
$R_4$, $R_5$, $R_6$, identiques ou différents, représentent un groupe alkyle ayant de 1 à 18 atomes de carbone ou un radical benzyle et de préférence un groupe alkyle ayant de 1 à 6 atomes de carbone;
$R_1$ et $R_2$, identiques ou différents, représentent hydrogène ou un groupe alkyle ayant de 1 à 6 atomes de carbone et de préférence méthyle ou éthyle;
X désigne un anion dérivé d'un acide minéral ou organique tel qu'un anion méthosulfate ou un halogénure tel que chlorure ou bromure.

Les polymères de la famille (1) peuvent contenir en outre un ou plusieurs motifs dérivant de comonomères pouvant être choisis dans la famille des acrylamides, méthacrylamides, diacétones acrylamides, acrylamides et méthacrylamides substitués sur l'azote par des alkyles inférieurs ($C_1$-$C_4$), des acides acryliques ou méthacryliques ou leurs esters, des vinyllactames tels que la vinylpyrrolidone ou le vinylcaprolactame, des esters vinyliques.
Ainsi, parmi ces polymères de la famille (1), on peut citer :

- les copolymères d'acrylamide et de diméthylaminoéthyl méthacrylate quaternisé au sulfate de diméthyle ou avec un hologénure de diméthyle, tel que celui vendu sous la dénomination HERCOFLOC par la société HERCULES,
- les copolymères d'acrylamide et de chlorure de méthacryloyloxyéthyltriméthylammonium décrits par exemple dans la demande de brevet EP-A-080976 et vendus sous la dénomination BINA QUAT P 100 par la société CIBA GEIGY,
- le copolymère d'acrylamide et de méthosulfate de méthacryloyloxyéthyltriméthylammonium vendu sous la dénomination RETEN par la société HERCULES,
- les copolymères vinylpyrrolidone / acrylate ou méthacrylate de dialkylaminoalkyle quatemisés ou non, tels que les produits vendus sous la dénomination "GAFQUAT" par la société ISP comme par exemple "GAFQUAT 734" ou "GAFQUAT 755" ou bien les produits dénommés "COPOLYMER 845, 958 et 937". Ces polymères sont décrits en détail dans les brevets français 2.077.143 et 2.393.573,
- les terpolymères méthacrylate de diméthyl amino éthyle/ vinylcaprolactame/vinylpyrrolidone tel que le produit vendu sous la dénomination GAFFIX VC 713 par la société ISP,
- les copolymère vinylpyrrolidone / méthacrylamidopropyl dimethylamine commercialisés notamment sous la dénomination STYLEZE CC 10 par ISP,
- et les copolymères vinylpyrrolidone / méthacrylamide de diméthylaminopropyle quatemisés tel que le produit vendu sous la dénomination "GAFQUAT HS 100" par la société ISP .

(**2**) Les dérivés d'éthers de cellulose comportant des groupements ammonium quaternaire décrits dans le brevet français 1 492 597, et en particulier les polymères commercialisés sous les dénominations "JR" (JR 400, JR 125, JR 30M) ou "LR" (LR 400, LR 30M) par la Société Union Carbide Corporation. Ces polymères sont également définis dans le dictionnaire CTFA comme des ammonium quaternaires d'hydroxyéthylcellulose ayant réagi avec un époxyde substitué par un groupement triméthylammonium.

(**3**) Les dérivés de cellulose cationiques tels que les copolymères de cellulose ou les dérivés de cellulose greffés avec un monomère hydrosoluble d'ammonium quaternaire, et décrits notamment dans le brevet US 4 131 576, tels que les hydroxyalkyl celluloses, comme les hydroxyméthyl-, hydroxyéthyl- ou hydroxypropyl celluloses greffées notamment avec un sel de méthacryloyléthyl triméthylammonium, de méthacrylmidopropyl triméthylammonium, de diméthyl-diallylammonium.
Les produits commercialisés répondant à cette définition sont plus particulièrement les produits vendus sous la dénomination "Celquat L 200" et "Celquat H 100" par la Société National Starch.

(**4**) Les polysaccharides cationiques décrits plus particulièrement dans les brevets US 3 589 578 et 4 031 307 tel que les gommes de guar contenant des groupements cationiques trialkylammonium. On utilise par exemple des gommes de guar modifiées par un sel (par ex. chlorure) de 2,3-époxypropyl triméthylammonium.
De tels produits sont commercialisés notamment sous les dénominations commerciales de JAGUAR C13 S, JAGUAR C 15, JAGUAR C 17 ou JAGUAR C162 par la société MEYHALL.

(**5**) Les polymères constitués de motifs pipérazinyle et de radicaux divalents alkylène ou hydroxyalkylène à chaînes droites ou ramifiées, éventuellement interrompues par des atomes d'oxygène, de soufre, d'azote ou par des cycles aromatiques ou hétérocycliques, ainsi que les produits d'oxydation et/ou de quatemisation de ces polymères. De tels polymères sont notamment décrits dans les brevets français 2.162.025 et 2.280.361.

**(6)** Les polyaminoamides solubles dans l'eau préparés en particulier par polycondensation d'un composé acide avec une polyamine ; ces polyaminoamides peuvent être réticulés par une épihalohydrine, un diépoxyde, un dianhydride, un dianhydride non saturé, un dérivé bis-insaturé, une bis-halohydrine, un bis-azétidinium, une bis-haloacyldiamine, un bis-halogénure d'alkyle ou encore par un oligomère résultant de la réaction d'un composé bifonctionnel réactif vis-à-vis d'une bis-halohydrine, d'un bis-azétidinium, d'une bis-haloacyldiamine, d'un bis-halogénure d'alkyle, d'une épilhalohydrine, d'un diépoxyde ou d'un dérivé bis-insaturé ; l'agent réticulant étant utilisé dans des proportions allant de 0,025 à 0,35 mole par groupement amine du polyaminoamide ; ces polyaminoamides peuvent être alcoylés ou s'ils comportent une ou plusieurs fonctions amines tertiaires, quatemisées. De tels polymères sont notamment décrits dans les brevets français 2.252.840 et 2.368.508.

(**7**) Les dérivés de polyaminoamides résultant de la condensation de polyalcoylènes polyamines avec des acides polycarboxyliques suivie d'une alcoylation par des agents bifonctionnels. On peut citer par exemple les polymères acide adipique-diacoylaminohydroxyalcoyldialoylène triamine dans lesquels le radical alcoyle comporte de 1 à 4 atomes de carbone et désigne de préférence méthyle, éthyle, propyle. De tels polymères sont notamment décrits dans le brevet français 1.583.363. Parmi ces dérivés, on peut citer plus particulièrement les polymères acide adipique/diméthylaminohydroxypropyl/diéthylène triamine vendus sous la dénomination "Cartaretine F, F4 ou F8" par la société Sandoz.

(**8**) Les polymères obtenus par réaction d'une polyalkylène polyamine comportant deux groupements amine primaire et au moins un groupement amine secondaire avec un acide dicarboxylique choisi parmi l'acide diglycolique et les acides dicarboxyliques aliphatiques saturés ayant de 3 à 8 atomes de carbone. Le rapport molaire entre le polyalkylène polylamine et l'acide dicarboxylique étant compris entre 0,8 : 1 et 1,4 : 1; le polyaminoamide en résultant étant amené à réagir avec l'épichlorhydrine dans un rapport molaire d'épichlorhydrine par rapport au groupement amine secondaire du polyaminoamide compris entre 0,5 : 1 et 1,8 : 1. De tels polymères sont notamment décrits dans les brevets américains 3.227.615 et 2.961.347.
Des polymères de ce type sont en particulier commercialisés sous la dénomination "Hercosett 57" par la société Hercules Inc. ou bien sous la dénomination de "PD 170" ou "Delsette 101" par la société Hercules dans le cas du copolymère d'acide adipique/époxypropyl/diéthylène-triamine.

(**9**) Les cyclopolymères d'alkyl diallyl amine ou de dialkyl diallyl ammonium tels que les homopolymères ou copolymères comportant comme constituant principal de la chaîne des motifs répondant aux formules (V) ou (VI) :

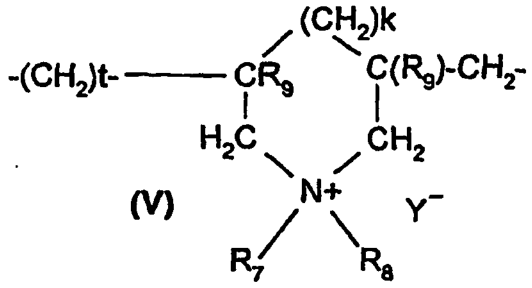

(V)

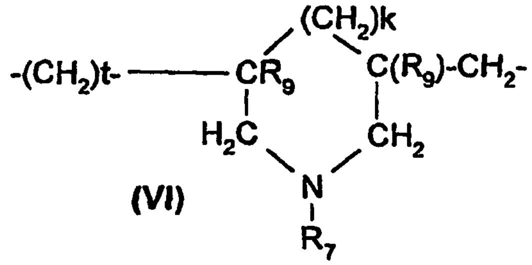

(VI)

formules dans lesquelles k et t sont égaux à 0 ou 1, la somme k + t étant égale à 1; $R_9$ désigne un atome d'hydrogène ou un radical méthyle ; $R_7$ et $R_8$, indépendamment l'un de l'autre, désignent un groupement alkyle ayant de 1 à 22 atomes de carbone, un groupement hydroxyalkyle dans lequel le groupement alkyle a de préférence 1 à 5 atomes de carbone, un groupement amidoalkyle inférieur ($C_1$-$C_4$), ou $R_7$ et $R_8$ peuvent désigner conjointement avec l'atome d'azote auquel ils sont rattachés, des groupement hétérocycliques, tels que pipéridinyle ou morpholinyle ; $R_7$ et $R_8$ indépendamment l'un de l'autre désignent de préférence un groupement alkyle ayant de 1 à 4 atomes de carbone ; $Y^-$ est un anion tel que bromure, chlorure, acétate, borate, citrate, tartrate, bisulfate, bisulfite, sulfate, phosphate. Ces polymères sont notamment décrits dans le brevet français 2.080.759 et dans son certificat d'addition 2.190.406. Parmi les polymères définis ci-dessus, on peut citer plus particulièrement l'homopolymère de chlorure de diméthyldiallylammonium vendu sous la dénomination "Merquat 100" par la société Calgon (et ses homologues de faible masse moléculaire moyenne en poids) et les copolymères de chlorure de diallyldiméthylammonium et d'acrylamide commercialisés sous la dénomination "MERQUAT 550".

(**10**) Le polymère de diammonium quaternaire contenant des motifs récurrents répondant à la formule :

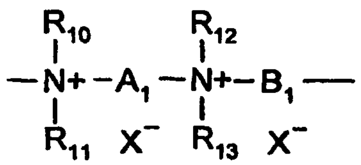

(VII)

formule (VII) dans laquelle :

$R_{10}$, $R_{11}$, $R_{12}$ et $R_{13}$, identiques ou différents, représentent des radicaux aliphatiques, alicycliques, ou arylaliphatiques contenant de 1 à 20 atomes de carbone ou des radicaux hydroxyalkylaliphatiques inférieurs, ou bien $R_{10}$, $R_{11}$, $R_{12}$ et $R_{13}$, ensemble ou séparément, constituent avec les atomes d'azote auxquels ils sont rattachés des hétérocycles contenant éventuellement un second hétéroatome autre que l'azote ou bien $R_{10}$, $R_{11}$, $R_{12}$ et $R_{13}$ représentent un radical alkyle en $C_1$-$C_6$ linéaire ou ramifié substitué par un groupement nitrile, ester, acyle, amide ou -CO-OR$_{14}$-D ou -CO-NH- R$_{14}$-D où $R_{14}$ est un alkylène et D un groupement ammonium quaternaire ;
A1 et B1 représentent des groupements polyméthyléniques contenant de 2 à 20 atomes de carbone pouvant être linéaires ou ramifiés, saturés ou insaturés, et pouvant contenir, liés à ou intercalés dans la chaîne principale, un ou plusieurs cycles aromatiques, ou un ou plusieurs atomes d'oxygène, de soufre ou des groupements sulfoxyde, sulfone, disulfure, amino, alkylamino, hydroxyle, ammonium quaternaire, uréido, amide ou ester, et $X^-$ désigne un anion dérivé d'un acide minéral ou organique;
A1, $R_{10}$ et $R_{12}$ peuvent former avec les deux atomes d'azote auxquels ils sont rattachés un cycle pipérazinique ;

en outre si A1 désigne un radical alkylène ou hydroxyalkylène linéaire ou ramifié, saturé ou insaturé, B1 peut également désigner un groupement $-(CH_2)n-CO-D-OC-(CH_2)n-$ dans lequel D désigne :

a) un reste de glycol de formule : -O-Z-O-, où Z désigne un radical hydrocarboné linéaire ou ramifié ou un groupement répondant à l'une des formules suivantes :

$$-(CH_2-CH_2-O)x-CH_2-CH_2-$$

$$-[CH_2-CH(CH_3)-O]y-CH_2-CH(CH_3)-$$

où x et y désignent un nombre entier de 1 à 4, représentant un degré de polymérisation défini et unique ou un nombre quelconque de 1 à 4 représentant un degré de polymérisation moyen ;
b) un reste de diamine bis-secondaire tel qu'un dérivé de pipérazine ;
c) un reste de diamine bis-primaire de formule : -NH-Y-NH-, où Y désigne un radical hydrocarboné linéaire ou ramifié, ou bien le radical bivalent

$$-CH_2-CH_2-S-S-CH_2-CH_2- ;$$

d) un groupement uréylène de formule: -NH-CO-NH-.

De préférence, $X^-$ est un anion tel que le chlorure ou le bromure. Ces polymères ont une masse moléculaire moyenne en nombre généralement comprise entre 1000 et 100000.
Des polymères de ce type sont notamment décrits dans les brevets français 2.320.330, 2.270.846, 2.316.271, 2.336.434 et 2.413.907 et les brevets US 2.273.780, 2.375.853, 2.388.614, 2.454.547, 3.206.462, 2.261.002, 2.271.378, 3.874.870, 4.001.432, 3.929.990, 3.966.904, 4.005.193, 4.025.617, 4.025.627, 4.025.653, 4.026.945 et 4.027.020.

On peut utiliser plus particulièrement les polymères qui sont constitués de motifs récurrents répondant à la formule (VIII) suivante:

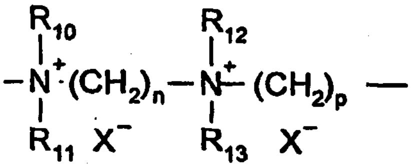

(VIII)

dans laquelle $R_{10}$, $R_{11}$, $R_{12}$ et $R_{13}$, identiques ou différents, désignent un radical alkyle ou hydroxyalkyle ayant de 1 à 4 atomes de carbone environ, n et p sont des nombres entiers variant de 2 à 20 environ et, $X^-$ est un anion dérivé d'un acide minéral
ou organique.

(**11**) Les polymères de polyammonium quaternaire constitués de motifs récurrents de formule (IX) :

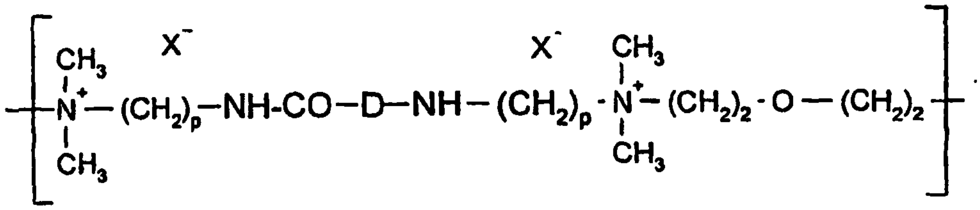

(IX)

dans laquelle p désigne un nombre entier variant de 1 à 6 environ, D peut être nul ou peut représenter un groupement $-(CH_2)_r$ -CO- dans lequel r désigne un nombre égal à 4 ou à 7, $X^-$ est un anion ;
De tels polymères peuvent être préparés selon les procédés décrits dans les brevets U.S.A. n° 4 157 388, 4 702 906, 4 719 282. Ils sont notamment décrits dans la demande de brevet EP-A-122 324.

Parmi eux, on peut par exemple citer, les produits "Mirapol A 15", "Mirapol AD1", "Mirapol AZ1" et "Mirapol 175" vendus par la société Miranol.

(**12**) Les polymères quaternaires de vinylpyrrolidone et de vinylimidazole tels que par exemple les produits commercialisés sous les dénominations Luviquat FC 905, FC 550 et FC 370 par la société B.A.S.F.

(**13**) Les polyamines comme le Polyquart H vendu par HENKEL, référencé sous le nom de " POLYETHYLENEGLYCOL (15) TALLOW POLYAMINE " dans le dictionnaire CTFA.

(**14**) Les polymères réticulés de sels de méthacryloyloxyalkyl($C_1$-$C_4$) trialkyl($C_1$-$C_4$)ammonium tels que les polymères obtenus par homopolymérisation du diméthylaminoéthylméthacrylate quaternisé par le chlorure de méthyle, ou par copolymérisation de l'acrylamide avec le diméthylaminoéthylméthacrylate quaternisé par le chlorure de méthyle, l'homo ou la copolymérisation étant suivie d'une réticulation par un composé à insaturation oléfinique, en particulier le méthylène bis acrylamide. On peut plus particulièrement utiliser un copolymère réticulé acrylamide/chlorure de méthacryloyloxyéthyl triméthylammonium (20/80 en poids) sous forme de dispersion contenant 50 % en poids dudit copolymère dans de l'huile minérale. Cette dispersion est commercialisée sous le nom de " SALCARE® SC 92 " par la Société ALLIED COLLOIDS. On peut également utiliser un homopolymère réticulé du chlorure de méthacryloyloxyéthyl triméthylammonium contenant environ 50 % en poids de l'homopolymère dans de l'huile minérale ou dans un ester liquide. Ces dispersions sont commercialisées sous les noms de " SALCARE® SC 95 " et " SALCARE® SC 96 " par la Société ALLIED COLLOIDS.

D'autres polymères cationiques utilisables dans le cadre de l'invention sont des polyalkylèneimines, en particulier des polyéthylèneimines, des polymères contenant des motifs vinylpyridine ou vinylpyridinium, des condensats de polyamines et d'épichlorhydrine, des polyuréylènes quaternaires et les dérivés de la chitine.

Parmi tous les polymères cationiques susceptibles d'être utilisés dans le cadre de la présente invention, on préfère mettre en oeuvre les polymères des familles (1), (9), (10) (11) et (14) et encore plus préférentiellement les polymères aux motifs récurrents de formules (W) et (U) suivantes :

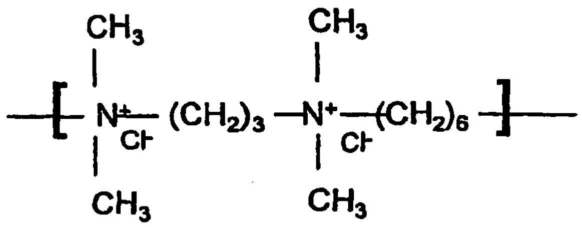

**(W)**

et notamment ceux dont le poids moléculaire, déterminé par chromatographie par perméation de gel, est compris entre 9500 et 9900;

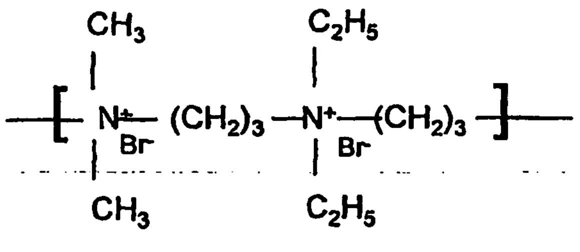

**(U)**

et notamment ceux dont le poids moléculaire, déterminé par chromatographie par perméation de gel, est d'environ 1200.

La concentration en polymère substantif cationique dans la composition selon la présente invention peut varier de 0,01 à 10% en poids par rapport au poids total de la composition, de préférence de 0,05 à 5% et plus préférentiellement encore de 0,1 à 3%.

<u>Polymères substantifs amphotères :</u>

Les polymères amphotères utilisables conformément à la présente invention peuvent être choisis parmi les polymères comportant des motifs K et M répartis statistiquement dans la chaîne polymère, où K désigne un motif dérivant

d'un monomère comportant au moins un atome d'azote basique et M désigne un motif dérivant d'un monomère acide comportant un ou plusieurs groupements carboxyliques ou sulfoniques, ou bien K et M peuvent désigner des groupements dérivant de monomères zwittérioniques de carboxybétaïnes ou de sulfobétaïnes;
K et M peuvent également désigner une chaîne polymère cationique comportant des groupements amine primaire, secondaire, tertiaire ou quaternaire, dans laquelle au moins l'un des groupements amine porte un groupement carboxylique ou sulfonique relié par l'intermédiaire d'un radical hydrocarboné, ou bien K et M font partie d'une chaîne d'un polymère à motif éthylène $\alpha,\beta$-dicarboxylique dont l'un des groupements carboxyliques a été amené à réagir avec une polyamine comportant un ou plusieurs groupements amine primaire ou secondaire.

Les polymères amphotères répondant à la définition donnée ci-dessus plus particulièrement préférés sont choisis parmi les polymères suivants :

(1) Les polymères résultant de la copolymérisation d'un monomère dérivé d'un composé vinylique portant un groupement carboxylique tel que plus particulièrement l'acide acrylique, l'acide méthacrylique, l'acide maléique, l'acide alpha-chloracrylique, et d'un monomère basique dérivé d'un composé vinylique substitué contenant au moins un atome basique tel que plus particulièrement les dialkylaminoalkylméthacrylate et acrylate, les dialkylaminoalkylméthacrylamide et acrylamide. De tels composés sont décrits dans le brevet américain n° 3 836 537. On peut également citer le copolymère acrylate de sodium / chlorure d'acrylamidopropyl trimethyl ammonium vendu sous la dénomination POLYQUART KE 3033 par la Société HENKEL.
Le composé vinylique peut être également un sel de dialkyldiallylammonium tel que le chlorure de diméthyldiallylammonium. Les copolymères d'acide acrylique et de ce dernier monomère sont proposés sous les appelations MERQUAT 280, MERQUAT 295 et MERQUAT PLUS 3330 par la société CALGON.

(2) Les polymères comportant des motifs dérivant :

a) d'au moins un monomère choisi parmi les acrylamides ou les méthacrylamides substitués sur l'azote par un radical alkyle,
b) d'au moins un comonomère acide contenant un ou plusieurs groupements carboxyliques réactifs, et
c) d'au moins un comonomère basique tel que des esters à substituants amine primaire, secondaire, tertiaire et quaternaire des acides acrylique et méthacrylique et le produit de quaternisation du méthacrylate de diméthylaminoéthyle avec le sulfate de diméthyle ou diéthyle.

Les acrylamides ou méthacrylamides N-substitués plus particulièrement préférés selon l'invention sont les groupements dont les radicaux alkyle contiennent de 2 à 12 atomes de carbone et plus particulièrement le N-éthylacrylamide, le N-tertiobutyl-acrylamide, le N-tertiooctyl-acrylamide, le N-octylacrylamide, le N-décylacrylamide, le N-dodécylacrylamide ainsi que les méthacrylamides con-espondants.
Les comonomères acides sont choisis plus particulièrement parmi les acides acrylique, méthacrylique, crotonique, itaconique, maléique, fumarique ainsi que les monoesters d'alkyle ayant 1 à 4 atomes de carbone des acides ou des anhydrides maléique ou fumarique.
Les comonomères basiques préférés sont des méthacrylates d'aminoéthyle, de butyl aminoéthyle, de N,N'-diméthylaminoéthyle, de N-tertio-butylaminoéthyle.
On utilise particulièrement les copolymères dont la dénomination CTFA (4ème Ed., 1991) est Octylacrylamidelacrylates/butylaminoethylmethacrylate copolymer tels que les produits vendus sous la dénomination AMPHOMER ou LOVOCRYL 47 par la société NATIONAL STARCH.

(3) Les polyaminoamides réticulés et alcoylés partiellement ou totalement dérivant de polyaminoamides de formule générale :

$$\left[ CO-R_{19}-CO-Z \right] \qquad \textbf{(X)}$$

dans laquelle $R_{19}$ représente un radical divalent dérivé d'un acide dicarboxylique saturé, d'un acide aliphatique mono ou dicarboxylique à double liaison éthylénique, d'un ester d'un alcanol inférieur ayant 1 à 6 atomes de carbone de ces acides ou d'un radical dérivant de l'addition de l'un quelconque desdits acides avec une amine bis primaire ou bis secondaire, et Z désigne un radical d'une polyalkylène-polyamine bis-primaire, mono ou bis-secondaire et de préférence représente :

a) dans les proportions de 60 à 100 moles %, le radical

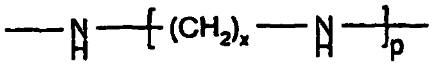 (XI)

où x=2 et p=2 ou 3, ou bien x=3 et p=2 ce radical dérivant de la diéthylène triamine, de la triéthylène tétraamine ou de la dipropylène triamine;

b) dans les proportions de 0 à 40 moles % le radical (XI) ci-dessus, dans lequel x=2 et p=1 et qui dérive de l'éthylènediamine, ou le radical dérivant de la pipérazine :

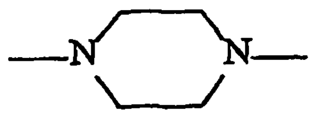

c) dans les proportions de 0 à 20 moles % le radical $-NH-(CH_2)_6-NH-$ dérivant de l'hexaméthylènediamine, ces polyaminoamines étant réticulées par addition d'un agent réticulant bifonctionnel choisi parmi les épihalohydrines, les diépoxydes, les dianhydrides, les dérivés bis insaturés, au moyen de 0,025 à 0,35 mole d'agent réticulant par groupement amine du polyaminoamide et alcoylés par action d'acide acrylique, d'acide chloracétique ou d'une alcane sultone ou de leurs sels. Les acides carboxyliques saturés sont choisis de préférence parmi les acides ayant 6 à 10 atomes de carbone tels que l'acide adipique, triméthyl-2,2,4-adipique et triméthyl-2,4,4-adipique, téréphtalique, les acides à double liaison éthylénique comme par exemple les acides acrylique, méthacrylique, itaconique. Les alcanes sultones utilisées dans l'alcoylation sont de préférence la propane ou la butane sultone, les sels des agents d'alcoylation sont de préférence les sels de sodium

ou de potassium.

(4) Les polymères comportant des motifs zwittérioniques de formule :

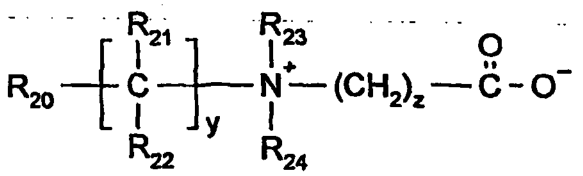 (XII)

dans laquelle $R_{20}$ désigne un groupement insaturé polymérisable tel qu'un groupement acrylate, méthacrylate, acrylamide ou méthacrylamide, y et z représentent un nombre entier de 1 à 3, $R_{21}$ et $R_{22}$ représentent un atome d'hydrogène, méthyle, éthyle ou propyle, $R_{23}$ et $R_{24}$ représentent un atome d'hydrogène ou un radical alkyle de telle façon que la somme des atomes de carbone dans $R_{23}$ et $R_{24}$ ne dépasse pas 10.

Les polymères comprenant de telles unités peuvent également comporter des motifs dérivés de monomères non zwittérioniques tels que l'acrylate ou le méthacrylate de diméthyl ou diéthylaminoéthyle ou des alkyle acrylates ou méthacrylates, des acrylamides ou méthacrylamides ou l'acétate de vinyle.

A titre d'exemple, on peut citer le copolymère de méthacrylate de butyle / méthacrylate de diméthylcarboxyméthylammonio-éthyle tel que le produit vendu sous la dénomination DIAFORMER Z301 par la société SANDOZ.

(5) les polymères dérivés du chitosane comportant des motifs monomères répondant aux formules (XIII), (XIV), (XV) suivantes :

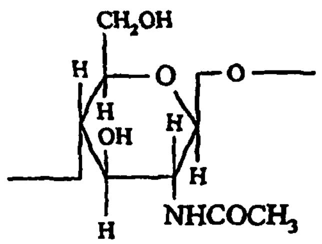

(XIII)

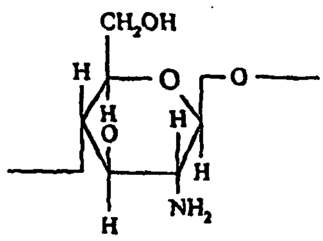

(XIV)

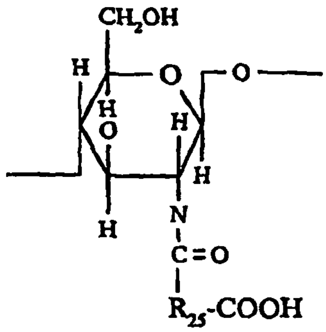

(XV)

le motif (XIII) étant présent dans des proportions comprises entre 0 et 30%, le motif (XIV) dans des proportions comprises entre 5 et 50% et le motif (XV) dans des proportions comprises entre 30 et 90%, étant entendu que dans ce motif (XV), $R_{25}$ représente un radical de formule:

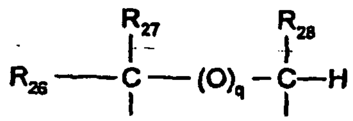

dans laquelle q désigne zéro ou 1 ;
si q=0, $R_{26}$, $R_{27}$ et $R_{28}$, identiques ou différents, représentent chacun un atome d'hydrogène, un reste méthyle, hydroxyle, acétoxy ou amino, un reste monoalcoylamine ou un reste dialcoylamine éventuellement interrompus par un ou plusieurs atomes d'azote et/ou éventuellement substitués par un ou plusieurs groupes amine, hydroxyle, carboxyle, alcoylthio, sulfonique, un reste alcoylthio dont le groupe alcoyle porte un reste amino, l'un au moins des radicaux $R_{26}$, $R_{27}$ et $R_{28}$ étant dans ce cas un atome d'hydrogène ;
ou si q=1, $R_{26}$, $R_{27}$ et $R_{28}$ représentent chacun un atome d'hydrogène, ainsi que les sels formés par ces composés avec des bases ou des acides.

(6) Les polymères dérivés de la N-carboxyalkylation du chitosane comme le N-carboxyméthyl chitosane ou le N-carboxybutyl chitosane vendu sous la dénomination "EVALSAN" par la société JAN DEKKER.

(7) Les polymères répondant à la formule générale (XI) tels que ceux décrits par exempte dans le brevet français 1 400 366 :

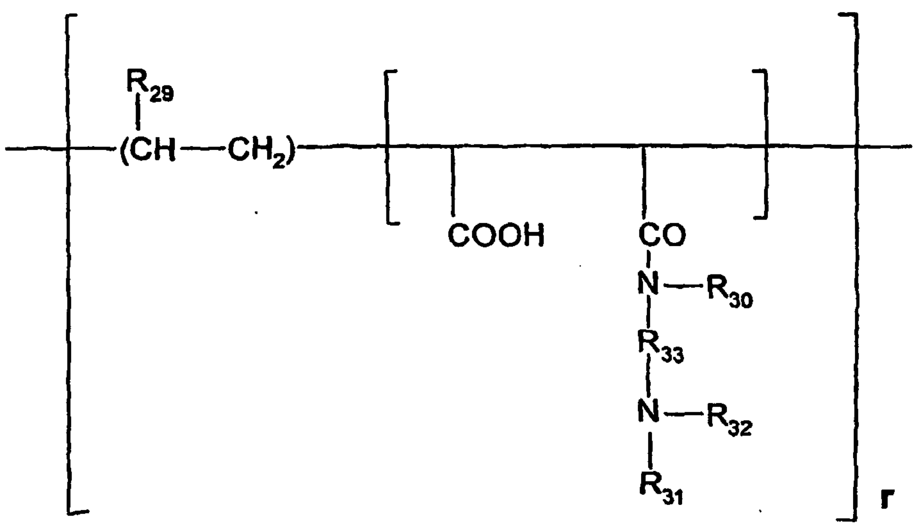

(XVI)

dans laquelle $R_{29}$ représente un atome d'hydrogène, un radical $CH_3O$, $CH_3CH_2O$, phényle, $R_{30}$ désigne l'hydrogène ou un radical alkyle inférieur tel que méthyle, éthyle, $R_{31}$désigne l'hydrogène ou un radical alkyle inférieur tel que méthyle, éthyle, $R_{32}$ désigne un radical alkyle inférieur tel que méthyle, éthyle ou un radical répondant à la formule : $-R_{33}-N(R_{31})_2$, $R_{33}$ représentant un groupement $-CH_2-CH_2-$ , $-CH_2-CH_2-CH_2-$ , $-CH_2-CH(CH_3)-$ , $R_{31}$ ayant les significations mentionnées ci-dessus,
ainsi que les homologues supérieurs de ces radicaux et contenant jusqu'à 6 atomes de carbone,
r est tel que le poids moléculaire est compris entre 500 et 6000000 et de préférence entre 1000 et 1000000;

(8) Des polymères amphotères du type -D-X-D-X- choisis parmi:

a) les polymères obtenus par action de l'acide chloracétique ou le chloracétate de sodium sur les composés comportant au moins un motif de formule :

-D-X-D-X-D- (XVII)

où D désigne un radical

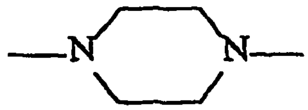

et X désigne le symbole E ou E', E ou E' identiques ou différents désignent un radical bivalent qui est un radical alkylène à chaîne droite ou ramifiée comportant jusqu'à 7 atomes de carbone dans la chaîne principale non substituée ou substituée par des groupements hydroxyle et pouvant comporter en outre des atomes d'oxygène, d'azote, de soufre, 1 à 3 cycles aromatiques et/ou hétérocycliques; les atomes d'oxygène, d'azote et de soufre étant présents sous forme de groupements éther, thioéther, sulfoxyde, sulfone, sulfonium, alkylamine, alkénylamine, des groupements hydroxyle, benzylamine, oxyde d'amine, ammonium quaternaire, amide, imide, alcool, ester et/ou uréthanne ;

b) les polymères de formule :

-D-X-D-X- (XVIII)

où D désigne un radical

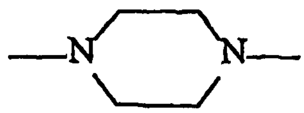

et X désigne le symbole E ou E' et au moins une fois E'; E ayant la signification indiquée ci-dessus et E' est un radical bivalent qui est un radical alkylène à chaîne droite ou ramifiée ayant jusqu'à 7 atomes de carbone dans la chaîne principale, substitué ou non par un ou plusieurs radicaux hydroxyle et comportant un ou plusieurs atomes d'azote, l'atome d'azote étant substitué par une chaîne alkyle interrompue éventuellement par un atome d'oxygène et comportant obligatoirement une ou plusieurs fonctions carboxyle ou une ou plusieurs fonctions hydroxyle et bétaïnisées par réaction avec l'acide chloracétique ou du chloracétate de soude.

(9) Les copolymères alkyl($C_1$-$C_5$)vinyléther / anhydride maléique modifié partiellement par semiamidification avec une N,N-dialkylaminoalkylamine telle que la N,N-diméthylaminopropylamine ou par semiestérification avec une N,N-dialcanolamine. Ces copolymères peuvent également comporter d'autres comonomères vinyliques tels que le vinylcaprolactame.

Les polymères amphotères particulièrement préférés selon l'invention sont ceux de la famille (1).

Selon l'invention, le ou les polymères substantifs amphotères peuvent représenter de 0,01 % à 10 % en poids, de préférence de 0,05 % à 5 % en poids, et encore plus préférentiellement de 0,1 % à 3 % en poids, du poids total de la composition.

D'autres adjuvants, tels que des agents de pénétration, des agents séquestrants, des parfums, des agents dispersants, des silicones volatiles ou non volatiles, modifiées ou non modifiées, des agents filmogènes, des céramides, des agents conservateurs, des agents opacifiants, des huiles minérales ou végétales, des cires, des vitamines, peuvent également être présents dans la composition de décoloration utile selon l'invention.

Bien entendu, l'homme de l'art veillera à choisir ce ou ces éventuels composés complémentaires de manière telle que les propriétés avantageuses attachées intrinsèquement à la composition de décoloration utile selon l'invention, ne soient pas, ou substantiellement pas, altérées par la ou les adjonctions envisagées.

La composition utile selon l'invention, peut se présenter sous des formes diverses, telles que sous forme de solutions, d'émulsions, de crèmes, de gels, éventuellement pressurisés sous forme de mousses, ou sous toute autre forme appropriée pour réaliser une décoloration des fibres kératiniques humaines, et notamment des cheveux.

Elle peut être obtenue par mélange extemporané au moment de l'emploi, soit :

(a)d'une composition anhydre contenant au moins un dérivé d'acide sulfinique de formule (I) tel que défini ci-avant ou l'un de ses sels, et d'une composition aqueuse à pH acide, soit,

(b)de deux compositions aqueuses dont une contient au moins un dérivé d'acide sulfinique de formule (I) tel que défini ci-avant ou l'un de ses sels à pH acide ou alcalin, l'autre une composition aqueuse à pH acide.

De préférence selon la présente invention, la composition de décoloration à pH compris entre 1,5 et 9 et de préférence entre 1,8 et 6 est obtenue par mélange extemporané au moment de l'emploi, de deux compositions aqueuses, dont l'une contient au moins un dérivé d'acide sulfinique de formule (I) tel que défini ci-avant ou l'un de ses sels à pH acide ou alcalin, et l'autre une composition aqueuse à pH acide.

Dans ce cas et préférentiellement, la composition aqueuse à pH acide est sous forme de gel ou d'émulsion et peut contenir un parfum, l'émulsion contenant au moins une huile ou un corps gras et au moins un émulsionnant hydrosoluble.

Selon le procédé de décoloration, on applique sur les fibres teintes, à une température d'application comprise entre la température ambiante et 80°C, au moins une composition de décoloration telle que définie précédemment, pendant un temps suffisant pour dégrader partiellement ou totalement la coloration née de la teinture oxydante ou non oxydante des fibres kératiniques. De façon préférentielle, les fibres sont ensuite rincées, ou éventuellement lavées au shampooing, puis séchées.

Une variante de ce procédé consiste, après rinçage et shampooing, à procéder à une étape de neutralisation à l'aide s'une solution aqueuse de peroxyde d'hydrogène, puis à rincer à nouveau, laver éventuellement au shampooing, puis sécher.

La température d'application est de préférence comprise entre la température ambiante et 60°C et encore plus préférentiellement entre 35°C et 50°C.

Le temps suffisant au développement de la décoloration des fibres kératiniques humaines est généralement compris entre 1 et 60 minutes et encore plus précisément entre 5 et 30 minutes.

Les exemples qui suivent sont destinés à illustrer l'invention sans pour autant en limiter la portée.

Dans ces exemples, on a évalué la performance de la décoloration selon l'invention en mesurant la décoloration ΔE des cheveux à l'aide d'un colorimètre MINOLTA CM 2002 dans le système international CIE L*a*b*.

Selon ce système, plus la valeur de L est élevée, plus la couleur est claire ou peu intense. Inversement, plus la valeur de L est faible, plus la couleur est foncée ou très intense [L*=0 est noir, L*=100 est blanc].

a* et b* indiquent deux axes de couleurs,

a* indique l'axe de couleur vert/rouge (+a* est rouge, -a* est vert)) et

b* l'axe de couleur bleu/jaune (+b* est jaune et - b* est bleu).

Des valeurs proches de zéro pour a* et b* correspondent à des nuances grises.

La décoloration ΔE est calculée en appliquant l'équation suivante :

$$\Delta E = \sqrt{(L^*-L_o^*)^2 + (a^*-a_o^*)^2 + (b^*-b_o^*)^2}$$

Dans cette équation, ΔE représente la différence de couleur entre deux mèches, (dans le cas présent, la décoloration), L* , a* , et b* représentent respectivement les mesures de la mèche décolorée, $L_o^*$ , $a_o^*$ et $b_o^*$ représentant respectivement les mesures de la mèche témoin avant qu'elle n'ait été colorée.

Plus la valeur de ΔE est faible, plus la différence de couleur entre les deux mèches est faible, et dans le cas présent, plus la décoloration est importante.

EXEMPLES 1 et 2

Des mèches de cheveux ont été préalablement teintes par une teinture d'oxydation du commerce MAJIROUGE Nuance 6.66 à laquelle on a ajouté 0,039% en poids du colorant direct cationique BASIC RED 51 (colorant de formule (IV2) et 0,035% en poids du colorant direct cationique BASIC YELLOW 87 (colorant de formule (VI4).
Elles ont ensuite été décolorées par immersion pendant 20 minutes dans les solutions des exemples 1 et 2 suivants à raison de 10 g de solution pour 1g de cheveux traité. Après rinçage à l'eau, shampooing et séchage, elles ont été lues au colorimètre.

(concentrations exprimées en grammes)

| | Exemple 1 | Exemple 2 |
|---|---|---|
| Brüggolite FF6* vendu par la société BRUGGEMANN | 10** | |
| Sulfite : $Na_2SO_3$ | | 3,5*** |
| Oléfine sulfonate de sodium | 0,25 | 0,25 |
| Acide orthophosphorique qs.pH | 2,7 | 2,7 |
| Eau qsp | 100 | 100 |
| * 50% poids du dérivé d'acide sulfinique de formule (I): $NaSO_2$-CHOH-COONa, 15% poids de $NaSO_3$-CHOH-COONa, 35% poids de sulfite : $Na_2SO_3$<br>** = 0,028 mole de dérivé d'acide sulfinique de formule (I): $NaSO_2$-CHOH-COONa,<br>***= 0,028 mole de sulfite $Na_2SO_3$ | | |

Sur une moyenne de trois mèches teintes, les résultats ont été les suivants :

| Exemples | 1 | 2 |
|---|---|---|
| ΔE Moyen | 25 | 42,4 |
| Ecart type | 1,4 | 0,8 |

Ces résultats montrent que le composé de formule (I) selon l'invention décolore beaucoup plus (ΔE =25) que le sulfite ($Na_2SO_3$) de l'art antérieur (ΔE =42,4), le résultat en terme de retour à la teinte initiale des cheveux (avant que ceux-ci ne soient teints) étant plus faible.

## Revendications

1. Utilisation pour la décoloration des fibres kératiniques humaines teintes par des colorants d'oxydation et/ou des colorants directs d'une comprenant, composition cosmétique dans un milieu cosmétiquement acceptable à un pH compris entre 1,5 et 9, à titre de réducteur de colorant, au moins un dérivé d'acide sulfinique de formule (I) suivante

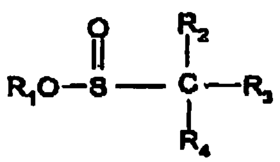 (I)

dans laquelle,

$R_1$ est choisi parmi, un atome d'hydrogène, un ion $NH3^+$, un ion de métal monovalent ou un équivalent ionique de métal bivalent des groupes Ia, IIa, IIb, IVa et VIIIb du système périodique des éléments,
$R_2$ est choisi parmi, un radical OH, un radical $NR_5R_6$ dans lequel $R_5$ et $R_6$, identiques ou différents, sont choisis parmi un atome d'hydrogène ou un radical alkyle en $C_1$-$C_6$,
$R_3$ est choisi parmi, un atome d'hydrogène, un radical alkyle ou alcényle ou cycloalkyle ou aryle non substitué

ou substitué par 1 à 3 substituants choisis, identiques ou différents, parmi les radicaux OH, alkyle en $C_1$-$C_6$, O-alkyle en $C_1$-$C_6$, halogène ou $CF_3$,
$R_4$ est choisi parmi, un radical $COOR_1$, $SO_3R_1$, $COR_5$, $CONR_5R_6$ ou $COOR_5$ dans lesquels $R_1$, $R_5$, et $R_6$ ont les siginifications précédentes,
et $R_4$ désigne également un atome d'hydrogène, lorsque $R_3$ désigne un radical aryle, et en particulier, un radical aryle substitué comme décrit précédemment, et les sels desdits composés cosmétiquement acceptables.

2. Utilisation selon la revendication 1, **caractérisée par** le fait que dans la formule (I), $R_1$ est choisi parmi un ion $NH3^+$, un ion de métal alcalin, un équivalent ionique de métal alcalino-terreux ou de zinc.

3. Utilisation selon la revendication 1, **caractérisée par** le fait que dans la formule (I), $R_2$ est un radical OH ou un radical $NH_2$.

4. Utilisation selon la revendication 1, **caractérisée par** le fait que dans la formule (I), $R_3$ est choisi parmi, un atome d'hydrogène, un radical alkyle non substitué ou substitué par un ou deux radicaux OH ou un ou deux radicaux alkyle en $C_1$-$C_6$ ou O-alkyle en $C_1$-$C_6$.

5. Utilisation selon la revendication 1, **caractérisée par** le fait que dans la formule (I), $R_4$ est un radical $COOR_1$ ou $COOR_5$, dans lesquels $R_1$ et $R_5$ ont les significations de la revendication 1.

6. Utilisation selon la revendication 1, **caractérisée par** le fait que dans la formule (I), $R_1$ est Na, $R_2$ est OH, $R_3$ est H et $R_4$ est COONa, c'est à dire le composé de formule (II) suivante :

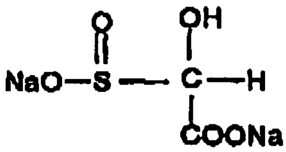

(II)

7. Utilisation selon la revendication 1, **caractérisée par** le fait que la composition cosmétique comprend 40 à 60% en poids d'un composé de formule (II), 10 à 20% en poids de $NaSO_3$-CHOH-COONa et de 30 à 40% de $Na_2SO_3$.

8. Utilisation selon l'une quelconque des revendications 1 à 7, **caractérisée par** le fait que la composition cosmétique présente un pH compris entre 1,8 et 6.

9. Utilisation selon l'une quelconque des revendications 1 à 8, **caractérisée par** le fait que le ou les composés de formule (I) représentent 0,01 à 20% en poids du poids total de la composition.

10. Utilisation selon la revendication 9, **caractérisée par** le fait qu'ils représentent 0,1 à 10% en poids du poids total de la composition.

11. Utilisation selon l'une quelconque des revendications 1 à 10, **caractérisée par** le fait que la composition cosmétique contient au moins un agent épaississant.

12. Utilisation selon la revendication 11 , **caractérisée par** le fait que l'agent épaississant est un dérivé de cellulose, un dérivé de guar, une gomme d'origine microbienne ou végétale, un épaississant synthétique.

13. Utilisation selon les revendications 11 ou 12, **caractérisée par** le fait que le ou les agents épaississants représentent 0,01 à 10% en poids du poids total de la composition.

14. Utilisation selon l'une quelconque des revendications 1 à 13, **caractérisée par** le fait que la composition cosmétique contient en outre au moins un agent alcalinisant ou acidifiant dans des quantités allant de 0,01 à 30% en poids du poids total de la composition.

15. Utilisation selon la revendication 14, **caractérisée par** le fait que l'agent acidifiant est choisi parmi les acides minéraux ou organiques comme l'acide étidronique, l'acide chlorhydrique, l'acide orthophosphorique, les acides carboxyliques comme l'acide tartrique, l'acide citrique, l'acide lactique, ou les acides sulfoniques.

**16.** Utilisation selon la revendication 14 , **caractérisée par** le fait que l'agent alcalinisant est choisi parmi l'ammoniaque, les carbonates alcalins, les alcanolamines telles que les mono-, di- et triéthanolamines, le 2-méthyl-2-amino-1-propanol ainsi que leurs dérivés, les hydroxydes de sodium ou de potassium et les composés de formule (III) suivante :

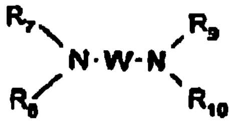 (III)

dans laquelle W est un reste propylène éventuellement substitué par un groupement hydroxyle ou un radical alkyle en $C_1$-$C_4$ ; $R_7$, $R_8$, $R_9$ et $R_{10}$, identiques ou différents, représentent un atome d'hydrogène, un radical alkyle en $C_1$-$C_4$ ou hydroxyalkyle en $C_1$-$C_4$.

**17.** Utilisation selon l'une quelconque des revendications 1 à 16, **caractérisée par** le fait que la composition cosmétique est obtenue par mélange extemporané au moment de l'emploi d'une composition anhydre contenant au moins un dérivé d'acide sulfinique de formule (I) et d'une composition aqueuse à un pH compris entre 1,5 et 9.

**18.** Utilisation selon la revendication 17, **caractérisée par** le fait que la composition cosmétique est obtenue par mélange extemporané au moment de l'emploi d'une composition anhydre contenant au moins un dérivé d'acide sulfinique de formule (I) et d'une composition aqueuse à un pH compris entre 1,8 et 6.

**19.** Utilisation selon la revendication 16, **caractérisée par** le fait que la composition cosmétique est obtenue par mélange extemporané au moment de l'emploi, de deux compositions aqueuses, dont l'une contient au moins un dérivé d'acide sulfinique de formule (I), à pH acide ou alcalin, et l'autre une composition aqueuse à pH acide.

**20.** Utilisation selon l'une quelconque des revendications 1 à 19, **caractérisée par** le fait que la composition cosmétique se présente sous forme de gel, de crème, de mousse, de solution ou d'émulsion.

**21.** Dispositif à plusieurs compartiments destiné (i)à la teinture puis (ii)à la décoloration des fibres kératiniques humaines teintes par des colorants d'oxydation et/ou des colorants directs, en particulier des cheveux, **caractérisé par** le fait qu'il comprend un premier compartiment comprenant une composition pour la teinture oxydante ou la teinture non-oxydante desdites fibres, et dans un second compartiment une composition pour la décoloration réductrice desdites fibres colorées et telle que définie à l'une quelconque des revendications 1 à 20.

**22.** Dispositif selon la revendication 21, **caractérisée par** le fait que dans le premier compartiment, la composition pour la teinture oxydante contient des bases d'oxydation et/ou des coupleurs qui forment une teinture d'oxydation par mélange avec un oxydant.

**23.** Dispositif selon la revendication 22, **caractérisée par** le fait que dans le premier compartiment, la composition pour la teinture oxydante contient en outre des colorants directs.

**24.** Dispositif selon la revendication 21, **caractérisée par** le fait que dans le premier compartiment, la composition pour la teinture oxydante contient des colorants directs de préférence cationiques qui forment une teinture directe éclaircissante par mélange avec un oxydant.

**25.** Dispositif selon la revendication 21, **caractérisée par** le fait que dans le premier compartiment, la composition pour la teinture non oxydante contient des colorants directs et de préférence des colorants directs cationiques.

**26.** Dispositif selon les revendications 24 ou 25, **caractérisé par** le fait que les colorants directs cationiques sont choisis parmi les colorants de structures (I) à (VII) suivantes et leurs formes mésomères :

(i) colorants de formules (I), (II)a, (II)b, (III)a, (III)b:

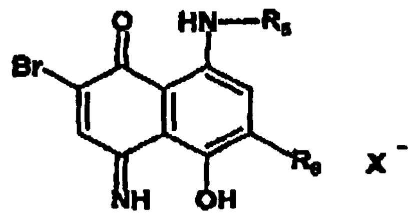

(I)

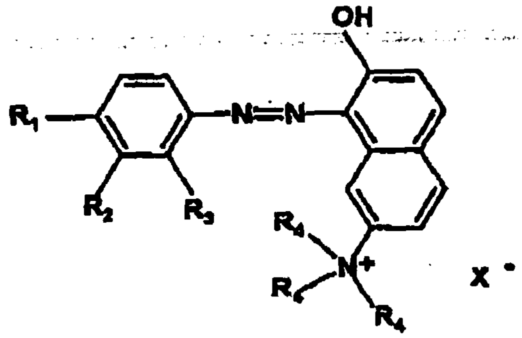

(II)a

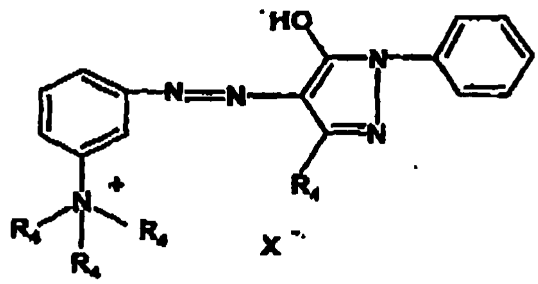

(II)b

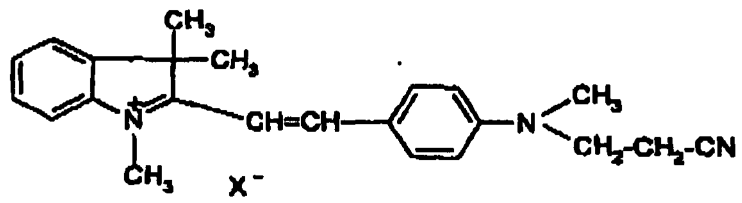

(III)a

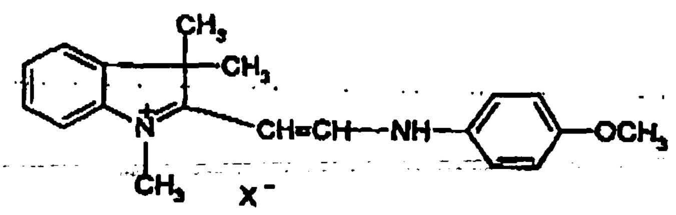

(III)b

formules (I) , (II)a, (II)b, (III)a, (III)b dans lesquelles :

$R_1$ représente un atome d'hydrogène ou un radical amino ;
$R_2$ représente un atome d'hydrogène ou un groupement nitro ;
$R_3$ représente un atome d'hydrogène, un groupement nitro ou un radical alcoxy en $C_1$-$C_4$ ;
$R_4$ représente un radical alkyle en $C_1$-$C_4$ ;
$R_5$ représente un atome d'hydrogène ou un groupement paratrialkyl$C_1$-$C_4$ ammoniophényle ;
$R_6$ représente un atome de brome ou un groupement NHparatrialkyle$C_1$-$C_4$ ammoniophényle ;

$X^-$ représente un anion ;

(ii) colorants de formules (IV), (V), (VI), (VI'), (VII) :

a) les composés de formule (IV) suivante :

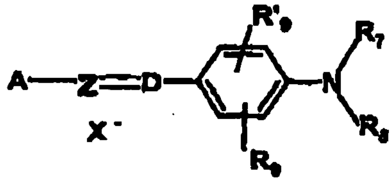

(IV)

dans laquelle:

Z et D représentent, identiques ou différents, un atome d'azote ou le radical -CH-,
$R_7$ et $R_8$, identiques ou différents, représentent un atome d'hydrogène ; un radical alkyle en $C_1$-$C_4$ pouvant être substitué par un radical -CN, -OH ou $-NH_2$ ou forment avec un atome de carbone du cycle benzénique un hétérocycle éventuellement oxygéné ou azoté, pouvant être substitué par un ou plusieurs radicaux alkyle en $C_1$-$C_4$ ; un radical 4'-aminophényle,
$R_9$ et $R'_9$, identiques ou différents, représentent un atome d'hydrogène ou d'halogène, un radical cyano, alkyl en $C_1$-$C_4$, alcoxy en $C_1$-$C_4$ ou acétyloxy,
$X^-$ représente un anion ;
A représente un groupement choisi par les structures A1 à A19 suivantes :

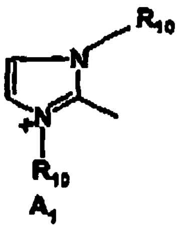
$A_1$ ;

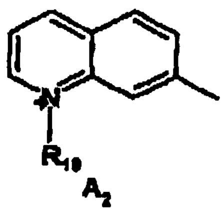
$A_2$ ;

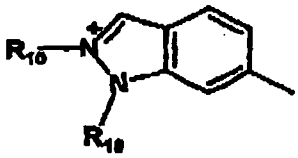
$A_3$

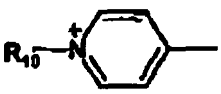
$A_4$ ;

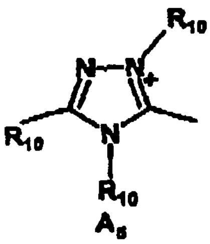
$A_5$ ;

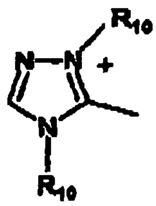
$A_6$ ;

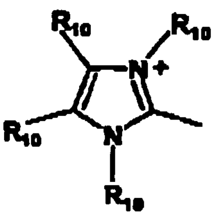
$A_7$ ;

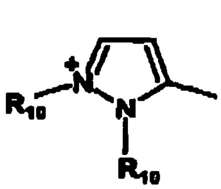
$A_8$ ;

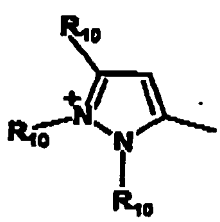
$A_9$ ;

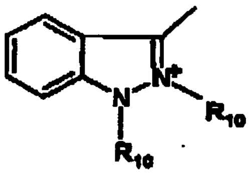

A10 ;

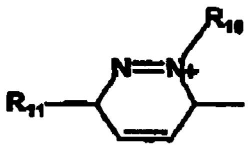

A11 ;

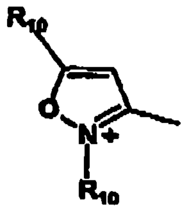

A12 ;

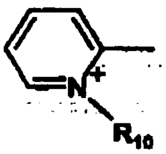

A13 ;

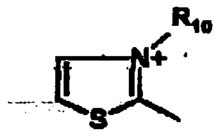

A14 ;

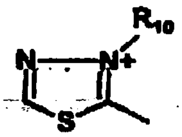

A15

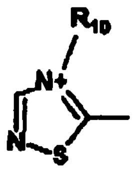

A16 ;

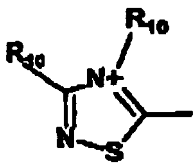

A17 ;

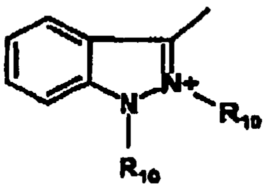

A18

et

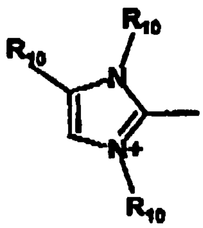

A19

dans lesquelles $R_{10}$ représente un radical alkyle en $C_1$-$C_4$ pouvant être substitué par un radical hydroxyle et $R_{11}$ représente un radical alcoxy en $C_1$-$C_4$,;

b) les composés de formule (V) suivante :

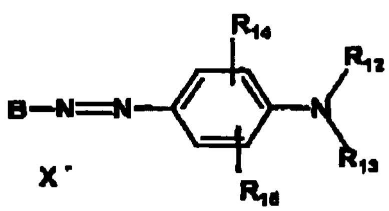

(V)

dans laquelle :

$R_{12}$ représente un atome d'hydrogène ou un radical alkyle en $C_1$-$C_4$,

$R_{13}$ représente un atome d'hydrogène, un radical alkyle pouvant être substitué par un radical -CN ou par un groupement amino, un radical 4'-aminophényle ou forme avec $R_{12}$ un hétérocycle éventuellement oxygéné et/ou azoté pouvant être substitué par un radical alkyle en $C_1$-$C_4$,

$R_{14}$ et $R_{15}$, identiques ou différents, représentent un atome d'hydrogène, un atome d'halogène, un radical alkyle en $C_1$-$C_4$ ou alcoxy en $C_1$-$C_4$, un radical -CN,

$X^-$ représente un anion ;

B représente un groupement choisi par les structures B1 à B6 suivantes :

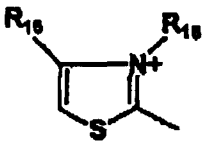

B1

;

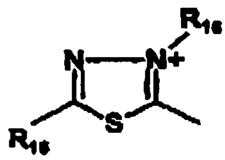

B2

;

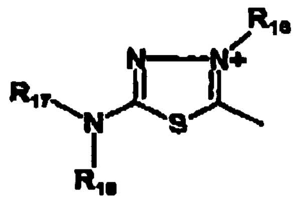

B3

;

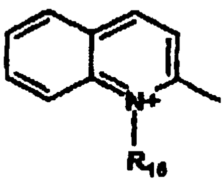

B4

;

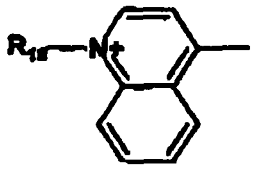

B5

et

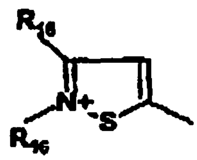

B6

;

dans lesquelles $R_{16}$ représente un radical alkyle en $C_1$-$C_4$, $R_{17}$ et $R_{18}$, identiques ou différents, représentent un atome d'hydrogène ou un radical alkyle en $C_1$-$C_4$ ;

c) les composés de formules (VI) et (VI') suivantes :

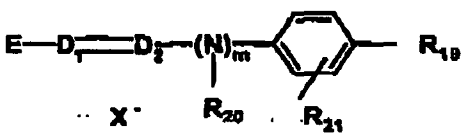

(VI)

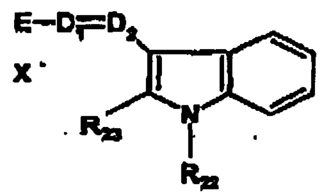

(VI')

dans lesquelles :

$R_{19}$ représente un atome d'hydrogène, un radical alcoxy en $C_1$-$C_4$, un atome d'halogène tel que le brome, le chlore, l'iode ou le fluor ou un radical amino,

$R_{20}$ représente un atome d'hydrogène, un radical alkyle en $C_1$-$C_4$ ou forme avec un atome de carbone du cycle benzénique un hétérocycle éventuellement oxygéné et/ou substitué par un ou plusieurs groupements alkyle en $C_1$-$C_4$,

$R_{21}$ représente un atome d'hydrogène ou d'halogène,

$R_{22}$ et $R_{23}$, identiques ou différents, représentent un atome d'hydrogène ou un radical alkyle en $C_1$-$C_4$,

$D_1$ et $D_2$, identiques ou différents, représentent un atome d'azote ou le groupement -CH,

m = 0 ou 1,

$X^-$ représente un anion ;

E représente un groupement choisi par les structures E1 à E8 suivantes :

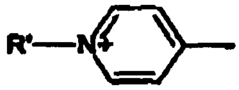

E1

;

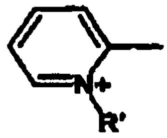

E2

;

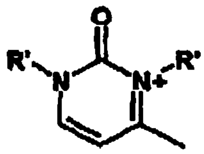

E3

;

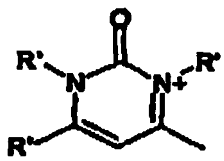

E4

;

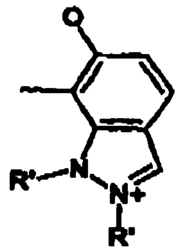

E5

;

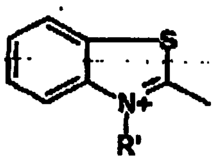

E6

;

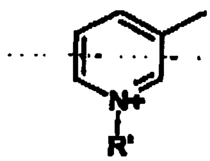

E7

et

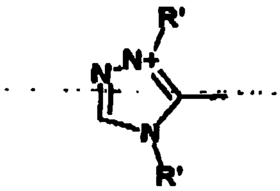

E8

dans lesquelles R' représente un radical alkyle en $C_1$-$C_4$ ;
lorsque m = 0 et que $D_{13}$ représente un atome d'azote, alors E peut également désigner un groupement de structure E9 suivante :

E9

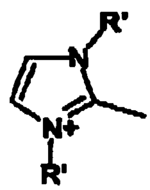

;

dans laquelle R' représente un radical alkyle en $C_1$-$C_4$.
d) les composés de formule (VII) suivante :

**G-N=N -J** (VII)

dans laquelle :

le symbole G représente un groupement choisi parmi les structures $G_1$ à $G_3$ suivantes

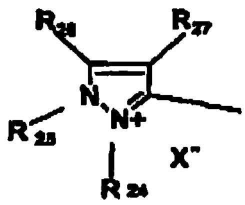

$G_1$

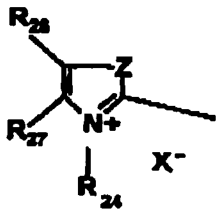

$G_2$

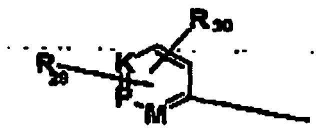

$G_3$

structures $G_1$ à $G_3$ dans lesquelles,

$R_{24}$ désigne un radical alkyle en $C_1$-$C_4$, un radical phényle pouvant être substitué par un radical alkyle en $C_1$-$C_4$ ou un atome d'halogène choisi parmi le chlore, le brome, l'iode et le fluor ;
$R_{25}$ désigne un radical alkyle en $C_1$-$C_4$ ou un radical phényle;
$R_{26}$ et $R_{27}$, identiques ou différents, représentent un radical alkyle en $C_1$-$C_4$, un radical phényle, ou forment ensemble dans $G_1$ un cycle benzénique substitué par un ou plusieurs radicaux alkyle en $C_1$-$C_4$ , alcoxy en $C_1$-$C_4$, ou $NO_2$, ou forment ensemble dans $G_2$ un cycle benzénique éventuellement substitué par un ou plusieurs radicaux alkyle en $C_1$-$C_4$ , alcoxy en $C_1$-$C_4$, ou $NO_2$;
$R_{26}$ peut désigner en outre un atome d'hydrogène;
Z désigne un atome d'oxygène, de soufre ou un groupement $-NR_{25}$;
M représente un groupement -CH, -CR (R désignant alkyle en $C_1$-$C_4$), ou $-NR_{28}(X^-)_r$;
K représente un groupement -CH, -CR (R désignant alkyle en $C_1$-$C_4$), ou $-NR_{28}(X^-)_r$;
P représente un groupement -CH, -CR (R désignant alkyle en $C_1$-$C_4$), ou $-NR_{28}(X^-)_r$; r désigne zéro ou 1;
$R_{28}$ représente un atome $O^-$, un radical alcoxy en $C_1$-$C_4$, ou un radical alkyle en $C_1$-$C_4$;
$R_{29}$ et $R_{30}$, identiques ou différents, représentent un atome d'hydrogène ou d'halogène, un radical alkyle en $C_1$-$C_4$, alcoxy en $C_1$-$C_4$, un radical $-NO_2$;
$X^-$ représente un anion;

le symbole J représente :

- (**a**) un groupement de structure $J_1$ suivante :

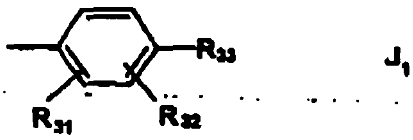

$J_1$

structure $J_1$ dans laquelle,
$R_{31}$ représente un atome d'hydrogène, un atome d'halogène, un radical alkyle en $C_1$-$C_4$, alcoxy en $C_1$-$C_4$, un radical -OH, $-NO_2$, $-NHR_{34}$, $-NR_{35}R_{36}$, -NHCOalkyle en $C_1$-$C_4$, ou forme avec $R_{32}$ un cycle à 5 ou 6 chaînons contenant ou non un ou plusieurs hétéroatomes choisis parmi l'azote, l'oxygène ou le soufre;
$R_{32}$ représente un atome d'hydrogène, un atome d'halogène, un radical alkyle en $C_1$-$C_4$, alcoxy en $C_1$-$C_4$

ou forme avec $R_{33}$ ou $R_{34}$ un cycle à 5 ou 6 chaînons contenant ou non un ou plusieurs hétéroatomes choisis parmi l'azote, l'oxygène ou le soufre;
$R_{33}$ représente un atome d'hydrogène, un radical -OH, un radical $-NHR_{34}$, un radical - $NR_{35}R_{36}$;
$R_{34}$ représente un atome d'hydrogène, un radical alkyle en $C_1$-$C_4$, un radical monohydroxyalkyle en $C_1$-$C_4$, polyhydroxyalkyle en $C_2$-$C_4$, un radical phényle;
$R_{35}$ et $R_{36}$, identiques ou différents, représentent un radical alkyle en $C_1$-$C_4$ , un radical monohydroxyalkyle en $C_1$-$C_4$, polyhydroxyalkyle en $C_2$-$C_4$;
- (**b**) un groupement hétérocyclique azoté à 5 ou 6 chaînons susceptible de renfermer d'autres hétéroatomes et/ou des groupements carbonylés et pouvant être substitué par un ou plusieurs radicaux alkyle en $C_1$-$C_4$, amino ou phényle,
et notamment un groupement de structure $J_2$ suivante :

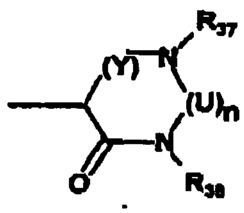

$J_2$

structure $J_2$ dans laquelle,
$R_{37}$ et $R_{38}$, identiques ou différents, représentent un atome d'hydrogène, un radical alkyle en $C_{13}$-$C_{10}$, un radical phényle;
Y désigne le radical -CO- ou le radical

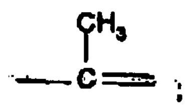

n = 0 ou 1, avec, lorsque n désigne 1, U désigne le radical -CO- , et les formes mésomères de ces structures (IV) à (VII).

**27.** Procédé de décoloration des fibres kératiniques humaines teintes par des colorants d'oxydation et/ou des colorants directs, en particulier des cheveux, **caractérisé par** le fait qu'on applique sur lesdites fibres, à une température d'application comprise entre la température ambiante et 80°C, une composition telle que définie dans l'une quelconque des revendications 1 à 20, pendant un temps suffisant pour dégrader partiellement ou totalement la teinture des cheveux.

**28.** Procédé selon la revendication 27, **caractérisé par** le fait que la température d'application est comprise entre 35°C et 50°C.

**29.** Procédé selon la revendication 27ou 28, **caractérisé par** le fait que le temps suffisant au développement de la décoloration est compris entre 1 et 60 minutes.

**30.** Procédé selon la revendication 29, **caractérisé par** le fait que le temps suffisant au développement de la décoloration est compris entre 5 et 30 minutes.

## Patentansprüche

**1.** Verwendung einer Zusammensetzung, die in einem kosmetisch unbedenklichen Medium bei einem pH-Wert von 1,5 bis 9 als Farbstoffreduktionsmittel mindestens ein Sulfinsäurederivat der folgenden Formel (I)

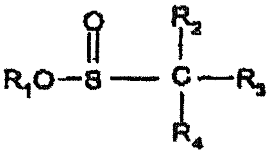

(I)

wobei
$R_1$ aus einem Wasserstoffatom, einem $NH_3^+$-Ion, einem einwertigen Metallion oder einem ionischen Äquivalent eines zweiwertigen Metalls aus den Gruppen Ia, IIa, IIb, IVa und VIIIb des Periodensystems ausgewählt ist,
$R_2$ aus einem OH-Rest oder einem $NR_5R_6$-Rest, worin $R_5$ und $R_6$ gleich oder verschieden sind und aus einem Wasserstoffatom oder einem $C_{1-6}$-Alkylrest ausgewählt sind, ausgewählt ist,
$R_3$ aus einem Wasserstoffatom oder einem Alkyl- oder Alkenyl- oder Cycloalkyl- oder Arylrest, der gegebenenfalls durch 1 bis 3 Substituenten, die gleich oder verschieden sind und unter OH-, $C_{1-6}$-Alkyl-, O-$C_{1-6}$-Alkyl-, Halogen- oder $CF_3$-Resten ausgewählt sind, substituiert ist, ausgewählt ist, $R_4$ aus einem $COOR_1$-, $SO_3R_1$-, $COR_5$-, $CONR_5R_6$- oder $COOR_5$-Rest ausgewählt ist, worin $R_1$, $R_5$ und $R_6$ die oben angegebenen Bedeutungen aufweisen,
und $R_4$ ferner auch für ein Wasserstoffatom steht, wenn $R_3$ für einen Arylrest und insbesondere eine substituierte Arylgruppe gemäß obiger Beschreibung steht,
und die kosmetisch unbedenklichen Salze dieser Verbindungen umfasst, zum Bleichen von menschlichen Keratinfasern, die mit Oxidationsfarbstoffen und/oder Direktfarbstoffen gefärbt sind.

2. Verwendung nach Anspruch 1, **dadurch gekennzeichnet, dass** in der Formel (I) $R_1$ aus einem $NH_3^+$-Ion, einem Alkalimetallion oder einem ionischen Äquivalent eines Erdalkalimetallions oder von Zink ausgewählt ist.

3. Verwendung nach Anspruch 1, **dadurch gekennzeichnet, dass** in der Formel (I) $R_2$ für einen OH-Rest oder einen $NH_2$-Rest steht.

4. Verwendung nach Anspruch 1, **dadurch gekennzeichnet, dass** in der Formel (I) $R_3$ aus einem Wasserstoffatom oder einem Alkylrest, der gegebenenfalls durch einen oder zwei OH-Reste oder einen oder zwei $C_{1-6}$-Alkyl- oder O-$C_{1-6}$-Alkylreste substituiert ist, ausgewählt ist.

5. Verwendung nach Anspruch 1, **dadurch gekennzeichnet, dass** in der Formel (I) $R_4$ für einen $COOR_1$-oder $COOR_5$-Rest steht, worin $R_1$ und $R_5$ die in Anspruch 1 angegebenen Bedeutungen aufweisen.

6. Verwendung nach Anspruch 1, **dadurch gekennzeichnet, dass** in der Formel (I) $R_1$ für Na steht, $R_2$ für OH steht, $R_3$ für H steht und $R_4$ für COONa steht, d.h. die Verbindung der folgenden Formel (II):

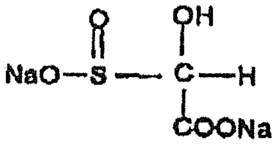

(II)

7. Verwendung nach Anspruch 1, **dadurch gekennzeichnet, dass** die kosmetische Zusammensetzung 40 bis 60 Gew.-% einer Verbindung der Formel (II), 10 bis 20 Gew.-% $NaSO_3$-CHOH-COONa und 30 bis 40% $Na_2SO_3$ umfasst.

8. Verwendung nach einem der Ansprüche 1 bis 7, **dadurch gekennzeichnet, dass** die kosmetische Zusammensetzung einen pH-Wert zwischen 1,8 und 6 aufweist.

9. Verwendung nach einem der Ansprüche 1 bis 8, **dadurch gekennzeichnet, dass** die Verbindung(en) der Formel (I) 0,01 bis 20 Gew.-% des Gesamtgewichts der Zusammensetzung ausmachen.

10. Verwendung nach Anspruch 9, **dadurch gekennzeichnet, dass** sie 0,1 bis 10 Gew.-% des Gesamtgewichts der Zusammensetzung ausmachen.

11. Verwendung nach einem der Ansprüche 1 bis 10, **dadurch gekennzeichnet, dass** die kosmetische Zusammensetzung mindestens ein Verdickungsmittel enthält.

**12.** Verwendung nach Anspruch 11, **dadurch gekennzeichnet, dass** es sich bei dem Verdickungsmittel um ein Cellulosederivat, ein Guarderivat, ein Gummi mikrobieller oder pflanzlicher Herkunft oder ein synthetisches Verdickungsmittel handelt.

**13.** Verwendung nach Anspruch 11 oder 12, **dadurch gekennzeichnet, dass** das bzw. die Verdickungsmittel 0,01 bis 10 Gew.-% des Gesamtgewichts der Zusammensetzung ausmachen.

**14.** Verwendung nach einem der Ansprüche 1 bis 13, **dadurch gekennzeichnet, dass** die kosmetische Zusammensetzung außerdem mindestens ein Alkalinisierungs- oder Ansäuerungsmittel in Mengen im Bereich von 0,01 bis 30 Gew.-% des Gesamtgewichts der Zusammensetzung enthält.

**15.** Verwendung nach Anspruch 14, **dadurch gekennzeichnet, dass** das Ansäuerungsmittel aus anorganischen oder organischen Säuren wie Etidronsäure, Salzsäure, Orthophosphorsäure, Carbonsäuren wie Weinsäure, Citronensäure oder Milchsäure oder Sulfonsäuren ausgewählt ist.

**16.** Verwendung nach Anspruch 14, **dadurch gekennzeichnet, dass** das Alkalisierungsmittel aus Ammoniak, alkalischen Carbonaten, Alkanolaminen wie Mono-Di- und Triethanolamin, 2-Methyl-2-amino-1-propanol sowie deren Derivaten, Natriumhydroxid oder Kaliumhydroxid und den Verbindungen der folgenden Formel (III) ausgewählt ist:

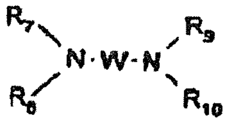

(III)

wobei W für eine gegebenenfalls durch eine Hydroxygruppe oder eine $C_{1-4}$-Alkylgruppe substituierte Propylengruppe steht und $R_7$, $R_8$, $R_9$ und $R_{10}$ gleich oder voneinander verschieden sind und für ein Wasserstoffatom oder einen $C_{1-4}$-Alkyl- oder $C_{1-4}$-Hydroxyalkylrest stehen.

**17.** Verwendung nach einem der Ansprüche 1 bis 16, **dadurch gekennzeichnet, dass** die kosmetische Zusammensetzung durch bedarfsgemäßes Mischen einer wasserfreien Zusammensetzung, die mindestens ein Sulfinsäurederivat der Formel (I) enthält, und einer wässrigen Zusammensetzung bei einem pH-Wert zwischen 1,5 und 9 zum Zeitpunkt der Anwendung hergestellt wird.

**18.** Verwendung nach Anspruch 17, **dadurch gekennzeichnet, dass** die kosmetische Zusammensetzung durch bedarfsgemäßes Mischen einer wasserfreien Zusammensetzung, die mindestens ein Sulfinsäurederivat der Formel (I) enthält, und einer wässrigen Zusammensetzung bei einem pH-Wert zwischen 1,8 und 6 zum Zeitpunkt der Anwendung hergestellt wird.

**19.** Verwendung nach Anspruch 16, **dadurch gekennzeichnet, dass** die kosmetische Zusammensetzung durch bedarfsgemäßes Mischen von zwei wässrigen Zusammensetzungen zum Zeitpunkt der Anwendung hergestellt wird, wobei die eine Zusammensetzung mindestens ein Sulfinsäurederivat der Formel (I) bei saurem oder alkalischem pH-Wert und die andere Zusammensetzung eine wässrige Zusammensetzung bei einem sauren pH-Wert enthält.

**20.** Verwendung nach einem der Ansprüche 1 bis 19, **dadurch gekennzeichnet, dass** die kosmetische Zusammensetzung in Form eines Gels, einer Creme, eines Schaums, einer Lösung oder einer Emulsion vorliegt.

**21.** Vorrichtung mit mehreren Kompartimenten, die (i) zum Färben und dann (ii) zum Bleichen von menschlichen Keratinfasern, die mit Oxidationsfarbstoffen und/oder Direktfarbstoffen gefärbt sind, insbesondere von Haaren, vorgesehen ist, **dadurch gekennzeichnet, dass** sie ein erstes Kompartiment, das eine Zusammensetzung zum oxidativen Färben oder nicht oxidativen Färben der Fasern umfasst, und in einem zweiten Kompartiment eine Zusammensetzung zum reduzierenden Bleichen der gefärbten Fasern gemäß einem der Ansprüche 1 bis 20 umfasst.

**22.** Vorrichtung nach Anspruch 21, **dadurch gekennzeichnet, dass** im ersten Kompartiment die Zusammensetzung zum oxidativen Färben Oxidationsbasen und/oder Kuppler, die durch Mischen mit einem Oxidationsmittel eine oxidative Färbung bilden, enthält.

**23.** Vorrichtung nach Anspruch 22, **dadurch gekennzeichnet, dass** im ersten Kompartiment die Zusammensetzung zum oxidativen Färben außerdem Direktfarbstoffe enthält.

**24.** Vorrichtung nach Anspruch 21, **dadurch gekennzeichnet, dass** im ersten Kompartiment die Zusammensetzung zum oxidativen Färben Direktfarbstoffe, vorzugsweise kationische Direktfarbstoffe, die durch Mischen mit einem Oxidationsmittel eine aufhellende Direktfärbung bilden, enthält.

**25.** Vorrichtung nach Anspruch 21, **dadurch gekennzeichnet, dass** im ersten Kompartiment die Zusammensetzung zum nicht oxidativen Färben Direktfarbstoffe und vorzugsweise kationische Direktfarbstoffe enthält.

**26.** Vorrichtung nach Anspruch 24 oder 25, **dadurch gekennzeichnet, dass** die kationischen Direktfarbstoffe aus den Farbstoffen der folgenden Strukturen (I) bis (VII) und deren mesomeren Formen ausgewählt sind:

(I) Farbstoffen der Formeln (I), (II)a, (II)b, (III)a, (III)b:

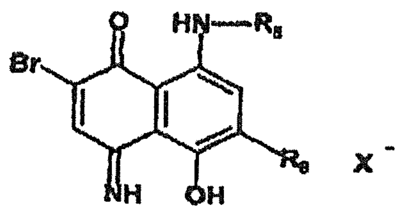

(I)

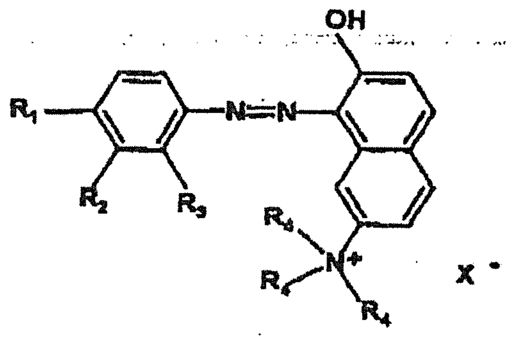

(II)a

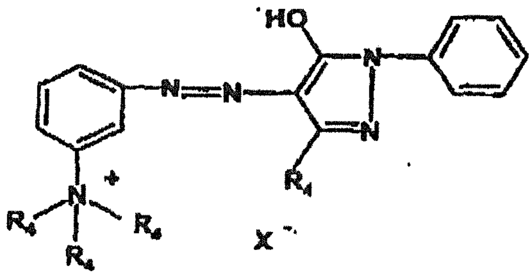

(II)b

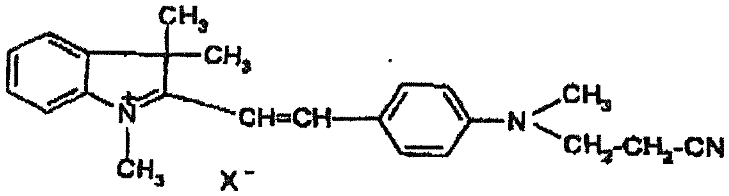

(III)a

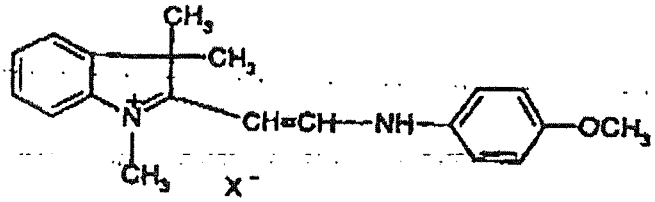

(III)b

wobei in den Formeln (I), (II)a, (II)b, (III)a, (III)b,

$R_1$ für ein Wasserstoffatom oder einen Aminorest steht;
$R_2$ für ein Wasserstoffatom oder eine Nitrogruppe steht;
$R_3$ für ein Wasserstoffatom, eine Nitrogruppe oder einen $C_1$-$C_4$-Alkoxyrest steht;
$R_4$ für einen $C_1$-$C_4$-Alkylrest steht;
$R_5$ für ein Wasserstoffatom oder eine para-Tri-$C_{1-4}$-alkylammoniophenylgruppe steht;
$R_6$ für ein Bromatom oder eine NH-para-Tri-$C_{1-4}$-alkylammoniophenylgruppe steht;
$X^-$ für ein Anion steht;

(II) Farbstoffen der Formeln (IV), (V), (VI), (VI'), (VII):

a) den Verbindungen der folgenden Formel (IV):

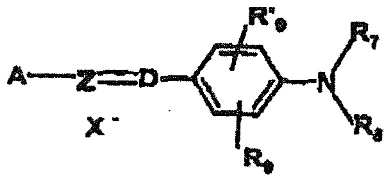

(IV)

wobei:

Z und D gleich oder verschieden sind und für ein Stickstoffatom oder einen -CH-Rest stehen;
$R_7$ und $R_8$ gleich oder verschieden sind und für ein Wasserstoffatom oder einen $C_{1-4}$-Alkylrest, der durch einen -CN-, -OH- oder -$NH_2$-Rest substituiert sein kann, stehen oder mit einem Kohlenstoffatom des Benzolrings einen gegebenenfalls sauerstoff- oder stickstoffhaltigen Heterocyclus, der durch einen oder mehrere $C_{1-4}$-Alkylreste substituiert sein kann, bilden; oder einen 4'-Aminophenylrest stehen,
$R_9$ und $R'_9$ gleich oder verschieden sind und für ein Wasserstoff- oder Halogenatom oder einen Cyano-, $C_{1-4}$-Alkyl-, $C_{1-4}$-Alkoxy- oder Acetyloxyrest stehen;
$X^-$ für ein Anion steht;
A für eine Gruppe steht, die aus den folgenden Strukturen A1 bis A19 ausgewählt ist:

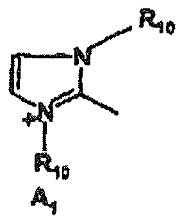

$A_1$ ;

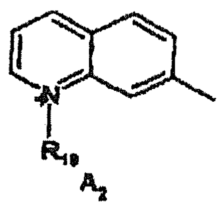

$A_2$ ;

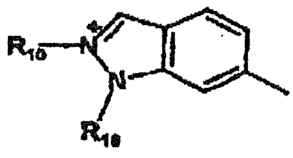

$A_3$

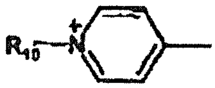

$A_4$

;

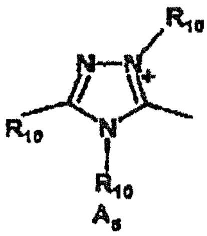

$A_5$

;

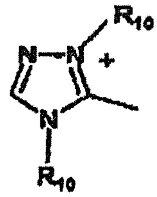

$A_6$

;

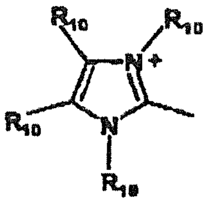

$A_7$

;

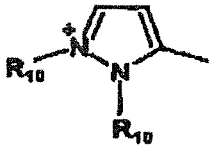

$A_8$

;

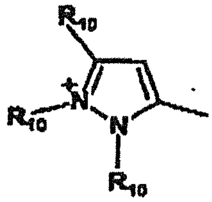

$A_9$

;

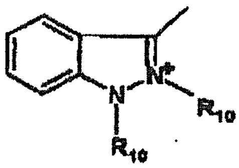

$A_{10}$

;

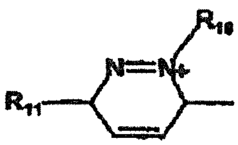

$A_{11}$

;

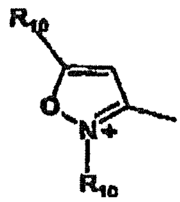

$A_{12}$

;

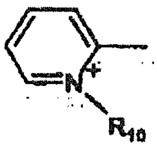

$A_{13}$

;

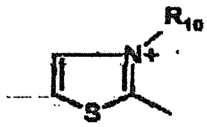

$A_{14}$

;

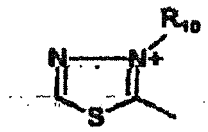

$A_{15}$

;

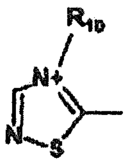

$A_{16}$

;

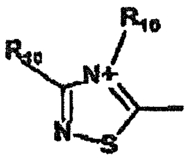

$A_{17}$

;

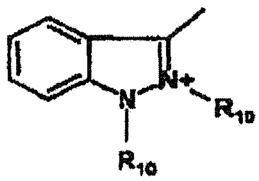

$A_{18}$

und

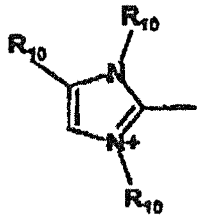

A10

wobei $R_{10}$ für einen $C_{1-4}$-Alkylrest, der durch einen Hydroxylrest substituiert sein kann, steht und $R_{11}$ für einen $C_{1-4}$-Alkoxyrest steht;

b) den Verbindungen der folgenden Formel (V):

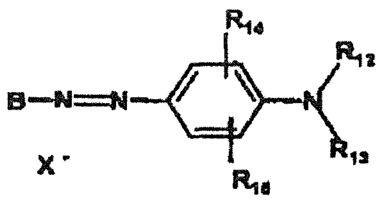

(V)

wobei:

$R_{12}$ für ein Wasserstoffatom oder einen $C_{1-4}$-Alkylrest steht,

$R_{13}$ für ein Wasserstoffatom, einen Alkylrest, der durch einen -CN-Rest oder eine Aminogruppe substituiert sein kann, oder einen 4'-Aminophenylrest steht oder mit $R_{12}$ einen gegebenenfalls sauerstoff- oder stickstoffhaltigen Heterocyclus, der durch einen $C_{1-4}$-Alkylrest substituiert sein kann, bildet,

$R_{14}$ und $R_{15}$ gleich oder verschieden sind und für ein Wasserstoffatom, ein Halogenatom, einen $C_{1-4}$-Alkyl- oder $C_{1-4}$-Alkoxyrest oder einen -CN-Rest stehen,

$X^-$ für ein Anion steht;

B für eine Gruppe steht, die aus den folgenden Strukturen B1 bis B6 ausgewählt ist:

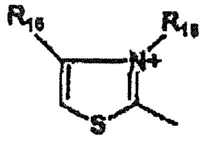

B1

;

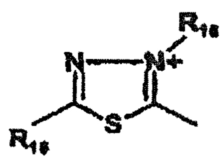

B2

;

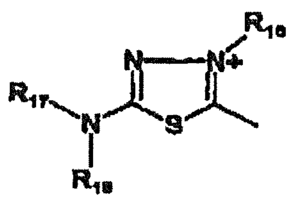

B3

;

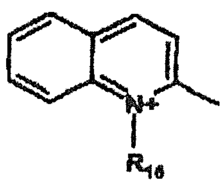

B4

;

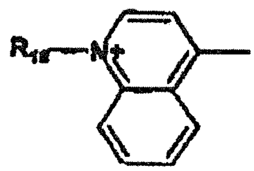

B5

und

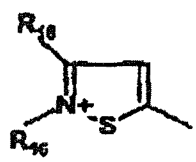

B6

;

wobei $R_{16}$ für einen $C_{1-4}$-Alkylrest steht und $R_{17}$ und $R_{18}$ gleich oder verschieden sind und für ein Wasserstoffatom oder einen $C_{1-4}$-Alkylrest stehen;

c) den Verbindungen der folgenden Formeln (VI) und (VI') :

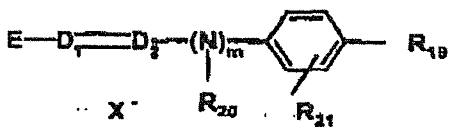

(VI)

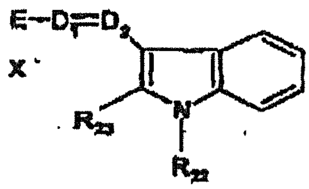

(VI')

wobei:

$R_{19}$ für ein Wasserstoffatom, einen $C_{1-4}$-Alkoxyrest, ein Halogenatom wie Brom, Chlor, Iod oder Fluor oder einen Aminorest steht,
$R_{20}$ für ein Wasserstoffatom oder einen $C_{1-4}$-Alkylrest steht oder mit einem Kohlenstoffatom des Benzolrings einen gegebenenfalls sauerstoff- oder stickstoffhaltigen Heterocyclus, der durch eine oder mehrere $C_{1-4}$-Alkylgruppen substituiert sein kann, bildet,
$R_{21}$ für ein Wasserstoff- oder Halogenatom steht,
$R_{22}$ und $R_{23}$ gleich oder verschieden sind und für ein Wasserstoffatom oder einen $C_{1-4}$-Alkylrest stehen,
$D_1$ und $D_2$ gleich oder verschieden sind und für ein Stickstoffatom oder eine -CH-Gruppe stehen,
m = 0 oder 1,
$X^-$ für ein Anion steht;
E für eine Gruppe steht, die aus den folgenden Strukturen E1 bis E8 ausgewählt ist:

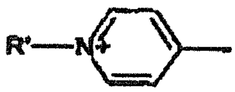

E1

;

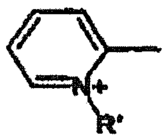

E2

;

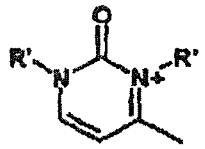

E3

;

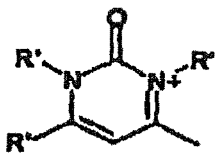

E4

;

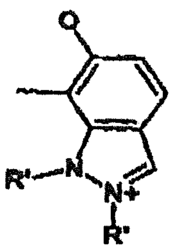

E5

;

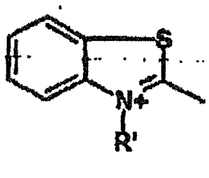

E6

;

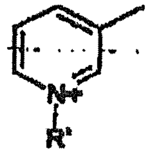

E7

und

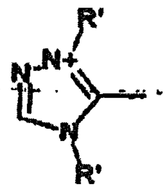

E8

wobei R' für einen $C_{1-4}$-Alkylrest steht;
dann, wenn m = 0 und $D_{13}$ für ein Stickstoffatom steht, die Gruppe E auch eine Gruppe der folgenden Struktur E9 sein kann:

E9 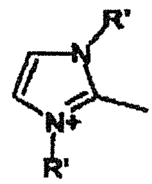

wobei R' für einen $C_{1-4}$-Alkylrest steht;
d) den Verbindungen der folgenden Formel (VII):

G-N=N-J (VII)

wobei:

das Symbol G für eine Gruppe steht, die aus den folgenden Strukturen $G_1$ bis $G_3$ ausgewählt ist:

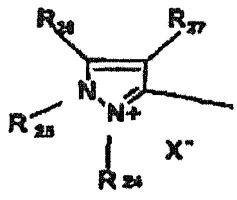

$G_1$

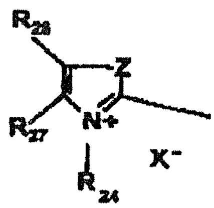

$G_2$

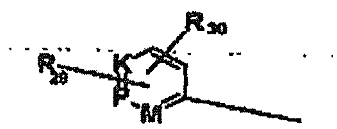

$G_3$

wobei in den Strukturen $G_1$ bis $G_3$
$R_{24}$ für einen $C_{1-4}$-Alkylrest oder einen Phenylrest, der durch einen $C_{1-4}$-Alkylrest oder ein aus Chlor, Brom, Iod und Fluor ausgewähltes Halogenatom substituiert sein kann, steht;
$R_{25}$ für einen $C_{1-4}$-Alkylrest oder einen Phenylrest steht;
$R_{26}$ und $R_{27}$ gleich oder verschieden sind und für einen $C_{1-4}$-Alkylrest oder einen Phenylrest stehen oder zusammen in $G_1$ einen Benzolring, der durch einen oder mehrere $C_{1-4}$-Alkyl-, $C_{1-4}$-Alkoxy- oder $NO_2$-Reste substituiert ist, bilden oder zusammen mit $G_2$ einen Benzolring, der gegebenenfalls der durch einen oder mehrere $C_{1-4}$-Alkyl-, $C_{1-4}$-Alkoxy- oder $NO_2$-Reste substituiert ist, bilden;
$R_{26}$ außerdem auch für ein Wasserstoffatom stehen kann,
Z für ein Sauerstoffatom, ein Schwefelatom oder eine $-NR_{25}$-Gruppe steht ;
M für eine -CH-, -CR- (wobei R für eine $C_{1-4}$-Alkylgruppe steht) oder $-NR_{28}(X-)_r$-Gruppe steht;
K für eine -CH-, -CR- (wobei R für eine $C_{1-4}$-Alkylgruppe steht) oder $-NR_{28}(X-)_r$-Gruppe steht;
P für eine -CH-, -CR- (wobei R für eine $C_{1-4}$-Alkylgruppe steht) oder $-NR_{28}(X-)_r$-Gruppe steht;
r für Null oder 1 steht;
$R_{28}$ für ein $O^-$Atom, einen $C_{1-4}$-Alkoxyrest oder einen $C_{1-4}$-Alkylrest steht;
$R_{29}$ und $R_{30}$ gleich oder verschieden sind und für ein Wasserstoff- oder Halogenatom, einen $C_{1-4}$-Alkyl- oder $C_{1-4}$-Alkoxyrest oder einen $-NO_2$-Rest stehen;
$X^-$ für ein Anion steht;
das Symbol J für:

- (a) eine Gruppe der folgenden Struktur $J_1$:

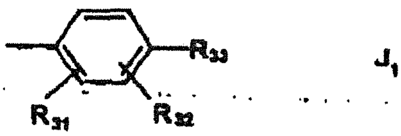

$J_1$

wobei in der Struktur $J_1$

$R_{31}$ für ein Wasserstoffatom, ein Halogenatom, einen $C_{1-4}$-Alkyl- oder $C_{1-4}$-Alkoxyrest oder einen -OH-, -$NO_2$-, -$NHR_{34}$-, -$NR_{35}R_{36}$- oder -NHCO-$C_{1-4}$-Alkylrest steht oder mit $R_{32}$ einen 5- oder 6-gliedrigen Ring, der gegebenenfalls ein oder mehrere aus Stickstoff, Sauerstoff oder Schwefel ausgewählte Heteroatome enthält, bildet;

$R_{32}$ für ein Wasserstoffatom, ein Halogenatom oder einen $C_{1-4}$-Alkyl- oder $C_{1-4}$-Alkoxyrest steht oder mit $R_{33}$ oder $R_{34}$ einen 5- oder 6-gliedrigen Ring, der gegebenenfalls ein oder mehrere aus Stickstoff, Sauerstoff oder Schwefel ausgewählte Heteroatome enthält, bildet;

$R_{33}$ für Wasserstoffatom, einen -OH-Rest, einen -$NHR_{34}$-Rest oder einen -$NR_{35}R_{36}$-Rest steht;

$R_{34}$ für ein Wasserstoffatom, einen $C_{1-4}$-Alkylrest, einen $C_{1-4}$-Monohydroxyalkyl- oder $C_{2-4}$-Polyhydroxyalkylrest oder einen Phenylrest steht;

$R_{35}$ und $R_{36}$ gleich oder verschieden sind und für einen $C_{1-4}$-Alkylrest oder einen $C_{1-4}$-Monohydroxyalkyl- oder $C_{2-4}$-Polyhydroxyalkylrest stehen;

- (b) eine 5- bis 6-gliedrige stickstoffhaltige heterocyclische Gruppe, die weitere Heteroatome und/oder Carbonylgruppe enthalten kann und durch einen oder mehrere $C_{1-4}$-Alkyl-, Amino- oder Phenylreste substituiert sein kann,

und insbesondere eine Gruppe der folgenden Struktur $J_2$ steht:

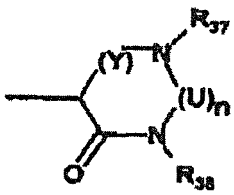

$J_2$

wobei in der Struktur $J_2$

$R_{37}$ und $R_{38}$ gleich oder verschieden sind und für ein Wasserstoffatom, einen $C_{13}$-$C_{10}$-Alkylrest oder einen Phenylrest stehen;

Y für einen -CO-Rest oder den Rest

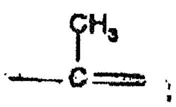

steht;

n = 0 oder 1, wobei dann, wenn n für 1 steht, U für einen -CO-Rest steht, und den mesomeren Formen dieser Strukturen (IV) bis (VII).

**27.** Verfahren zum Bleichen von menschlichen Keratinfasern, die mit Oxidationsfarbstoffen und/oder Direktfarbstoffen gefärbt sind, insbesondere von Haaren, **dadurch gekennzeichnet, dass** man auf die Fasern eine Zusammensetzung gemäß einem der Ansprüche 1 bis 20 bei einer Anwendungstemperatur zwischen Umgebungstemperatur und 80°C über einen Zeitraum, der zur teilweisen oder vollständigen Abtönung der Haarfärbung ausreicht, aufbringt.

**28.** Verfahren nach Anspruch 27, **dadurch gekennzeichnet, dass** die Anwendungstemperatur zwischen 35°C und 50°C liegt.

**29.** Verfahren nach Anspruch 27 oder 28, **dadurch gekennzeichnet, dass** die zur Entwicklung der Bleichung ausreichende Zeit zwischen 1 und 60 Minuten liegt.

**30.** Verfahren nach Anspruch 29, **dadurch gekennzeichnet, dass** die zur Entwicklung der Bleichung ausreichende Zeit zwischen 5 und 30 Minuten liegt.

## Claims

**1.** Use for bleaching human keratin fibres dyed with oxidation dyes and/or direct dyes of a cosmetic composition comprising, in a cosmetically acceptable medium at a pH of between 1.5 and 9, as dye-reducing agent, at least one sulfinic acid derivative of formula (I) below:

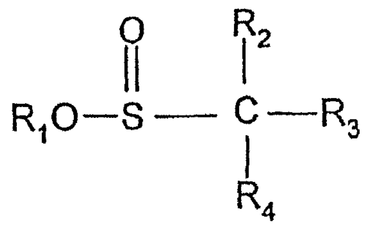

(I)

in which:

$R_1$ is chosen from a hydrogen atom, an $NH_3^+$ ion, a monovalent metal ion or an ionic equivalent of a divalent metal from groups Ia, IIa, IIb, IVa and VIIIb of the Periodic Table of Elements,
$R_2$ is chosen from an OH radical, a radical $NR_5R_6$ in which $R_5$ and $R_6$, which may be identical or different, are chosen from a hydrogen atom or a $C_1$-$C_6$ alkyl radical,
$R_3$ is chosen from a hydrogen atom, an alkyl or alkenyl or cycloalkyl or aryl radical which is unsubstituted or substituted by 1 to 3 identical or different susbtituents chosen from OH, $C_1$-$C_6$ alkyl, O-$C_1$-$C_6$ alkyl, halogen or $CF_3$ radicals,
$R_4$ is chosen from a radical $COOR_1$, $SO_3R_1$, $COR_5$, $CONR_5R_6$ or $COOR_5$ in which $R_1$, $R_5$ and $R_6$ have the meanings above,
and $R_4$ also denotes a hydrogen atom when $R_3$ denotes an aryl radical and in particular an aryl radical substituted as described above,
and the salts of said compounds which are cosmetically acceptable.

**2.** Use according to Claim 1, **characterized in that**, in the formula (I), $R_1$ is chosen from an $NH_3^+$ ion, an alkali metal ion and an ionic equivalent of an alkaline-earth metal or of zinc.

**3.** Use according to Claim 1, **characterized in that**, in the formula (I), $R_2$ is an OH radical or an $NH_2$ radical.

**4.** Use according to Claim 1, **characterized in that**, in the formula (I), $R_3$ is chosen from a hydrogen atom, an alkyl radical which is unsubstituted or substituted by one or two OH radicals or one or two $C_1$-$C_6$ alkyl or O-$C_1$-$C_6$ alkyl radicals.

**5.** Use according to Claim 1, **characterized in that**, in the formula (I), $R_4$ is a radical $COOR_1$ or $COOR_5$ in which $R_1$ and $R_5$ have the meanings from Claim 1.

**6.** Use according to Claim 1, **characterized in that**, in the formula (I), $R_1$ is Na, $R_2$ is OH, $R_3$ is H and $R_4$ is COONa, in other words the compound of the formula (II) below:

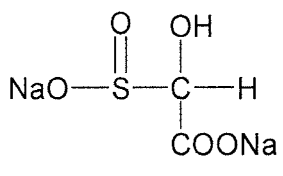

(II)

**7.** Use according to Claim 1, **characterized in that** the cosmetic composition contains 40% to 60% by weight of a compound of formula (II), 10% to 20% by weight of $NaSO_3$-CHOH-COONa and from 30% to 40% by weight of $Na_2SO_3$.

**8.** Use according to any one of Claims 1 to 7, **characterized in that** the cosmetic composition has a pH of between 1.8 and 6.

**9.** Use according to any one of Claims 1 to 8, **characterized in that** the compound(s) of formula (I) represent(s) 0.01% to 20% by weight relative to the total weight of the composition.

**10.** Use according to Claim 9, **characterized in that** they represent 0.1% to 10% by weight relative to the total weight of the composition.

**11.** Use according to any one of Claims 1 to 10, **characterized in that** the cosmetic composition contains at least one thickener.

**12.** Use according to Claim 11, **characterized in that** the thickener is a cellulose derivative, a guar derivative, a gum of microbial or plant origin, or a synthetic thickener.

**13.** Use according to Claim 11 or 12, **characterized in that** the thickener(s) represent(s) 0.01% to 10% by weight relative to the total weight of the composition.

**14.** Use according to any one of Claims 1 to 13, **characterized in that** the cosmetic composition also contains at least one acidifying or basifying agent in amounts ranging from 0.01% to 30% by weight relative to the total weight of the composition.

**15.** Use according to Claim 14, **characterized in that** the acidifying agent is chosen from mineral and organic acids, for instance etidronic acid, hydrochloric acid, orthophosphoric acid, carboxylic acids, for instance tartaric acid, citric acid or lactic acid, and sulfonic acids.

**16.** Use according to Claim 14, **characterized in that** the basifying agent is chosen from aqueous ammonia, alkaline carbonates, alkanolamines, such as monoethanolamine, diethanolamine and triethanolamine, 2-methyl-2-amino-1-propanol and derivatives thereof, sodium hydroxide, potassium hydroxide and the compounds of formula (III) below:

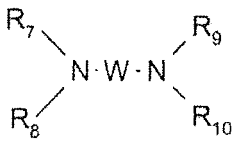

(III)

in which W represents a propylene residue optionally substituted with a hydroxyl group or a $C_1$-$C_4$ alkyl radical; $R_7$, $R_8$, $R_9$ and $R_{10}$, which may be identical or different, represent a hydrogen atom or a $C_1$-$C_4$ alkyl or $C_1$-$C_4$ hydroxyalkyl radical.

**17.** Use according to any one of Claims 1 to 16, **characterized in that** the cosmetic composition is obtained by extemporaneous mixing, at the time of use, of an anhydrous composition containing at least one sulfinic acid derivative of formula (I) as defined in any one of Claims 1 to 7 and of an aqueous composition at a pH of between 1.5 and 9.

**18.** Use according to Claim 17, **characterized in that** the cosmetic composition is obtained by extemporaneous mixing, at the time of use, of an anhydrous composition containing at least one sulfinic acid derivative of formula (I) as defined in any one of Claims 1 to 7 and of an aqueous composition at a pH of between 1.8 and 6.

**19.** Use according to Claim 16, **characterized in that** the cosmetic composition is obtained by extemporaneous mixing, at the time of use, of two aqueous compositions, one of which contains at least one sulfinic acid derivative of formula (I), at acidic or alkaline pH, and the other contains an aqueous composition at acidic pH.

**20.** Use according to any one of Claims 1 to 19, **characterized in that** the cosmetic composition is in the form of a gel, a cream, a mousse, a solution or an emulsion.

**21.** Multi-compartment device intended (i) for dyeing and then (ii) for bleaching human keratin fibres dyed with oxidation dyes and/or direct dyes, in particular the hair, **characterized in that** it comprises a first compartment comprising a composition for the oxidizing dyeing or non-oxidizing dyeing of said fibres, and, in a second compartment, a composition for the reductive bleaching of said dyed fibres and as defined in any one of Claims 1 to 20.

22. Device according to Claim 21, **characterized in that**, in the first compartment, the oxidation dye composition contains oxidation bases and/or couplers that form an oxidation dye by mixing with an oxidizing agent.

23. Device according to Claim 22, **characterized in that**, in the first compartment, the oxidation dye composition also contains direct dyes.

24. Device according to Claim 21, **characterized in that**, in the first compartment, the oxidation dye composition contains direct dyes that are preferably cationic direct dyes, which form a lightening direct dye by mixing with an oxidizing agent.

25. Device according to Claim 21, **characterized in that**, in the first compartment, the composition for non-oxidation dyeing contains direct dyes and preferably cationic direct dyes.

26. Device according to Claim 24 or 25, **characterized in that** the cationic direct dyes are chosen from the dyes of structures (I) to (VII) below, and the mesomeric forms thereof:

(i) dyes of formulae (I), (II)a, (II)b, (III)a and (III)b:

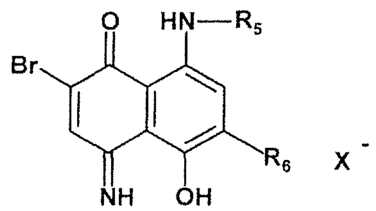

(I)

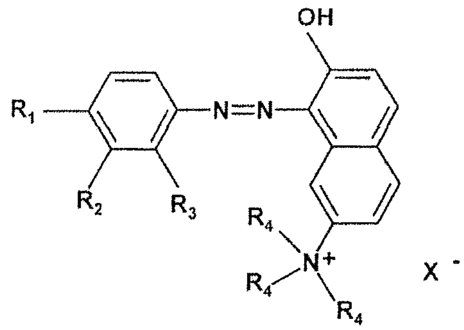

(II)a

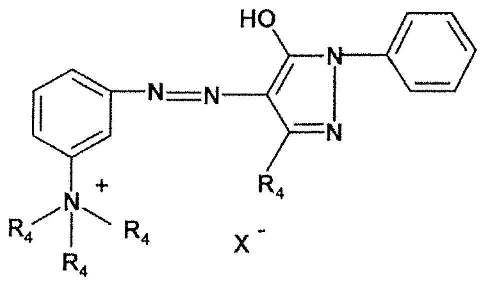

(II)b

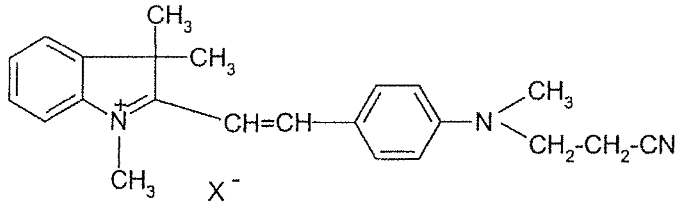

(III)a

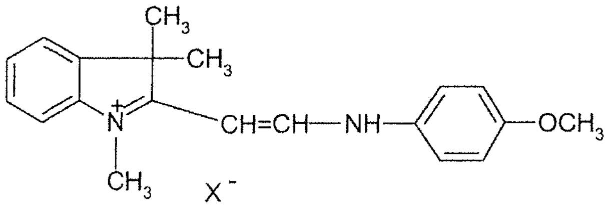

(III)b

in which formulae (I), (II)a, (II)b, (III)a and (III)b:

$R_1$ represents a hydrogen atom or an amino radical;
$R_2$ represents a hydrogen atom or a nitro group;
$R_3$ represents a hydrogen atom, a nitro group or a $C_1$-$C_4$ alkoxy radical;
$R_4$ represents a $C_1$-$C_4$ alkyl radical;
$R_5$ represents a hydrogen atom or a para-tri($C_1$-$C_4$) - alkylammoniophenyl group;
$R_6$ represents a bromine atom or an NH-para-tri($C_1$-$C_4$)alkylammoniophenyl group;
$X^-$ represents an anion;

(ii) dyes of formulae (IV), (V), (VI), (VI') and (VII) :

a) the compounds of formula (IV) below:

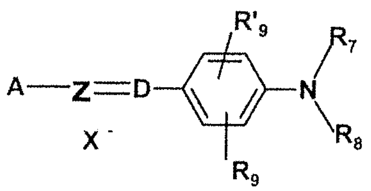

(IV)

in which:

Z and D, which may be identical or different, represent a nitrogen atom or a -CH- radical,
$R_7$ and $R_8$, which may be identical or different, represent a hydrogen atom; a $C_1$-$C_4$ alkyl radical which may be substituted with a -CN, -OH or $-NH_2$ radical, or form, with a carbon atom of the benzene ring, a heterocycle optionally containing oxygen or nitrogen, which may be substituted with one or more $C_1$-$C_4$ alkyl radicals; a 4'-aminophenyl radical,
$R_9$ and $R'_9$, which may be identical or different, represent a hydrogen atom or a halogen atom, or a cyano, $C_1$-$C_4$ alkyl, $C_1$-$C_4$ alkoxy or acetyloxy radical,
$X^-$ represents an anion;
A represents a group chosen from structures A1 to A19 below:

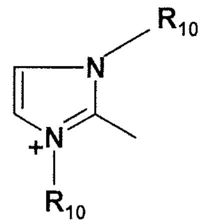

A1

;

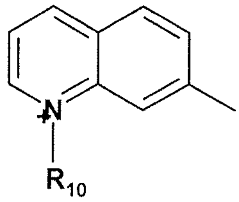

A2

;

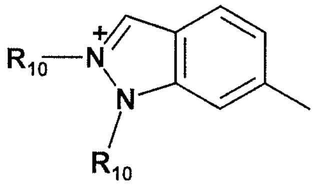

A3

;

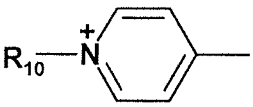

A4

;

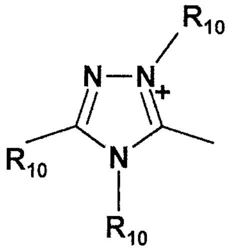

A5

;

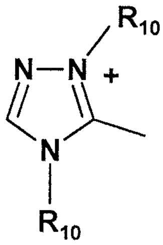

A6

;

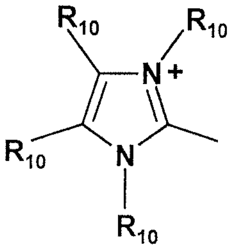

A7

;

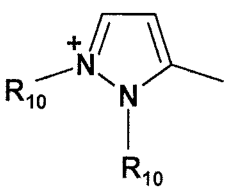

A8

;

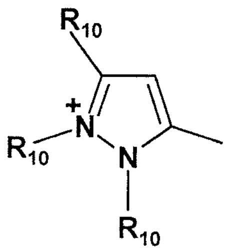

A9

;

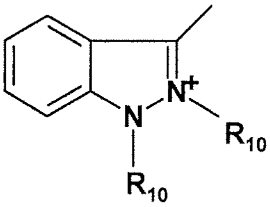

A10

;

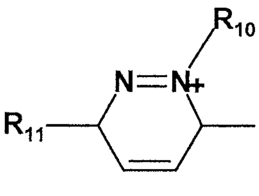

A11

;

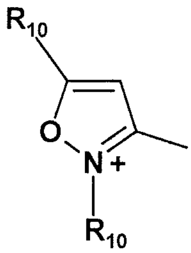

A12

;

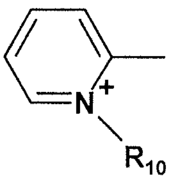

A13

;

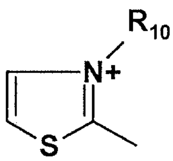

A14

;

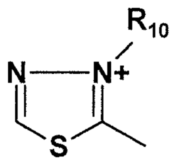

A15

;

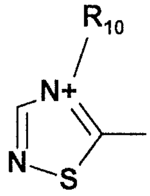

A16

;

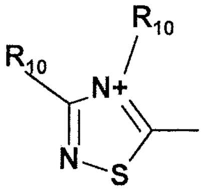

A17

;

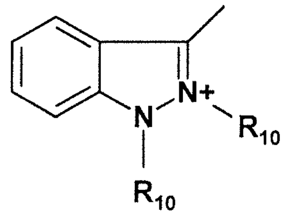

A18

and

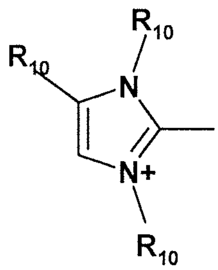

A19

in which $R_{10}$ represents a $C_1$-$C_4$ alkyl radical which may be substituted with a hydroxyl radical and $R_{11}$ represents a $C_1$-$C_4$ alkoxy radical;

b) the compounds of formula (V) below:

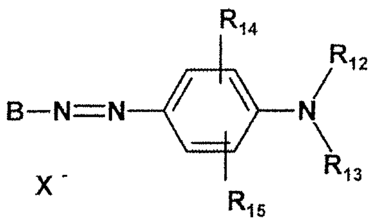

(V)

in which:

$R_{12}$ represents a hydrogen atom or a $C_1$-$C_4$ alkyl radical,
$R_{13}$ represents a hydrogen atom, an alkyl radical which may be substituted with a -CN radical or with an amino group, a 4'-aminophenyl radical, or forms with $R_{12}$ a heterocycle optionally containing oxygen and/or nitrogen, which may be substituted with a $C_1$-$C_4$ alkyl radical,
$R_{14}$ and $R_{15}$, which may be identical or different, represent a hydrogen atom, a halogen atom, a $C_1$-$C_4$ alkyl or $C_1$-$C_4$ alkoxy radical, or a -CN radical,
$X^-$ represents an anion;
B represents a group chosen from structures B1 to B6 below:

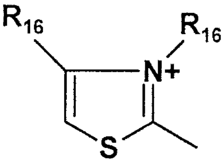 ; 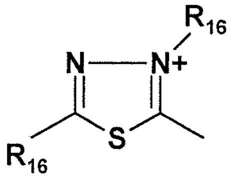 ; 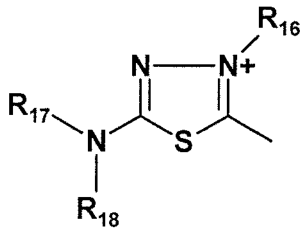 ;

B1 B2 B3

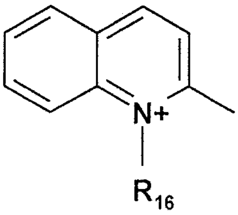 ; 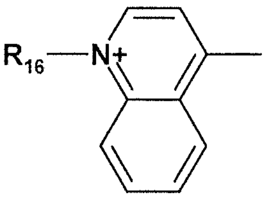 and 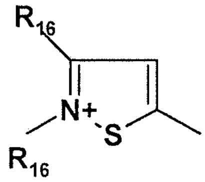 ;

B4 B5 B6

in which $R_{16}$ represents a $C_1$-$C_4$ alkyl radical,
$R_{17}$ and $R_{18}$, which may be identical or different, represent a hydrogen atom or a $C_1$-$C_4$ alkyl radical;
c) the compounds of formulae (VI) and (VI') below:

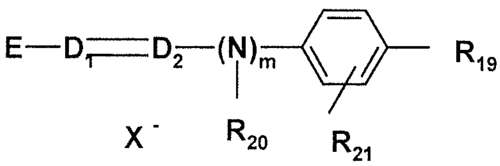

(VI)

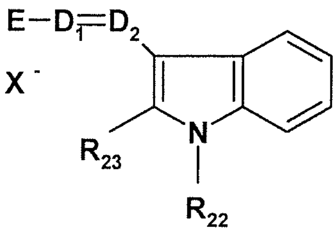

(VI')

in which:

$R_{19}$ represents a hydrogen atom, a $C_1$-$C_4$ alkoxy radical, a halogen atom such as bromine, chlorine, iodine or fluorine, or an amino radical,
$R_{20}$ represents a hydrogen atom, a $C_1$-$C_4$ alkyl radical or forms, with a carbon atom of the benzene ring, a heterocycle optionally containing oxygen and/or substituted with one or more $C_1$-$C_4$ alkyl groups,
$R_{21}$ represents a hydrogen atom or a halogen atom,
$R_{22}$ and $R_{23}$, which may be identical or different, represent a hydrogen atom or a $C_1$-$C_4$ alkyl radical,
$D_1$ and $D_2$, which may be identical or different, represent a nitrogen atom or a -CH group,
m = 0 or 1,
$X^-$ represents an anion;
E represents a group chosen from structures E1 to E8 below:

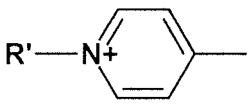 ; 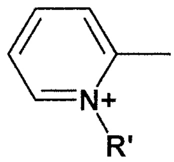 ;

E1 E2

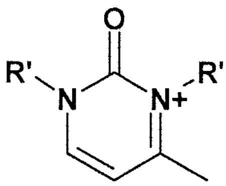 ; 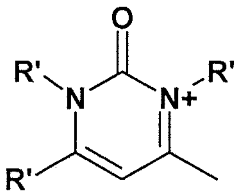 ; 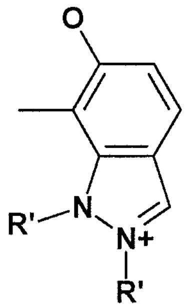 ;

E3 E4 E5

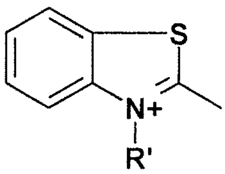 ; 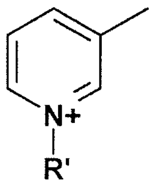 and 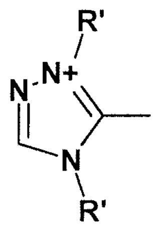

E6 E7 E8

in which R' represents a $C_1$-$C_4$ alkyl radical;
when m = 0 and $D_{13}$ represents a nitrogen atom, then E may also denote a group of structure E9 below:

E9 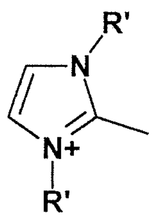

in which R' represents a $C_1$-$C_4$ alkyl radical;
d) the compounds of formula (VII) below:

$$G\text{-}N{=}N\text{-}J \quad (VII)$$

in which:

the symbol G represents a group chosen from the structures $G_1$ to $G_3$ below:

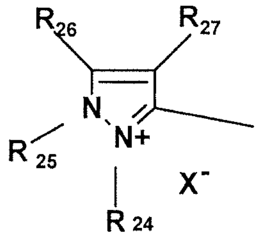

$G_1$

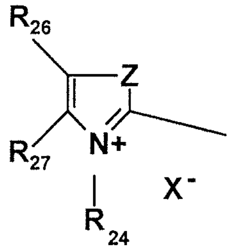

$G_2$

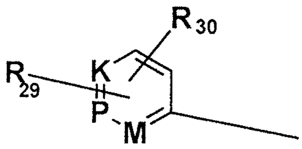

$G_3$

in which structures $G_1$ to $G_3$,
$R_{24}$ denotes a $C_1$-$C_4$ alkyl radical, a phenyl radical which may be substituted with a $C_1$-$C_4$ alkyl radical, or a halogen atom chosen from chlorine, bromine, iodine and fluorine;
$R_{25}$ denotes a $C_1$-$C_4$ alkyl radical or a phenyl radical;
$R_{26}$ and $R_{27}$, which may be identical or different, represent a $C_1$-$C_4$ alkyl radical, a phenyl radical, or form together in $G_1$ a benzene ring substituted with one or more $C_1$-$C_4$ alkyl, $C_1$-$C_4$ alkoxy or $NO_2$ radicals, or form together in $G_2$ a benzene ring optionally substituted with one or more $C_1$-$C_4$ alkyl, $C_1$-$C_4$ alkoxy or $NO_2$ radicals;
$R_{26}$ may also denote a hydrogen atom;
Z denotes an oxygen or sulfur atom or a group - $NR_{25}$;
M represents a group -CH, -CR (R denoting $C_1$-$C_4$ alkyl) or
-$NR_{28}(X^-)r$;
K represents a group -CH, -CR (R denoting $C_1$-$C_4$ alkyl) or
-$NR_{28}(X^-)r$;
P represents a group -CH, -CR (R denoting $C_1$-$C_4$ alkyl) or
-$NR_{28}(X^-)r$;
r denotes 0 or 1;
$R_{28}$ represents an $O^-$ atom, a $C_1$-$C_4$ alkoxy radical or a $C_1$-$C_4$ alkyl radical;
$R_{29}$ and $R_{30}$, which may be identical or different, represent a hydrogen atom or a halogen atom, a $C_1$-$C_4$ alkyl or $C_1$-$C_4$ alkoxy radical, or an -$NO_2$ radical;
$X^-$ represents an anion;
the symbol J represents:

- (a) a group of structure $J_1$ below:

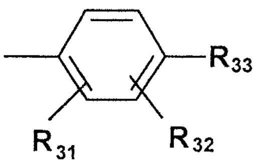

$J_1$

in which structure $J_1$,
$R_{31}$ represents a hydrogen atom, a halogen atom, a $C_1$-$C_4$ alkyl or $C_1$-$C_4$ alkoxy radical, an -OH, -$NO_2$, -$NHR_{34}$, -$NR_{35}R_{36}$ or $C_1$-$C_4$ -NHCOalkyl radical, or forms with $R_{32}$ a 5- or 6-membered ring optionally

containing one or more hetero atoms chosen from nitrogen, oxygen and sulfur;
$R_{32}$ represents a hydrogen atom, a halogen atom, a $C_1$-$C_4$ alkyl or $C_1$-$C_4$ alkoxy radical, or forms with $R_{33}$ or $R_{34}$ a 5- or 6-membered ring optionally containing one or more hetero atoms chosen from nitrogen, oxygen and sulfur;
$R_{33}$ represents a hydrogen atom, an -OH radical, a radical $-NHR_{34}$ or a radical $-NR_{35}R_{36}$;
$R_{34}$ represents a hydrogen atom, a $C_1$-$C_4$ alkyl radical, a $C_1$-$C_4$ monohydroxyalkyl or $C_2$-$C_4$ polyhydroxyalkyl radical or a phenyl radical;
$R_{35}$ and $R_{36}$, which may be identical or different, represent a $C_1$-$C_4$ alkyl radical or a $C_1$-$C_4$ monohydroxyalkyl or $C_2$-$C_4$ polyhydroxyalkyl radical;

- (b) a 5- or 6-membered nitrogenous heterocyclic group, which may contain other hetero atoms and/or carbonyl groups and may be substituted with one or more $C_1$-$C_4$ alkyl, amino or phenyl radicals, and especially a group of structure $J_2$ below:

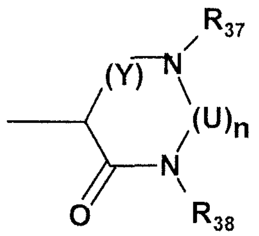

$J_2$

in which structure $J_2$,
$R_{37}$ and $R_{38}$, which may be identical or different, represent a hydrogen atom, a $C_{13}$-$C_{10}$ alkyl radical or a phenyl radical;
Y denotes a -CO- radical or a

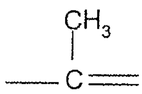

radical;
n = 0 or 1, with, when n denotes 1, U denotes a -CO- radical,
and the mesomeric forms of these structures (IV) to (VII).

**27.** Process for bleaching human keratin fibres dyed with oxidation dyes and/or direct dyes, in particular the hair, **characterized in that** a composition as defined in any one of Claims 1 to 20 is applied to said fibres, at an application temperature of between room temperature and 80°C, for a time that is sufficient to partially or totally strip the dye from the hair.

**28.** Process according to Claim 27, **characterized in that** the application temperature is between 35°C and 50°C.

**29.** Process according to Claim 27 or 28, **characterized in that** the time that is sufficient to develop the bleaching is between 1 and 60 minutes.

**30.** Process according to Claim 29, **characterized in that** the time that is sufficient to develop the bleaching is between 5 and 30 minutes.

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description

- WO 9515144 A
- WO 9501772 A
- EP 1025834 A
- DE 1151242
- US 2149319 A
- US 3838966 A
- JP 04356413 A
- US 3892845 A
- EP 0943316 A
- WO 95101772 A
- WO 9918067 A
- GB 1026978 A
- GB 1153196 A
- DE 2359399
- JP 63169571 A
- JP 3010659 A
- WO 9615765 A
- FR 2750048 A
- DE 3843892
- DE 4133957
- WO 9408969 A
- WO 9408970 A
- FR 2733749 A
- DE 19543988
- EP 714954 A
- FR 2633940
- EP 0216479 A
- US 3915921 A
- US 4509949 A
- EP 0173109 A
- WO 9844012 A
- US 2528378 A
- US 2781354 A
- EP 337354 A
- FR 2270846
- FR 2383660
- FR 2598611
- FR 2470596
- FR 2519863
- FR 2505348
- FR 2542997
- EP 080976 A
- FR 2077143
- FR 2393573
- FR 1492597
- US 4131576 A
- US 3589578 A
- US 4031307 A
- FR 2162025
- FR 2280361
- FR 2252840
- FR 2368508
- FR 1583363
- US 3227615 A
- US 2961347 A
- FR 2080759
- FR 2190406
- FR 2320330
- FR 2316271
- FR 2336434
- FR 2413907
- US 2273780 A
- US 2375853 A
- US 2388614 A
- US 2454547 A
- US 3206462 A
- US 2261002 A
- US 2271378 A
- US 3874870 A
- US 4001432 A
- US 3929990 A
- US 3966904 A
- US 4005193 A
- US 4025617 A
- US 4025627 A
- US 4025653 A
- US 4026945 A
- US 4027020 A
- US 4157388 A
- US 4702906 A
- US 4719282 A
- EP 122324 A
- FR 1400366

### Littérature non-brevet citée dans la description

- **ROBERT L. DAVIDSON.** Handbook of Water soluble gums and resins. Mc Graw Hill Book Company, 1980
- **G. FONNUM ; J. BAKKE ; FK. HANSEN.** *Colloid Polym. Sci,* 1993, vol. 271, 380-389

- **M.R. PORTER.** Handbook of Surfactants. Blackie & Son, 1991, 116-178
- le dictionnaire CTFA. 1993
- **RICHARD J. CRAWFORD.** *Journal of the Society of Cosmetic Chemists,* 1980, vol. 31 (5), 273-278